# EUROPÄISCHE PATENTSCHRIFT

(11) **EP 0 498 188 B1**
(45) Veröffentlichungstag und Bekanntmachung des Hinweises auf die Patenterteilung: **27.03.1996**
(21) Anmeldenummer: 92100713.4
(22) Anmeldetag: 17.01.1992
(51) Int. Cl.: C07C 255/61, A01N 37/34, C07C 251/48, A01N 35/10, A01N 37/50

(54) **Oximether und diese enthaltenden Fungizide**
Oximethers and fungicides containing them
Oximéthers et fongicides les contenant

(30) Priorität: 07.02.1991 DE 4103695
(43) Veröffentlichungstag der Anmeldung: 12.08.1992
(60) Teilanmeldung: 95110459.5
(73) Patentinhaber: BASF Aktiengesellschaft, D-67063 Ludwigshafen (DE)
(72) Erfinder: Benoit, Remy, Dr., W-6700 Ludwigshafen (DE); Sauter, Hubert, Dr., W-6800 Mannheim 1 (DE); Kirstgen, Reinhard, Dr., W-6730 Neustadt (DE); Lorenz, Gisela, Dr., W-6730 Neustadt (DE); Ammermann, Eberhard, Dr., W-6700 Ludwigshafen (DE)

(56) Entgegenhaltungen:
- EP-A- 0 006 254
- EP-A- 0 254 426
- EP-A- 0 363 818
- EP-A- 0 407 891

## Beschreibung

Die vorliegende Erfindung betrifft neue Oximetherderivate, ihre Herstellung und ihre Verwendung als Fungizide.

Es ist bekannt, z.B. den 1-Methoxyimino-1-(2-phenoxymethylphenyl)-essigsäuremethylester als Fungizide zu verwenden (EP 253 213). Ihre Wirkung ist jedoch unbefriedigend.

Es wurde nun gefunden, daß neue Oximether der Formel I 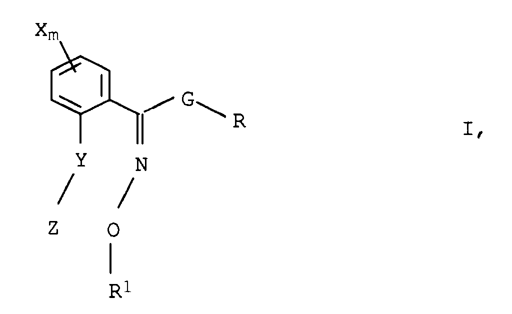
in der der Index und die Substituenten die folgende Bedeutung haben:
- G: eine Gruppe C=O,
- R: C₂-C₈-Alkenyl, C₂-C₈-Alkinyl, Phenyl, Hetaryl oder -CR²R³R⁴,
- R²,R⁴: Wasserstoff, C₁-C₈-Alkyl, Arylalkyl, Hetarylalkyl, Alkoxyalkyl, Aryloxyalkyl, Aryl oder Hetaryl, oder
- R²,R⁴: gemeinsam mit dem C-Atom, dessen Substituenten sie sind, C₃-C₈-Cycloalkyl,
- R³: Wasserstoff, Methyl, Cyano, Carboxyl oder C₁-C₈-Alkoxycarbonyl, oder
- R²,R³: gemeinsam mit dem C-Atom, an das sie gebunden sind, ein 5- bis 8-gliedriger Lactonring,
- R¹: C₁-C₈-Alkyl,
- X: Nitro, Cyano, Halogen, C₁-C₄-Alkyl, C₁-C₄-Alkoxy oder C₁-C₄-Halogenalkyl,
- m: 0, 1, 2, 3 oder 4, wobei die Reste X verschieden sein können, wenn m größer als 1 ist,
- Y: O, S(O)ₙ (n = 0, 1 oder 2), -O-CO-, -CO-O-, Azo, Carbonylamino, Aminocarbonyl, Aminocarbonyloxy,
C₁-C₈-Alkylen, C₂-C₈-Alkenylen oder C₂-C₈-Alkinylen,
C₁-C₈-Alkylenoxy, Oxy-C₁-C₄-Alkylen, C₁-C₈-Thioal-kylen, Oxycarbonyl-C₁-C₈-alkylen, Carbonyloxy-C₁-C₈-alkylen, Oxy-C₂-C₈-alkylenoxy, Oxy-C₂-C₈-alkenylen,
C₁-C₈-Alkylamino, Carbonyl-C₁-C₈-alkylamino, C₁-C₈-Alkylaminocarbonyl, C₁-C₈-Alkylaminocarbonyloxy, Oxyimino oder Iminooxyalkylen,
- Z: C₁-C₁₈-Alkyl, C₁-C₄-Alkoxy-C₁-C₁₀-alkyl, C₁-C₁₀-Halogenalkyl, C₂-C₁₈-Alkenyl, C₃-C₈-Alkinyl, C₁-C₄-Alkoxycarbonyl,
Aryl, Aryl-C₁-C₁₀-alkyl, Aryl-C₂-C₁₀-alkenyl, Aryloxy-C₁-C₁₀-alkyl oder Hetaryl,
wobei die für Z genannten Reste ggf. substituiert sind durch
Cyano, Formyl, Nitro, Halogen, C₁-C₄-Alkyl, C₁-C₄-Halogenalkyl, C₁-C₄-Alkoxy-C₁-C₄-alkyl, C₁-C₄-Dialkoxy-C₁-C₄-alkyl, C₂-C₄-Alkenyl, C₂-C₄-Halogenalkenyl, C₁-C₄-Alkoxy, C₁-C₄-Alkoxycarbonyl, ggf. subst. C₁-C₁₀-Alkoxyimino, C₂-C₁₀-Alkenyloxyimino, Aralkyloxyimino,
ggf. subst. C₂-C₁₀-Alkanoyl,
ggf. subst. Phenyl oder Phenoxy, oder
ggf. subst. C-verknüpfter 5-gliedriger Heterocyclus, der ein bis vier gleiche oder verschiedene Heteroatome (Stickstoff, Sauerstoff oder Schwefel) enthält, in dem zwei benachbarte Substituenten einen ggf. subst. aromatischen oder heteroaromatischen Ring bilden können,
eine sehr gute fungizide Wirkung haben, die besser als die der bekannten Fungizide ist. Die neuen Verbindungen haben auch eine insektizide Wirkung. Die neuen Verbindungen der Formel I können bei ihrer Herstellung aufgrund der C==N-Doppelbindungen als E-Z-Isomerengemische anfallen, die in üblicher Weise, z.B. durch Kristallisation oder Chromatographie, in die einzelnen Komponenten getrennt werden können. Sowohl die einzelnen isomeren Verbindungen als auch ihre Gemische werden von der Erfindung umfaßt und sind als Fungizide brauchbar.

R bedeutet bevorzugt Phenyl, Furyl, Thienyl, Cyclopropyl, Cyclobutyl, Cyclopentyl, Cyclohexyl, Vinyl oder 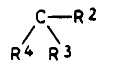
R¹ bedeutet bevorzugt Methyl, Ethyl, iso-Propyl, Propyl.

R² bedeutet bevorzugt Wasserstoff, Methyl, Ethyl, Propyl.

R³ bedeutet bevorzugt Wasserstoff oder -CO-OCH₃.

R⁴ bedeutet bevorzugt Wasserstoff, Methyl, Ethyl, Propyl.

Y bedeutet bevorzugt Methylen, Ethylen, Ethenylen, Ethinylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-CH₂-, -O-N==C-, -C==N-O-, -N==N-Gruppe oder O oder S.

Z bedeutet bevorzugt C₁-C₁₈-Alkyl (z. B. Methyl, Ethyl, n- und iso-Propyl, n-, sec-, iso- und tert.-Butyl, 3-Methylbutyl, 2,2-Dimethylpropyl, 1,1-Dimethylpropyl, 3-Hexyl, n-Pentyl, n-Hexyl, n-Heptyl, n-Octyl, n-Nonyl, n-Decyl, n-Pentadecyl, n-Heptadecyl, 2,6-Dimethylhept-1-yl); Cyanmethyl;
C₂-C₉-Alkenyl (z.B. Vinyl, 2-Propen-2-yl, 1-Propen-1-yl, 2-Buten-2-yl, 2-Methyl-prop-1-en-1-yl, Allyl, 2-Buten-1-yl, 3-Methyl-2-buten-1-yl, 1,3-Pentadien-1-yl, 2,6-Dimethyl-1,5-heptadien-1-yl, 2,6-Dimethyl-hept-5-en-1-yl); C₂-Alkinyl (z.B. Ethinyl, 2-Phenylethinyl);
C₁-C₂-Alkoxy-C₁-C₂-alkyl (Methoxymethyl, Ethoxymethyl, 1-Methoxyethyl), und Halogen-C₁-C₂-alkyl (z.B. Chlormethyl, 1-Chlorethyl, Dichlormethyl, Trichlormethyl, Brommethyl, Trifluormethyl), Phenyl, substituiertes Phenyl, wie Halogenphenyl (z.B. 2-Fluor-, 3-Fluor-, 4-Fluor-, 6-Fluor-, 2-Chlor-, 2,4-Difluor-, 2,6-Difluor-, 2,3,4,5,6-Pentafluor-, 2-Chlor-, 3-Chlor-, 4-Chlor-, 2,5-Dichlor-, 2,5-Dichlor-, 2,6-Dichlor-, 3,4-Dichlor-, 3,5-Dichlor-, 2,4,5-Trichlor-, 2,3,4,5,6-Pentachlor-, 2-Brom-, 3-Brom-, 4-Bromphenyl); C₁-C₄-Alkylphenyl (z.B. 2-Methyl-, 3-Methyl-, 2,3-Dimethyl-, 2,4-Dimethyl-, 2,5-Dimethyl-, 2,6-Dimethyl-, 3,4-Dimethyl-, 3,5-Dimethyl-, 2,4,6-Trimethyl-, 4-t-Butyl-Phenyl); Arylphenyl (z.B. 2-Phenyl-, 4-Phenylphenyl); Halogen-C₁-alkylphenyl (z.B. 2-Trifluor-, 3-Trifluor-, 4-Trifluormethylphenyl); C₁-C₄-Alkoxyphenyl (z.B. 2-Methoxy-, 3-Methoxy-, 4-Methoxy-, 3,4-Dimethoxy-, 3,4,5-Trimethoxy-, 4-t-Butoxyphenyl); 2-, 3-, 4-Phenoxyphenyl); C₁-C₄-Alkanoylphenyl (z.B. 4-Acetyl-, 3-Acetyl-, 2-Acetyl-, 4-Acetyl-2-methylphenyl); Formylphenyl (z.B. 2-Formyl-, 3-Formyl-, 4-Formylphenyl); C₁-C₄-Dialkoxy-C₁-C₄-alkylphenyl [z.B. 4-(1,1-Dimethoxyethyl), 4-(Dimethoxymethyl), 3-(1,1-Dimethoxyethyl), 3-(Dimethoxymethyl), 4-(1,1-Dimethoxyethyl)-2-methylphenyl]; C₁-C₁₀-O-Alkyloxyiminophenyl (z.B. 4-[1-(O-Ethyloxyimino)ethyl], 4-(O-Ethyloxyiminomethyl), 3-[1-(O-Ethyloxyimino)ethyl], 3-(O-Ethyloxyiminomethyl), 4-[1-(O-Ethyloxyimino)ethyl]-2-methyl, 4-[1-(O-Hexyloxyimino)ethyl], 4-(O-Hexyloxyiminomethyl), 3-[1-(O-Hexyloxyimino)ethyl], 3-(O-Hexyloxyiminomethyl),4-[1-(O-Hexyloxyimino)ethyl]-2-methyl, 4-[1-(O-iso-Butyloxyimino)ethyl], 4-(O-iso-Butyloxyiminomethyl), 3-[1-(O-iso-Butyloxyimino)ethyl], 3-(O-iso-Butyloxyiminomethyl), 4-[1-(O-iso-Butyloxyimino)ethyl]-2-methylphenyl);
C₂-C₁₀-Alkenyloxyiminophenyl (z.B. 4-[1-(O-2-Buten-1-oxyimino)ethyl], 4-(O-2-Buten-1-oxyiminomethyl), 3-[1-(O-2-Buten-1-oxyimino)ethyl], 3-(O-2-Buten-1-oxyiminomethyl), 4-[1-(O-2-Buten-1-oxyimino)ethyl]-2-methylphenyl); C₁-C₁₀-Arylalkyloxyiminophenyl (z.B. 4-[1-(O-Benzyloxyimino)ethyl], 4-(O-Benzyloxyiminomethyl), 3-[1-(O-Benzyloxyimino)ethyl], 3-(O-Benzyloxyiminomethyl), 4-[1-(O-Benzyloxyimino)ethyl]-2-methylphenyl, substituiertes Benzyl (z.B. Halogenbenzyl, 2-Fluor-, 3-Fluor-, 4-Fluor-, 2-Chlor-, 3-Chlor-, 4-Chlor-, 2-Chlor-, 6-Fluor-, 2,4-Dichlor-, 2,6-Dichlor-, 3,5-Dichlor-, 2,4,6-Trichlor-, 2-Brom-, 3-Brom-, 4-Brom-, 2-Methyl-, 3-Methyl-, 4-Methyl-, 4-t-Butyl-, 2,4-Dimethyl-, 2,6-Dimethyl-, 2,4,6-Trimethyl-, 2-Methoxy-, 3-Methoxy-, 4-Methoxy-, 2-Trifluormethyl-, 3-Trifluormethyl-, 4-Trifluormethyl-, 4-t-Butoxy-, 4-Phenoxy-, 4-Phenylbenzyl-, -Methyl--ethyl-, -iso-Propyl-, -Hydroxy-, 2-Methoxy--hydroxy-, 4-Methoxy-hydroxy-, 3,4-Dimethoxy- -hydroxy-, 2-Methoxy- -methoxy-, 4-Methoxy-methoxy-, 3,4-Dimethoxy--methoxybenzyl); gegebenenfalls substituiertes Phenethyl (z.B. Phenyl-, 1-Methyl-2-phenyl-, 2-(para-t-Butylphenyl-), 2-(para-t-Butylphenyl)-1-Methyl-, 2-(ortho-Chlorphenyl)-, 2-(meta-Chlorphenyl)-, 2-(para-Chlorphenyl)-ethyl); gegebenenfalls substituiertes Styryl (z.B. Styryl; 2'-Chlor-, 3'-Chlor-, 4'-Chlor-, 2',4'-Dichlor-, 2'-Fluor-, 4'-Fluor-, 2'-Methyl-, 4'-Methyl-, 4'-t-Butyl-, 2'-Methoxy-, 4'-Methoxy-, 4'-Phenoxy-styryl); Phenyl-C₃-C₆-alkyl (z.B. 3-Phenylpropyl, 2-Methyl-3-Phenylpropyl, 4-Phenylbutyl, 5-Phenylpentyl, 6-Phenylhexyl, 3-(4'-t-Butylphenyl)-2-Methyl-propyl); Aryloxy, (wenn Y = O, S, Oxycarbonyl, Oxyalkoxy, Oxyimino, Thioalkyl, -N==N-, Oxycarbonylalkyl, Oxyalkyl), wie substituiertes Phenoxy (z.B. 2-Chlor-, 3-Chlor-, 4-Chlor-, 2-Methyl-, 4-Methyl-, 2-Methoxy-, 4-Methoxy-, 2-Trifluormethyl-, 4-Trifluormethyl-phenoxy); Aryloxy-C₁-C₆-alkyl, wie gegebenenfalls substituiertes Phenoxymethyl (z.B. 2-Chlor-, 2-Methyl-, 4-Methyl-, 2-Methoxy-, 4-Methoxy-, 4-t-Butyl-phenoxymethyl); gegebenenfalls substituiertes Phenoxyethyl (z.B. 2-Chlor-, 4-Chlor-, 4-Fluor-, 2-Methyl-, 4-Methyl-, 2-Methoxy-, 4-Methoxy-, 4-t-Butyl-phenoxyethyl), gegebenenfalls substituiertes C₃-C₆-Phenoxyalkyl (z.B. 3-Phenoxypropyl, 3-(ortho-Chlorphenoxy)propyl, 3-(para-Chlor-phenoxy)propyl, 4-Phenoxybutyl, 4-(ortho-Chlorphenoxy)-butyl, 4-(para-Chlorphenoxybutyl), 5-Phenoxypentyl, 5-(ortho-Chlorphenoxy)pentyl, 5-(para-Chlorphenoxy)pentyl, 6-Phenoxyhexyl); Phenoxyethoxy (wenn Y = O, S, Oxycarbonyl, Oxyalkoxy, Oxyimino, Thioalkyl, -N==N-, Oxycarbonylalkyl, Oxyalkyl), Methoxycarbonyl, t-Butoxycarbonyl; gegebenenfalls substituiertes Heteroaryl (z.B. Furyl, 2-Furyl, 3-Furyl, 5-Nitro-2- und 3-Furyl, 5-Chlor-2- und 3-Furyl, Benzfuran-2-und 3-yl, Thienyl, 2-Thienyl, 3-Thienyl, 5-Nitro-2- und 3-thienyl, 5-Chlor-2- und 3-thienyl, Benzothien-2- und 3-yl.

N-Methyl-pyrrol-2- und 3-yl, N-Methyl-pyrazol-3-, 4- und 5-yl, N-Methylimidazol-2-, 4- und 5-yl, 1-Methyl1,2,3-triazol-4- und 5-yl, 1-Methyl-1,2,4-triazol-3- und 5-yl, 1-Methyl-tetrazol-5-yl, Isoxazol-3-, 4- und 5-yl, Benzisoxazol-3-yl, Benzoxazol-2-yl, Oxazol-2-, 4- und 5-yl, Thiazol-2-, 4- und 5-yl, Benzothiazol-2-yl, Benzisothiazol-3-yl, Isothiazol-3-, 4- und 5-yl, 1,2,3-Thiadiazol-4-yl, 1,2,4-Thiadiazol-5-yl, 1,3,4-Thiadiazol-3-yl, 1,3-Thiazolo-[4,5-b]-pyridin-2-yl; substituiertes Hetarylalkenyl (z.B. 2-(2'- und 3'-Furyl)-, 2-(5'-Nitro-2'- und 3'-furyl)-, 2-(2'- und 3'-Thienyl)-, 2-(5'-Nitro-2'- und 3'-thienyl)ethenyl).

Weitere bevorzugte Verbindungen sind die Oximether der Formel I in der
R C₃-C₈-Cycloalkyl, C₂-C₈-Alkinyl, Phenyl, Hetaryl oder 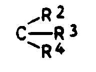
bedeutet,
R¹ Wasserstoff, C₁-C₆-Alkyl bedeutet,
R² Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
R³ Wasserstoff bedeutet,
R⁴ Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
Y Methylen, Ethylen, Ethenylen, Ethinylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-CH₂-, -O-N==C-, -C==N-O-, -N==N-Gruppe oder O oder S bedeutet,
Z ein geradkettiges oder verzweigtes C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolyl, Isochinolyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzoisoxazolyl, Benzopyrrazolyl, Benzoimidazolyl, Indolyl, Isoindolyl, Benzothiophenyl, Isobenzothiophenyl, Benzofuranyl, Isobenzofuranyl,
X Halogen, Cyano, C₁-C₄-Alkyl, C₁-C₄-Halogenalkyl, C₁-C₄-Alkoxy bedeutet und
m eine ganze Zahl zwischen 1 und 4 bedeutet.

Weitere bevorzugte Verbindungen sind Oximether der Formel I in der
R 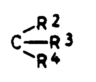
bedeutet,
R¹ Wasserstoff, C₁-C₆-Alkyl bedeutet,
R² Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
R³ Cyano bedeutet,
R⁴ Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
oder
R² + R⁴ zusammen mit dem C, dessen Substituenten sie sind, einen C₃-C₈-Cycloalkylring bedeuten,
Y Methylen, Ethylen, Ethenylen, Ethinylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-CH₂-, -O-N==C-, -C==N-O-, -N==N-Gruppe oder O oder S bedeutet,
Z ein geradkettiges oder verzweigtes C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolyl, Isochinolyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzoisoxazolyl, Benzopyrrazolyl, Benzoimidazolyl, Indolyl, Isoindolyl, Benzothiophenyl, Isobenzothiophenyl, Benzofuranyl, Isobenzofuranyl,
X Halogen, Cyano, C₁-C₄-Alkyl, C₁-C₄-Halogenalkyl, C₁-C₄-Alkoxy bedeutet und
m eine ganze Zahl zwischen 1 und 4 bedeutet.

Weitere bevorzugte Verbindungen sind Oximether der Formel I in der
R 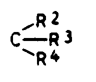
bedeutet,
R¹ Wasserstoff, C₁-C₆-Alkyl bedeutet,
R² Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
R³ -COO-A bedeutet,
A Wasserstoff, Methyl, Ethyl, iso-Propyl, Propyl, n-Butyl, sec-Butyl, tert-Butyl
oder
für den Fall daß R³ den Rest -COO-C₁-C₈-Alkyl bedeutet, R² und R³ zusammen mit dem C, dessen Substituenten sie sind, einen 5 bis 8gliedrigen Lactonring bedeuten,
R⁴ Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
oder
R² + R⁴ zusammen mit dem C, dessen Substituenten sie sind, einen C₃-C₈-Cycloalkylring bedeuten,
Y Methylen, Ethylen, Ethenylen, Ethinylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-CH₂-, -O-N==C-, -C==N-O-, -N==N-Gruppe oder O oder S bedeutet,
Z ein geradkettiges oder verzweigtes C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolyl, Isochinolyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzoisoxazolyl, Benzopyrrazolyl, Benzoimidazolyl, Indolyl, Isoindolyl, Benzothiophenyl, Isobenzothiophenyl, Benzofuranyl, Isobenzofuranyl,
X Wasserstoff, Halogen, Cyano, geradkettiges oder verzweigtes C₁-C₄-Alkyl, C₁-C₄-Halogenalkyl, C₁-C₄-Alkoxy bedeutet und
m eine ganze Zahl zwischen 1 und 4 bedeutet.

Die Verbindungen der Formel Ib, Ic lassen sich herstellen, indem man ein Phenylglyoxylsäuremethylester-O-alkyloxim (EP 253 213) der Formel II 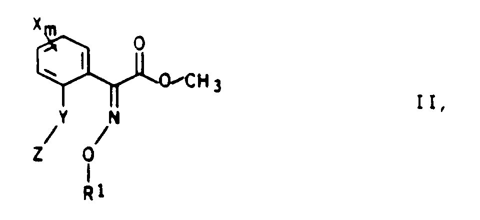
in der R¹, Y, Z, X, m die oben genannte Bedeutung haben, mit einer Verbindung der Formel III 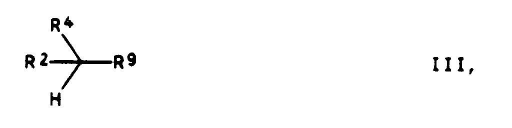
in der R² und R⁴ die oben genannte Bedeutung haben und R⁹ gleich -CN oder -COO-A bedeutet, wobei A die oben genannte Bedeutung hat, unter basischen Bedingungen umsetzt (E.V.P. Tao, G.S. Staten Org.Prep.Proc.Int. Briefs 17 (1985) 235). 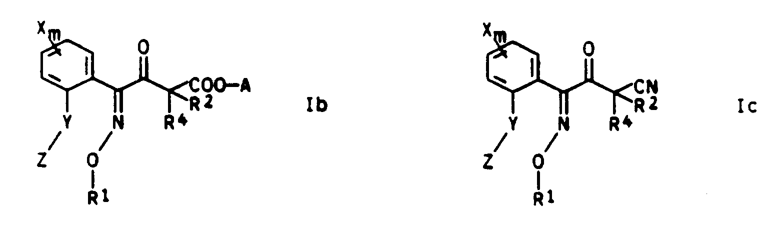
Die Verbindungen Ib lassen sich weiter zu Verbindungen des Typs Ia umsetzen (S. Takei, Y. Kawano, Tetrahedron Lett. 49 (1975) 4389). 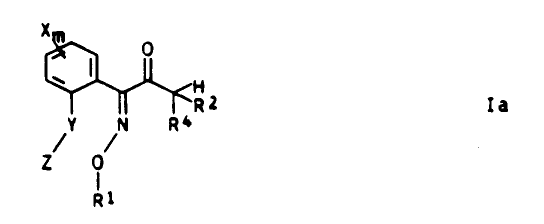
Ausgehend von Verbindungen der Formel II lassen sich die Verbindungen der Formel Id auf einem anderen Weg gewinnen. In bekannter Weise kann man die Verbindungen der Formel IV leicht herstellen (S. Nahm, S.M. Weinreb, Tetrahedron Lett. 39 (1981) 3815). 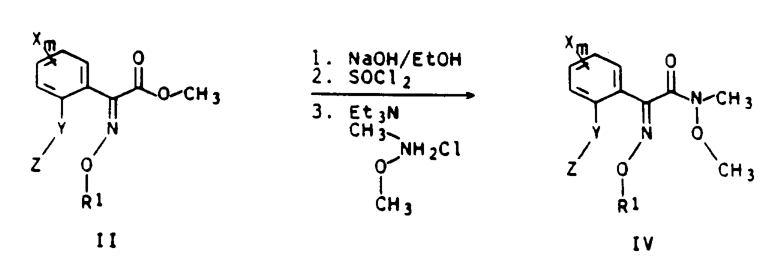
Die weitere Umsetzung der Amide IV mit Organolithiumreagenzien (S. Nahm, S.M. Weinreb, Tetrahedron Lett. 39 (1981) 3815), liefert die gewünschten α-Oxyiminoketone der Formel Id. 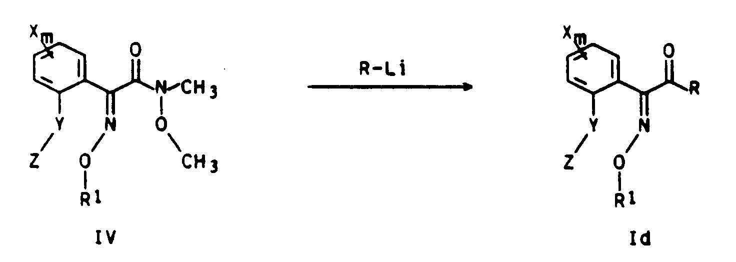
Die Herstellung der neuen Verbindungen und der Zwischenprodukte wird durch die folgenden Beispiele und Vorschriften erläutert:

### Herstellungsbeispiele

### Beispiel 1

### 1-Methoxyimino-1-[2'-(2-methylphenoxymethyl)phenyl]-3-cyano-propan-2-on (Verbindung Nr. 2)

1,74 g NaH 50 %ig wird in 50 ml Cyclohexan vorgelegt, 10 g (0,032 mol) 1-Methoxyimino-1-[2'-(2-methylphenoxymethyl)]phenylessigsäure-methylester in 40 ml Cyclohexan/Acetonitril (1:1) bei Erhitzen zum Rückfluß zugetropft, das Gemisch 6 Stunden weitergekocht und abgekühlt. Die organische Phase wird mit 100 ml Wasser extrahiert, die wäßrige Lösung mit Salzsäure angesäuert und mit Toluol extrahiert. Nach Trocknen und Einengen erhält man 8,7 g (84 %) des Produktes Nr. 2. Fp.: 65°C

### Beispiel 2

### 1-Methoxyimino-1-[2'-(2-methylphenoxymethyl)phenyl]-3-carbo-methoxy-butan-2-on (Verbindung Nr. 83)

10,5 g NaH 50 %ig wird in 150 ml Cyclohexan vorgelegt, 60 g (0,032 mol) 1-Methoxyimino-1-[2'-(2-methylphenoxymethyl)]phenylessigsäure-methylester und 84 g Propionsäuremethylester (0,95 mol) in 150 ml Cyclohexan bei Rückfluß zugetropft, das Gemisch 6 Stunden weitergekocht und abgekühlt. Die organische Phase wird mit 200 ml Wasser gewaschen, die wäßrige Lösung mit Salzsäure angesäuert, mit Cyclohexan rückextrahiert, die gesamten organischen Phasen getrocknet und eingeengt. Man erhält 55 g (78 %) Produkt Nr. 83. IR (cm⁻¹): 1747, 1699, 1495, 1241, 1033)

### Beispiel 3

### 1-Methoxyimino-1-[2'-(2-methylphenoxymethyl)phenyl]-butan-2-on (Verbindung Nr. 300)

50 g (0,135 mol) Verbindung Nr. 83 wird in 500 ml Ethanol und 150 ml Wasser gelöst, zum Rückfluß erhitzt, 50 ml konz. H₂SO₄ langsam zugegeben und das Gemisch 9 Stunden weitergekocht. Nach Abkühlung wird Wasser zugegeben, durch Zugabe von Natronlauge neutralisiert, mit MTB-Ether (Methyl-tert.-Butylether) extrahiert, die organische Phase getrocknet und eingeengt. Das Rohprodukt wird durch Säulenchromatographie gereinigt (Cyclohexan/Essigsäureethylester 9:1), und liefert 25,7 g (61 %) Produkt Nr. 300. IR (cm⁻¹: 2938, 1695, 1495, 1241, 1033)

### Beispiel 4

### 1-Methoxyimino-1-[2'-(2-methylphenoxymethyl)phenyl]-3-methyl-butan-2-on (Verbindung Nr. 380)

1,7 g (4,4 mmol) 1-Methoxyimino-1-[2'-(2-methylphenoxymethyl)phenyl]-3-methyl-3-carbomethoxy-butan-2-on wird in 15 ml Dimethylsulfoxid mit 0,9 g LiCl und 0,16 g Wasser versetzt und auf 140 °C erhitzt. Nach 20 Stunden wird das Reaktionsgemisch abgekühlt, Wasser zugegeben, mit MTB-Ether extrahiert, die Etherphase dreimal mit Wasser gewaschen, getrocknet und eingeengt. Man erhält 1,0 g (70 %) Produkt Nr. 380.
¹H-NMR (CDCl₃): 7.60-6.70 (m, 8H); 4.85 (s, 2H); 4.05 (s, 3H); 3.70 (m, 1H); 2.25 (s, 3H); 1.15 (d, 6H)

### Beispiel 5

### 1-Methoxyimino-1-(2'-methylphenyl)-3-carbomethoxy-3-methyl-butan-2-on (Verbindung Nr. 127)

37,5 g (0,143 mol) 1-Methoxyimino-1-(2'-methylphenyl)-3-carbomethoxybutan-2-on wird in 100 ml DMF (Dimethylformamid) mit 59 g K₂CO₃ und 61 g CH₃I gelöst und 48 Stunden bei Raumtemperatur gerührt. Das K₂CO₃ wird abgesaugt, mit DMF gewaschen, Wasser zu der DMF-Lösung gegeben, die wäßrige Lösung mit MTB-Ether extrahiert, die organische Phase mit Wasser gewaschen, getrocknet und eingeengt. Man erhält 34,3 g (90 %) Produkt Nr. 127.
¹H-NMR (CDCl₃): 7.35-6.90 (m, 4H); 3,95 (s, 3H); 3.75 (s, 3H); 2.10 (s, 3H); 1.55 (s, 6H)

### Vorschrift 1

### 1-Methoxyimino-1-(2'-brommethylphenyl)-3-carbomethoxy-3-methyl-butan-2-on (Verbindung Nr. 128)

15,4 g (0,058 mol) Verbindung Nr. 127 wird mit 12,4 g NBS (N-Bromosuccinimid) und 0,1 g Benzoylperoxyd in 150 ml Cyclohexan gelöst, bei Rückfluß 4 Stunden erhitzt, die Lösung abgekühlt, das Succinimid abgesaugt und die Mutterlauge eingeengt. Man erhält nach Säulenchromatographie (Cyclohexan/Essigsäureethylester 4:1) 15,4 g (78 %) Produkt Nr. 128.
¹H-NMR (CDCl₃): 7.55-6.95 (m, 4H); 4.30 (s, 2H); 4.00 (s, 3H); 3.75 (s, 3H); 1.55 (s, 6H)

### Beispiel 6

### 1-Methoxyimino-1-[2'-(4-acetylphenoxymethyl)phenyl]-3-carbomethoxy-3-methyl-butan-2-on (Verbindung Nr. 129)

15 g (0,42 mol) Verbindung Nr. 128 wird mit 7 g KI in 150 ml DMF gelöst, 7,3 g Kalium Salz des 4-Hydroxyacetophenons in 70 ml DMF zugegeben und das Gemisch über Nacht bei Raumtemperatur gerührt. Dann wird Wasser zugegeben, die wäßrige Lösung mit Ether extrahiert, die organische Phase mit Natronlauge und Wasser gewaschen, getrocknet und eingeengt. Man erhält 15,5 g (90 %) Verbindung Nr. 129.
¹H-NMR (CDCl₃): 7.95-6.85 (m, 8H); 4.90 (s, 2H); 3.95 (s, 3H); 3.65 (s, 3H); 2.55 (s, 3H); 1.50 (s, 6H)

### Vorschrift 2

### 1-Methoxyimino-1-(2'-methylphenyl)essigsäure

120 g (0,58 mol) 1-Methoxyimino-1-(2'-methylphenyl)essigsäuremethylester wird mit 730 g 1M wäßriger KOH-Lösung vorgelegt, 30 min. bei Rückfluß erhitzt, abgekühlt, mit konz. HCl bis pH = 1 angesäuert, abgesaugt, mit Wasser gewaschen und getrocknet. Man erhält 110 g (99 %) des gewünschten Produkts.
¹H-NMR (CDCl₃: 7.40-7.05 (m, 4H); 5.60 (m, 1H); 4.05 (s, 3H); 2.20 (s, 3H)

### Vorschrift 3

### 1-Methoxyimino-1-(2'-methylphenyl)essigsäure-N-methoxy-N-methyl-amid

23,5 g (0,122 mol) 1-Methoxyimino-1-(2'-methylphenyl)essigsäure wird in 22 g SOCl₂ 2 Stunden lang erhitzt, abgekühlt und überschüssiges SOCl₂ abgezogen. Das rohe Säurechlorid wird mit 11,2 g Methylmethoxyaminhydrochlorid in 150 ml CH₂Cl₂ gelöst, 23,1 g Triethylamin bei 5°C langsam zugetropft und das Reaktionsgemisch 24 Stunden bei Raumtemperatur weitergerührt. Das Triethylaminhydrochlorid wird abgesaugt, mit CH₂Cl₂ gewaschen, die vereinigten Filtrate mit Wasser, HCl-Lösung, Natronlauge gewaschen, getrocknet und eingeengt. Man erhält 23 g (94 %) des gewünschten Produktes. Fp. 30°C.

### Vorschrift 4

### 1-Methoxyimino-1-(2'-brommethylphenyl)essigsäure-N-methoxy-N-methylamid

10 g (0,042 mol) 1-Methoxyimino-1-(2'-methylphenyl)essigsäure-N-methoxy-N-methyl-amid, 9,8 g NBS und 0,1 g Benzoylperoxyd werden in 100 ml CCl₄ gelöst und 4 Stunden belichtet. Nach Abkühlung wird das Succinimid abgesaugt, mit CH₂Cl₂ gewaschen und die gesamt organische Phase eingeengt. Man erhält nach Säulenchromatographie (Cyclohexan/Essigsäureethylester 7:3) 8 g (60 %) des gewünschten Produktes.
¹H-NMR (CDCl₃): 7.65-7.25 (m, 4H); 4.50 (s, 2H); 3.95 (s, 3H); 3.60 (s, 3H); 3.25 (s, 3H)

### Vorschrift 5

### 1-Methoxyimino-1-[2'-(2-methyl-4-chlorphenoxymethyl)]phenylessig-säure-N-methoxy-N-methylamid

2,6 g (0,008 mol) 1-Methoxyimino-1-(2'-brommethylphenyl)essigsäure-N-methoxy-N-methyl-amid wird mit 1,4 g KI in 40 ml EtOH gelöst, 1,5 g Kalium-Salz des 4-Chlor-2-methylphenols in 20 ml EtOH zugegeben und das Gemisch über Nacht bei Raumtemperatur gerührt. Danach wird Wasser zugegeben, die wäßrige Lösung mit MTB-Ether extrahiert, die organische Phase mit Natronlauge und Wasser gewaschen, getrocknet und eingeengt. Man erhält nach Säulenchromatographie (Cyclohexan/Essigsäureethylester 4:1) 1,2 g (39 %) des gewünschten Produktes.
¹H-NMR (CDCl₃): 7.65-6.70 (m, 7H); 5.05 (s, 2H); 3.95 (s, 3H); 3.55 (s, 3H); 3.25 (s, 3H); 2.30 (s, 3H)

### Beispiel 7

### 1-Methoxyimino-1-[2'-(2-methylphenoxymethyl)phenyl]-pent-3-in-2-on (Verbindung Nr. 1134)

Unter Stickstoffatmosphäre wird 5 g (0,015 mol) 1-Methoxyimino-1-[2'-(2-methylphenoxymethyl)]phenylessigsäure-N-methoxy-N-methylamid in 50 ml absolutem THF (Tetrahydrofuran) gelöst, bei 0°C 18 ml einer 1M Propinyllithium-Lösung in Lösungsmittel langsam zugetropft, das Gemisch 30 min. bei 0°C weitergerührt, mit einer 1M NH₄Cl-Lösung versetzt, mit MTB-Ether extrahiert, die organische Phase getrocknet und eingeengt. Man erhält 4,5 g (93 %) Verbindung Nr. 1134.
¹H-NMR (CDCl₃): 7.60-6.70 (m, 8H); 4.90 (s, 2H); 4.10 (s, 3H); 2.25 (s, 3H); 2.10 (s, 3H)

### Vorschrift 6

### 1-Methoxyimino-1-(2'-brommethylphenyl)-butan-2-on (Verbindung Nr. 317)

10 g (0,049 mol) 1-Methoxyimino-1-(2'-methylphenyl)-butan-2-on, 11,3 g NBS und 0,1 g Benzoylperoxyd werden in 100 ml CCl₄ gelöst und 4 Stunden mit Licht bestrahlt. Nach Abkühlung wird das Succinimid abgesugt, mit CH₂Cl₂ gewaschen und die gesamt organische Phase eingeengt. Man erhält 13,2 g (91 %) Verbindung Nr. 317.
IR (cm⁻¹): 2975, 2937, 1697, 1032, 761

### Beispiel 8

### 1-Methoxyimino-1-[2'-(2-Methylcarboxyphenyl)phenyl]-butan-2-on (Verbindung Nr. 310)

2 g (0,007 mol) Verbindung Nr. ... wird mit 1,9 g o-Methylbenzoesäure, 1,9 g K₂CO₃ und 0,1 g KI in 50 ml Aceton gemischt und 72 Stunden bei Raumtemperatur gerührt. Wasser wird zugegeben, die wäßrige Lösung mit MTB-Ether extrahiert, die organische Phase mit natronlauge gewaschen, getrocknet und eingeengt. man erhält nach Säulenchromatographie (Cyclohexan/Essigsäureethylester 4:.1) 0,5 g (20 %) Verbindung Nr. 310.
¹H-NMR (CDCl₃): 7.85-7.05 (m, 8H); 5.10 (s, 2H); 3.95 (s, 3H); 2.90 (q, 2H); 2.55 (s, 3H); 1.05 (t. 3H)

In entsprechender Weise werden die in der folgenden Tabelle aufgeführten Verbindungen hergestellt.

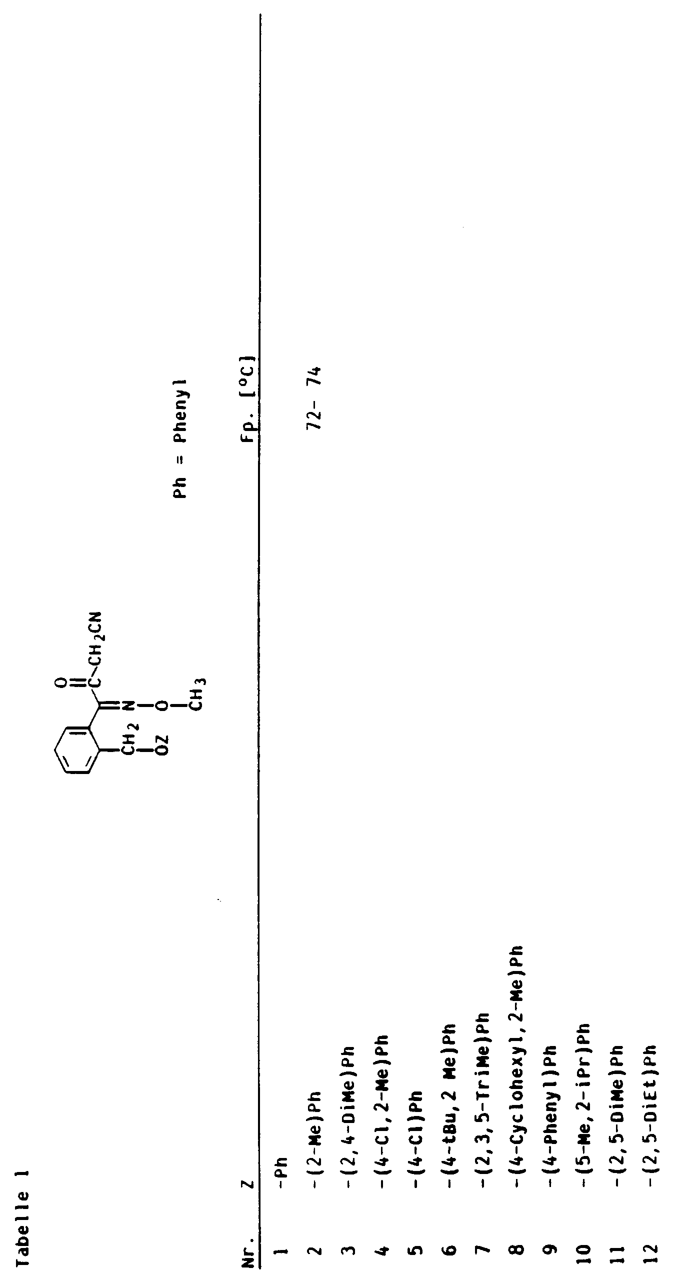

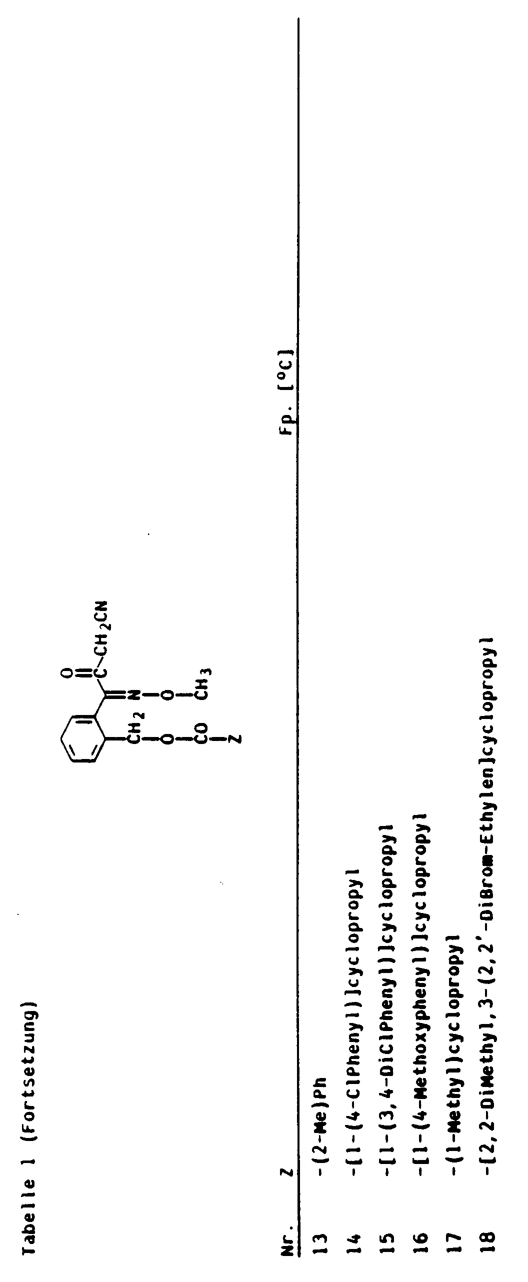

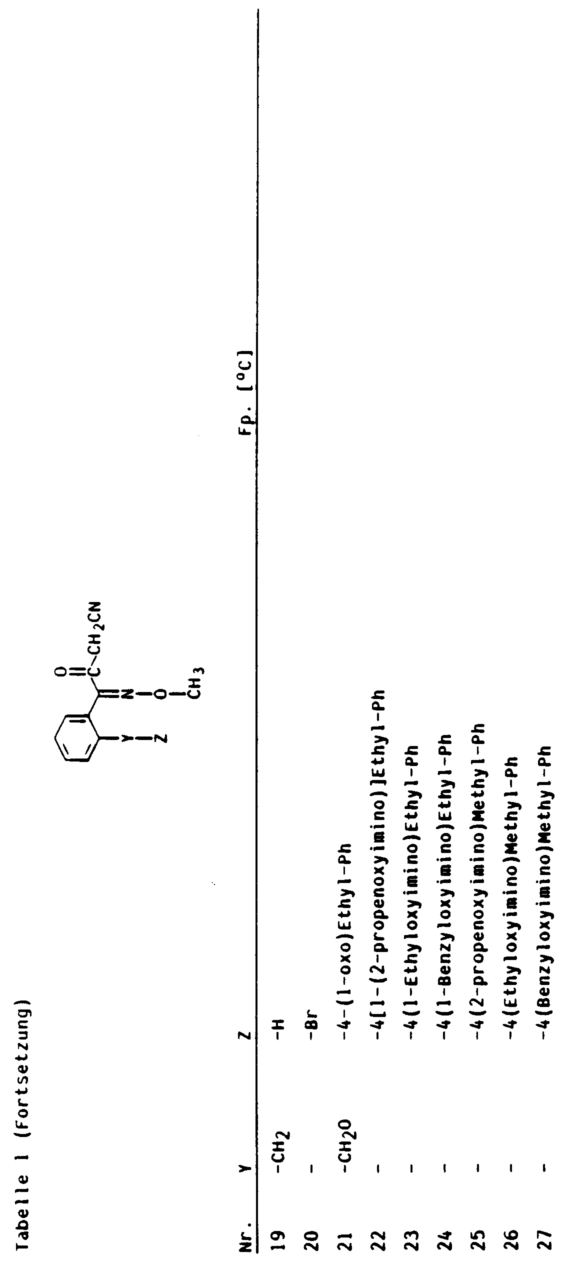

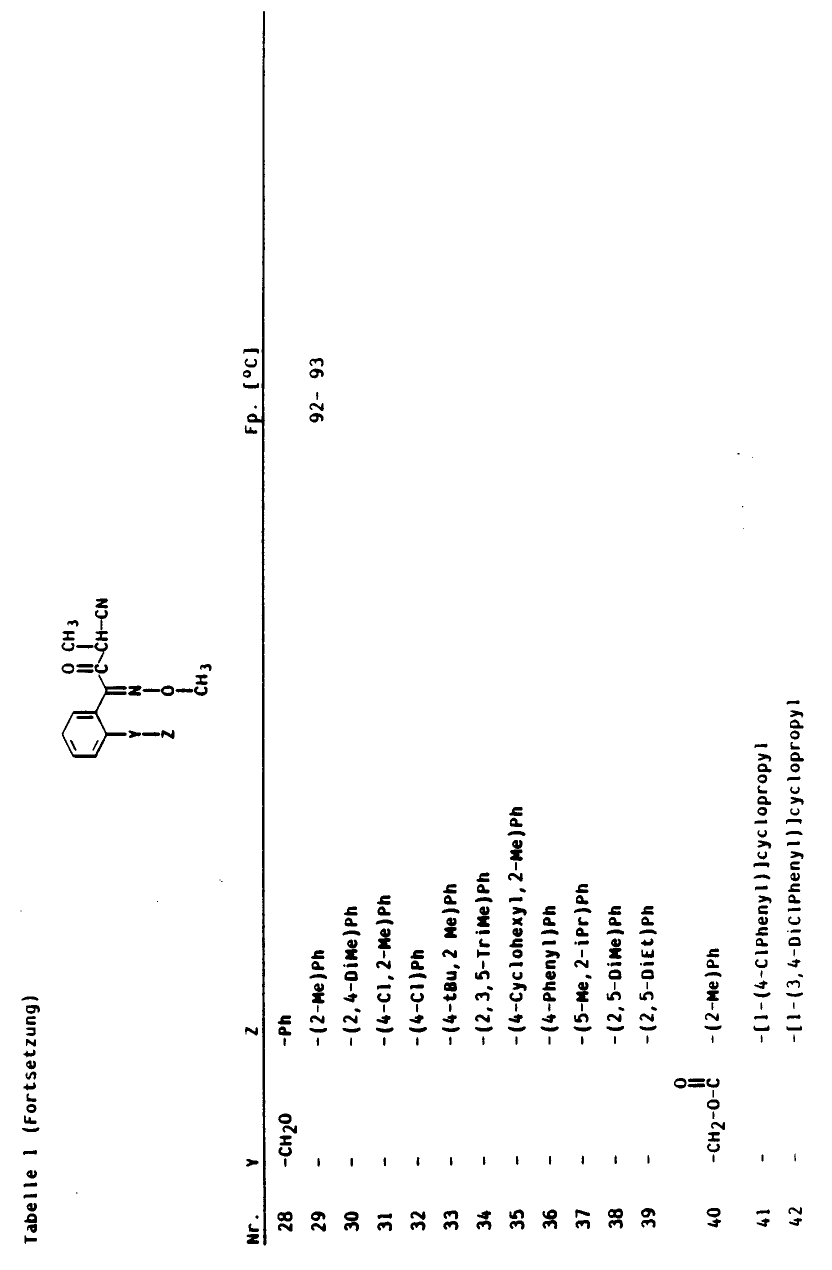

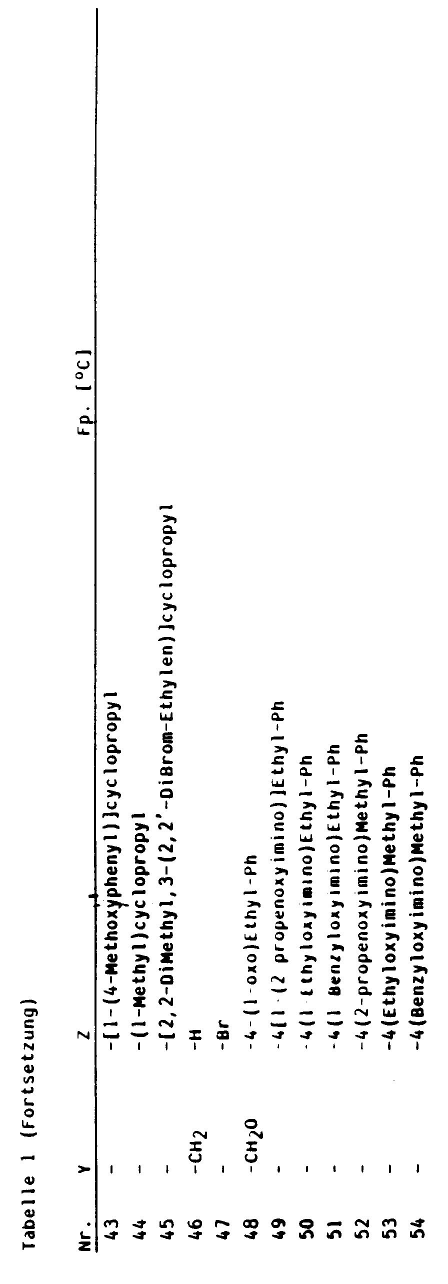

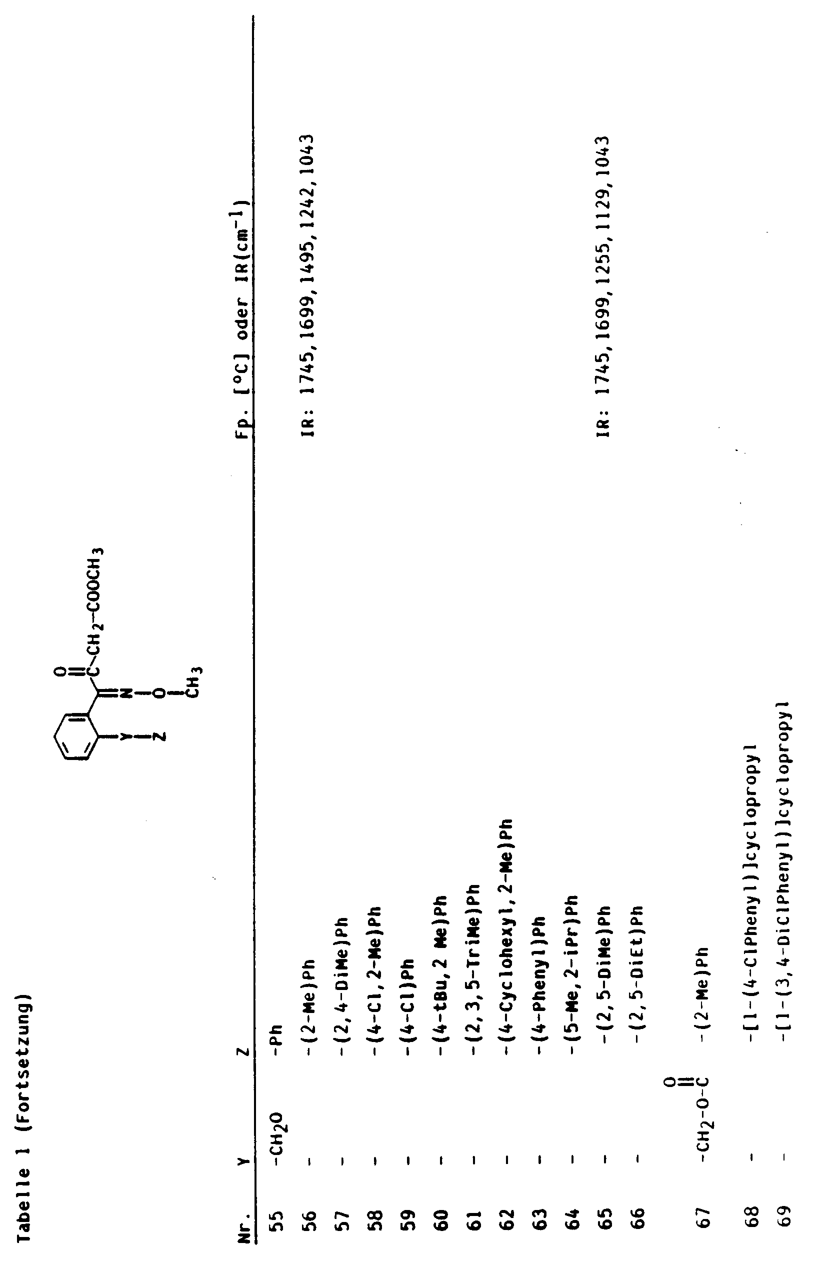

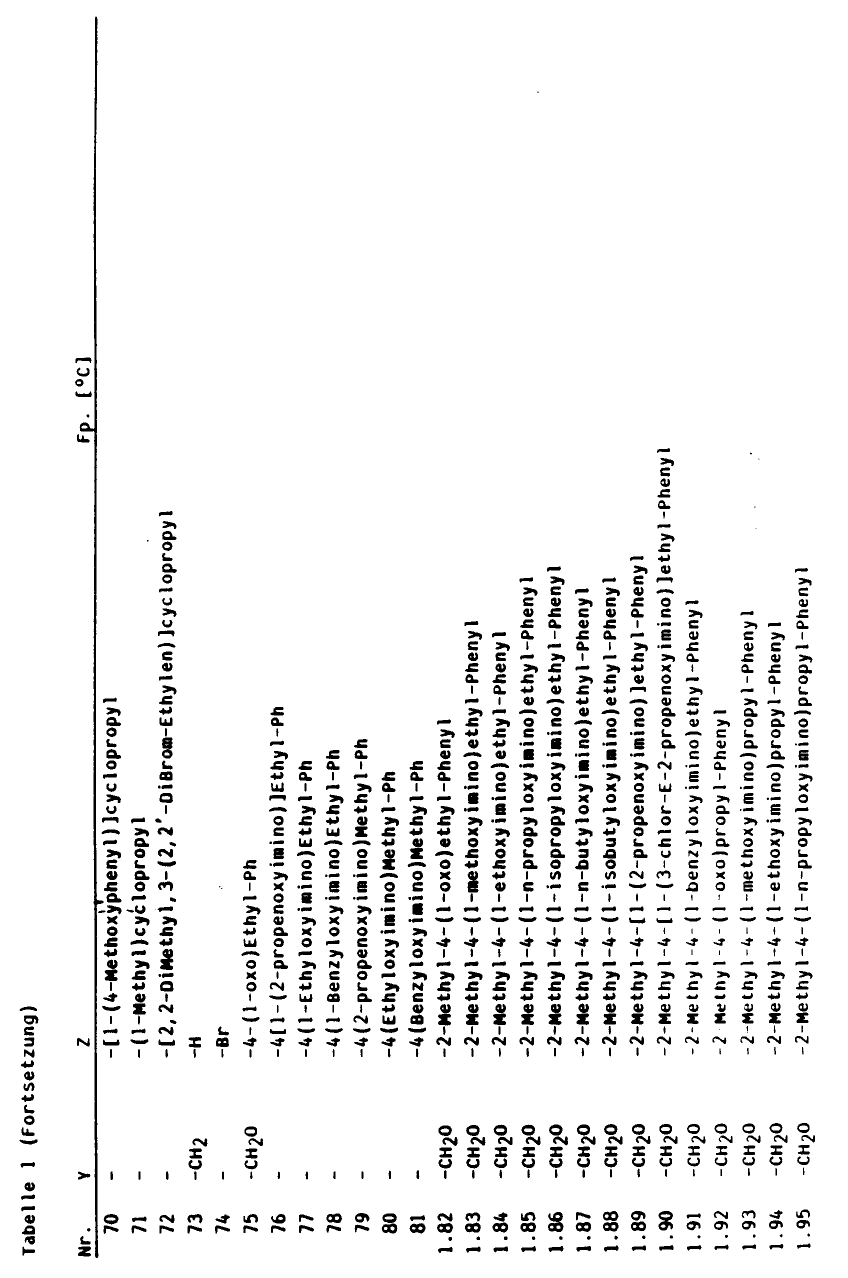

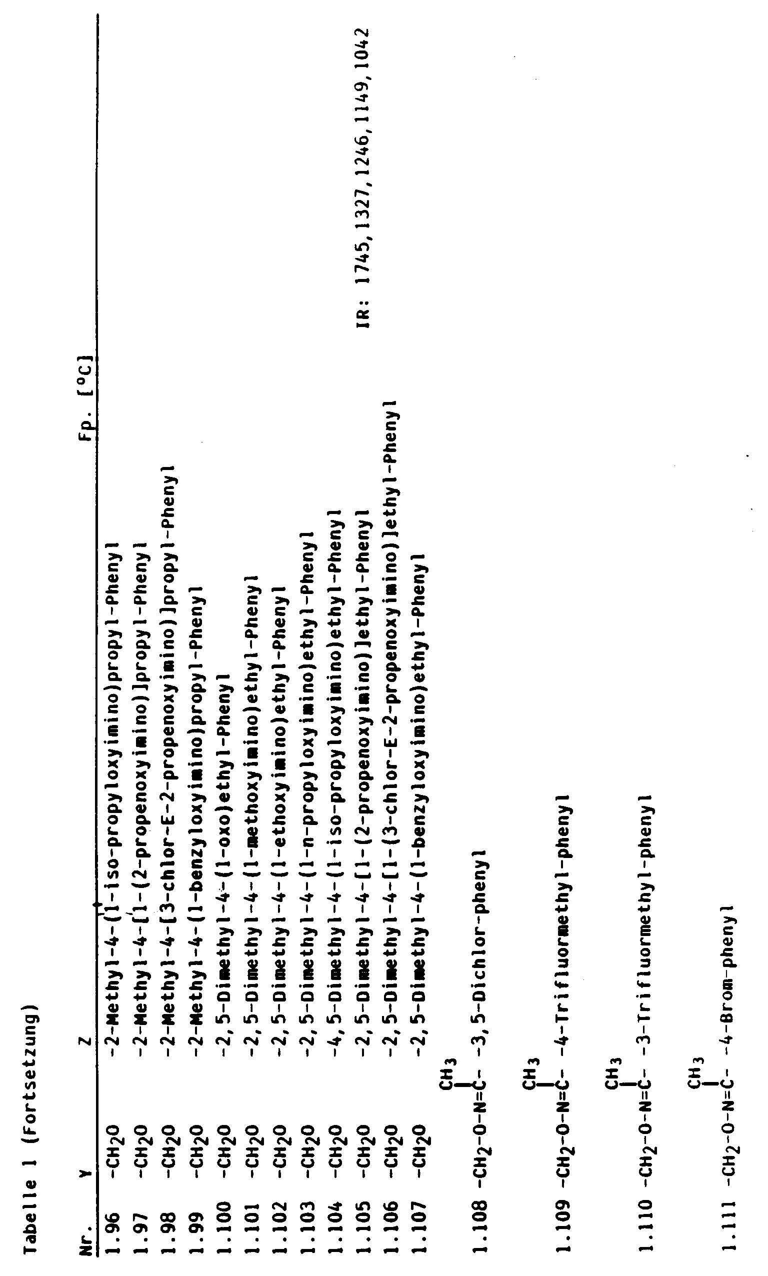

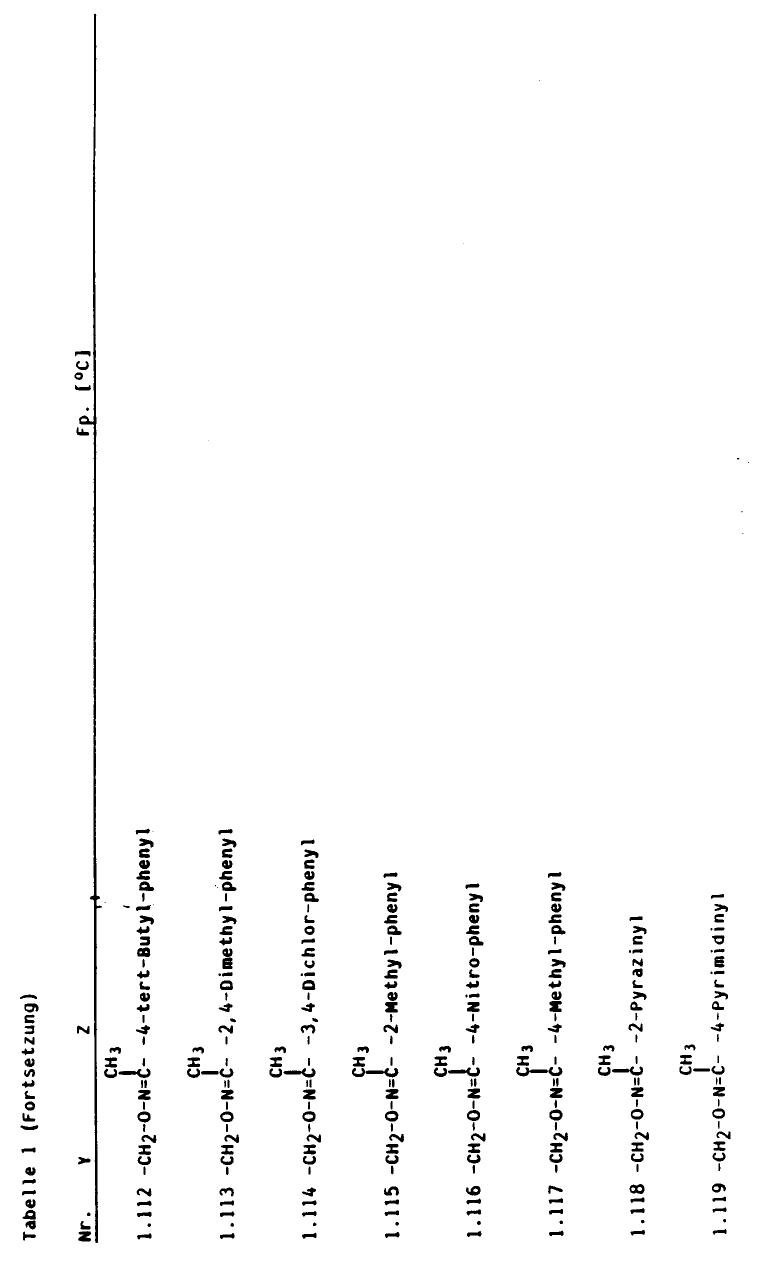

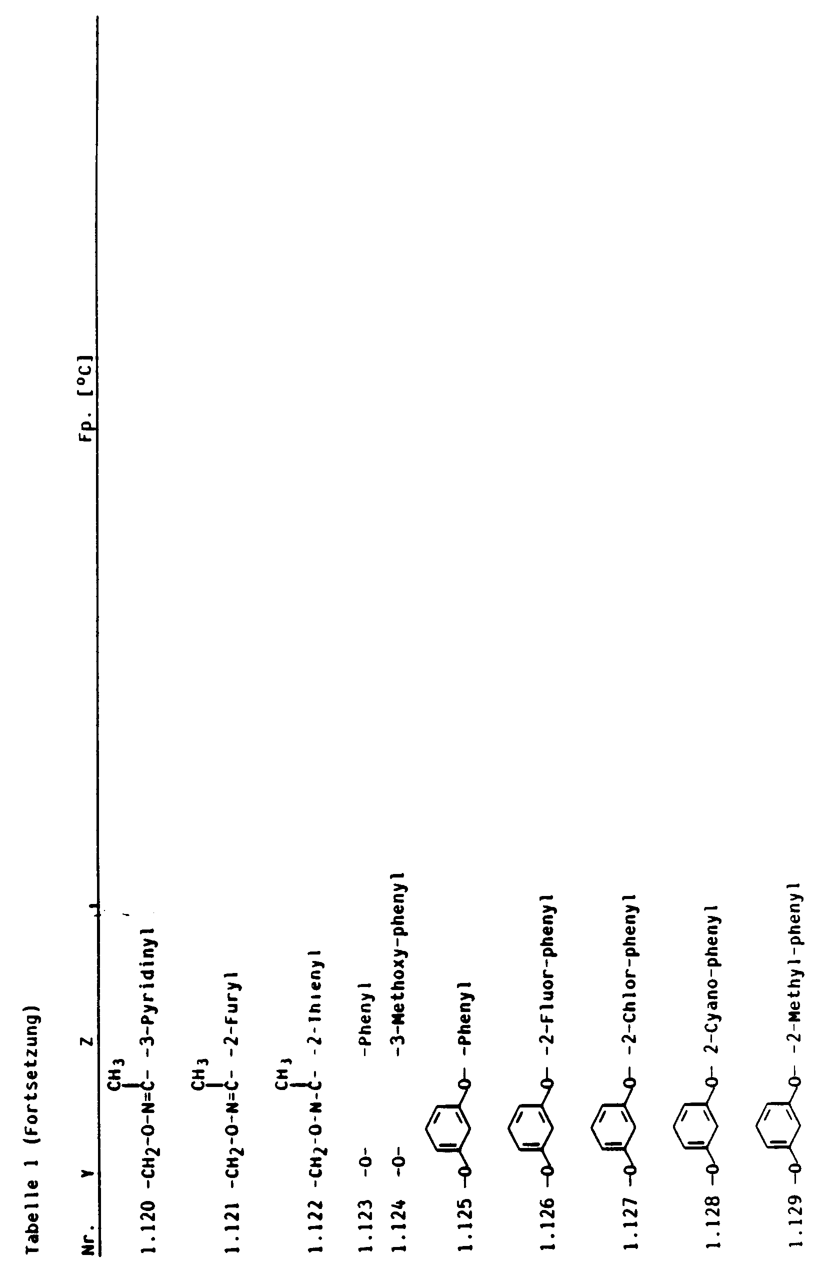

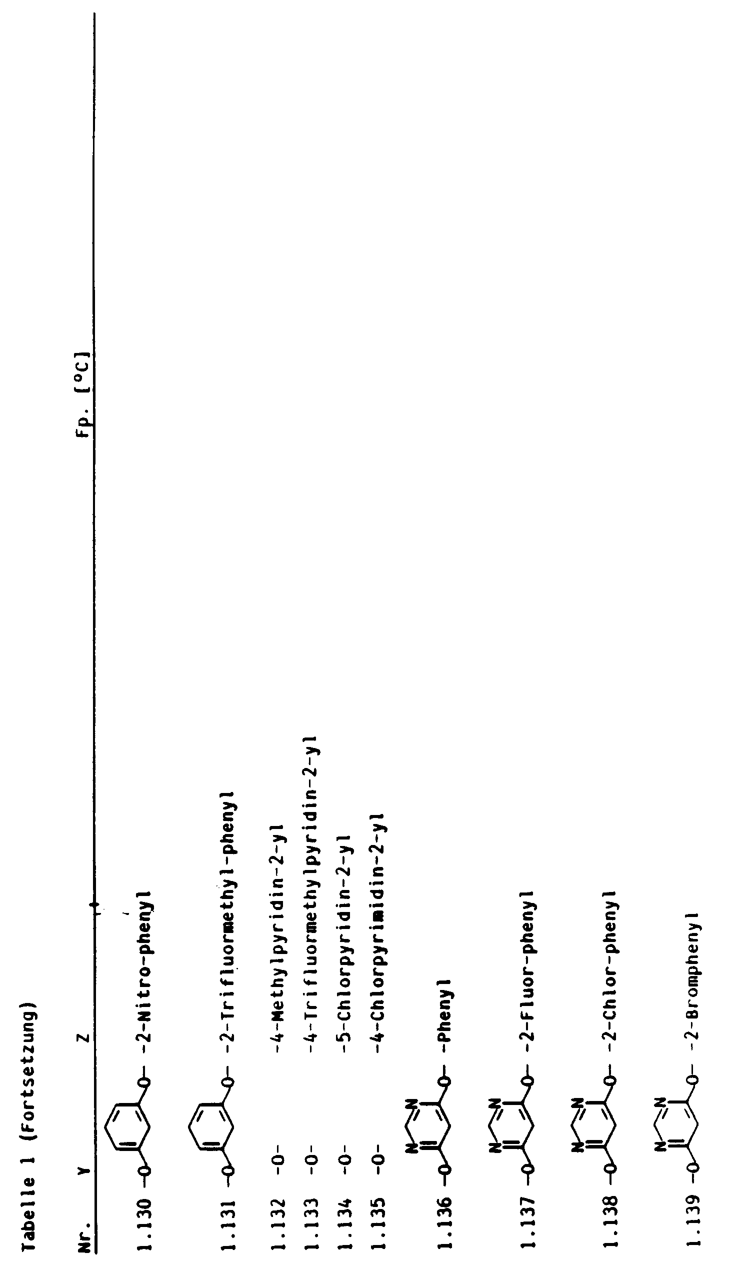

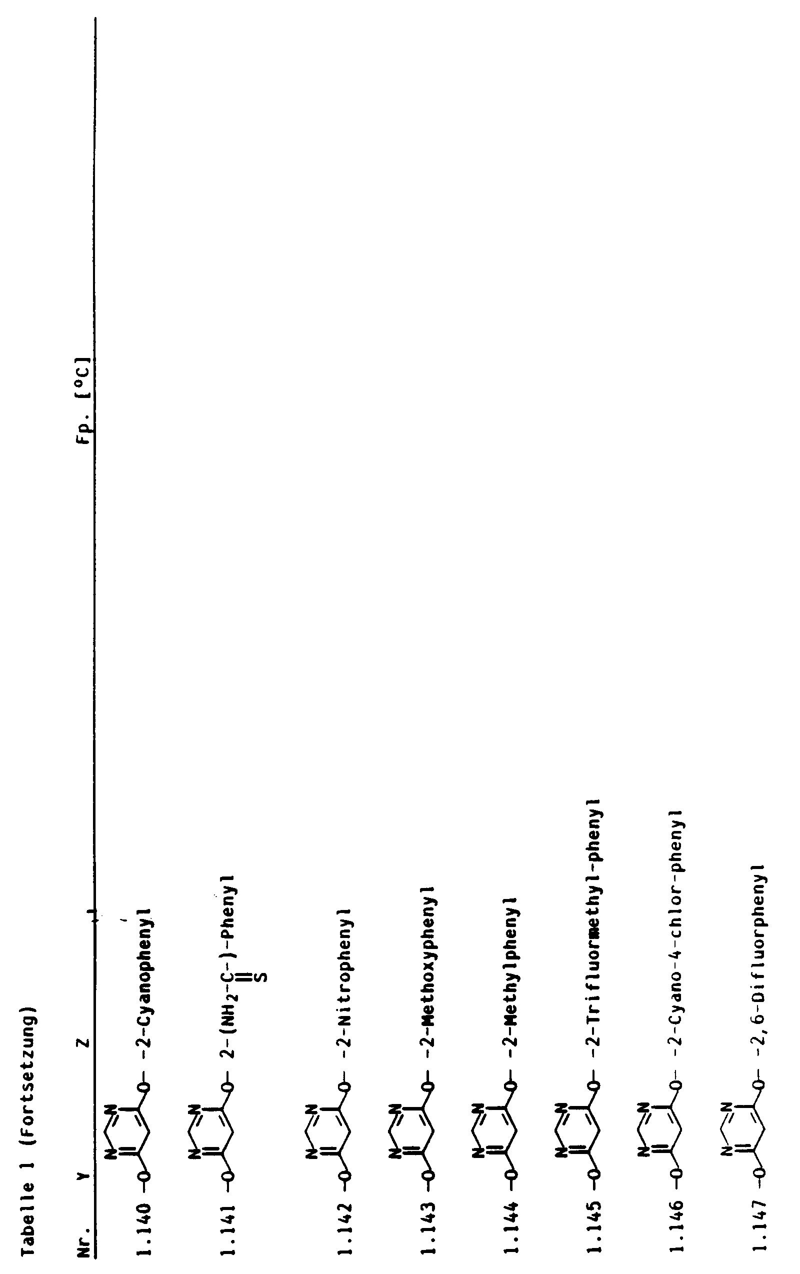

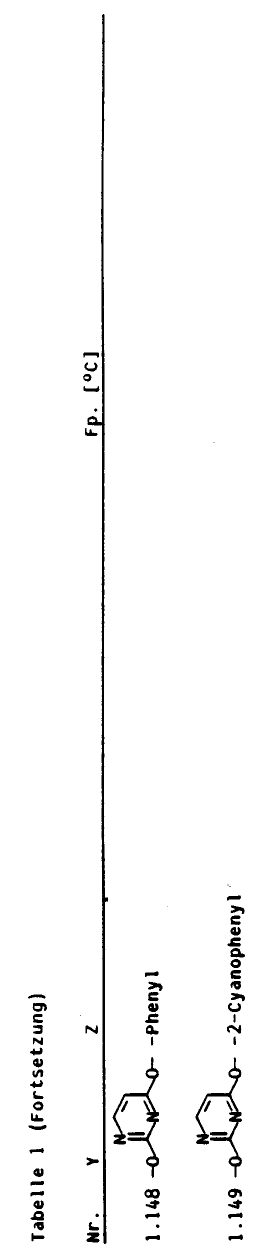

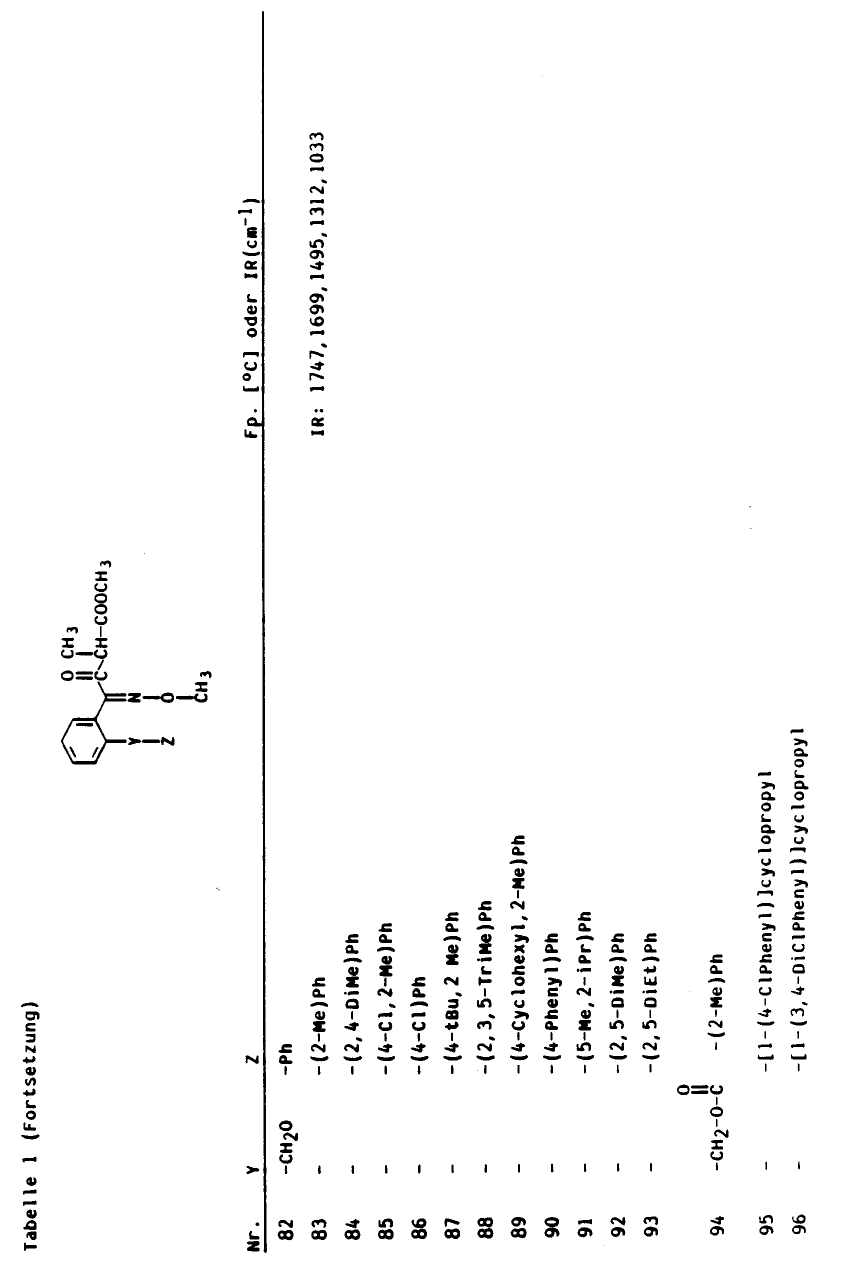

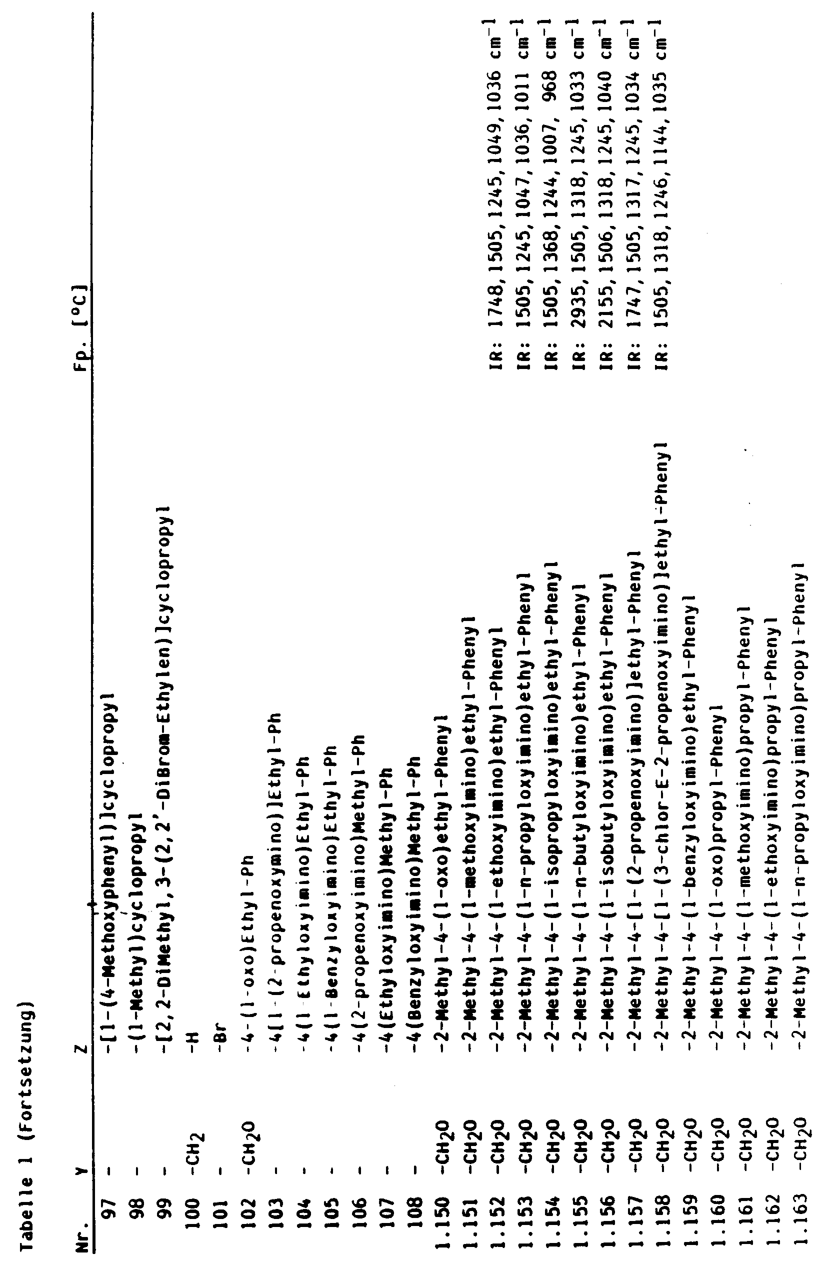

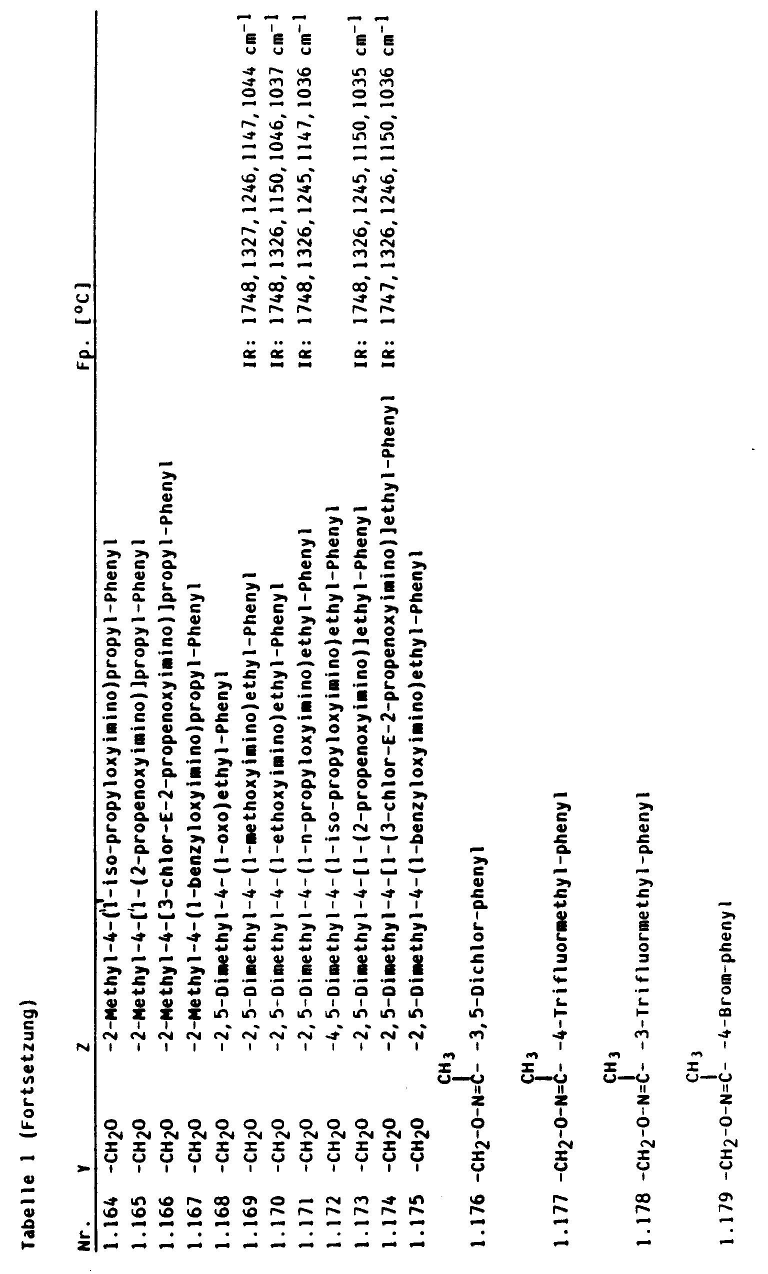

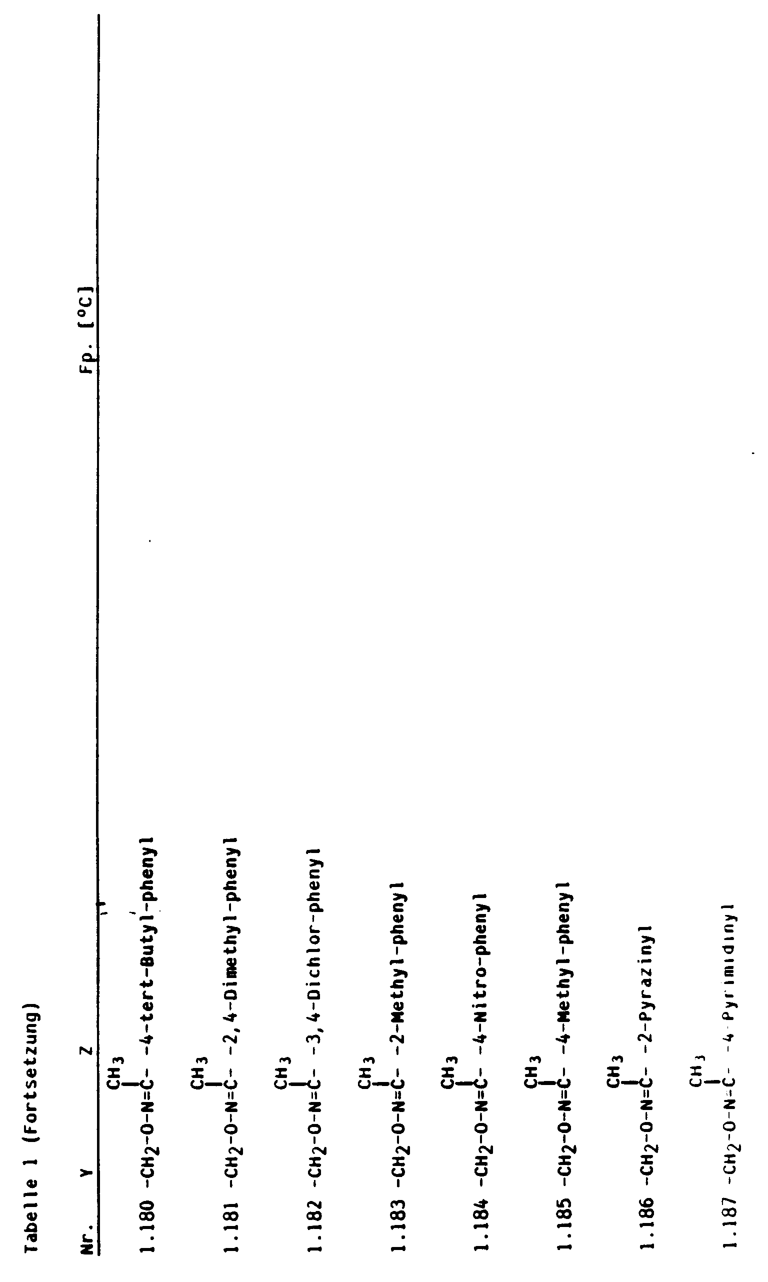

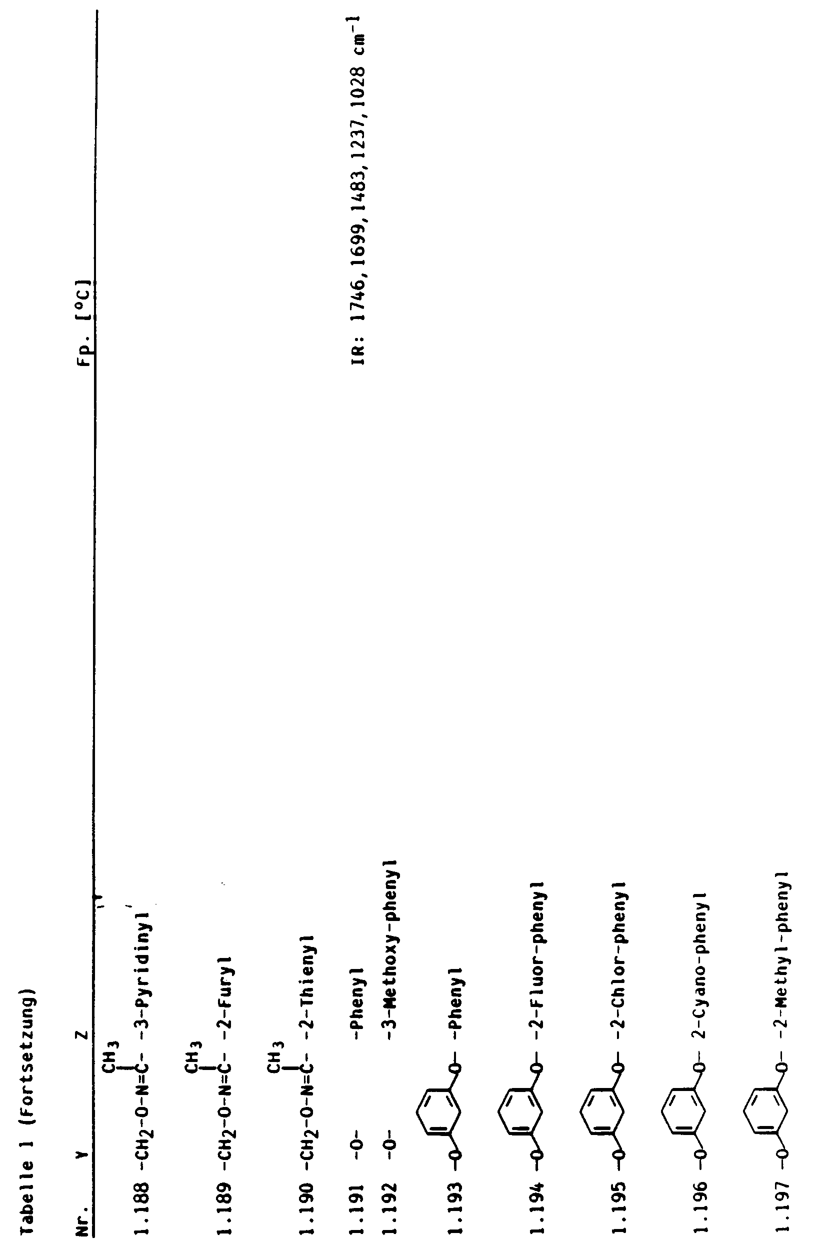

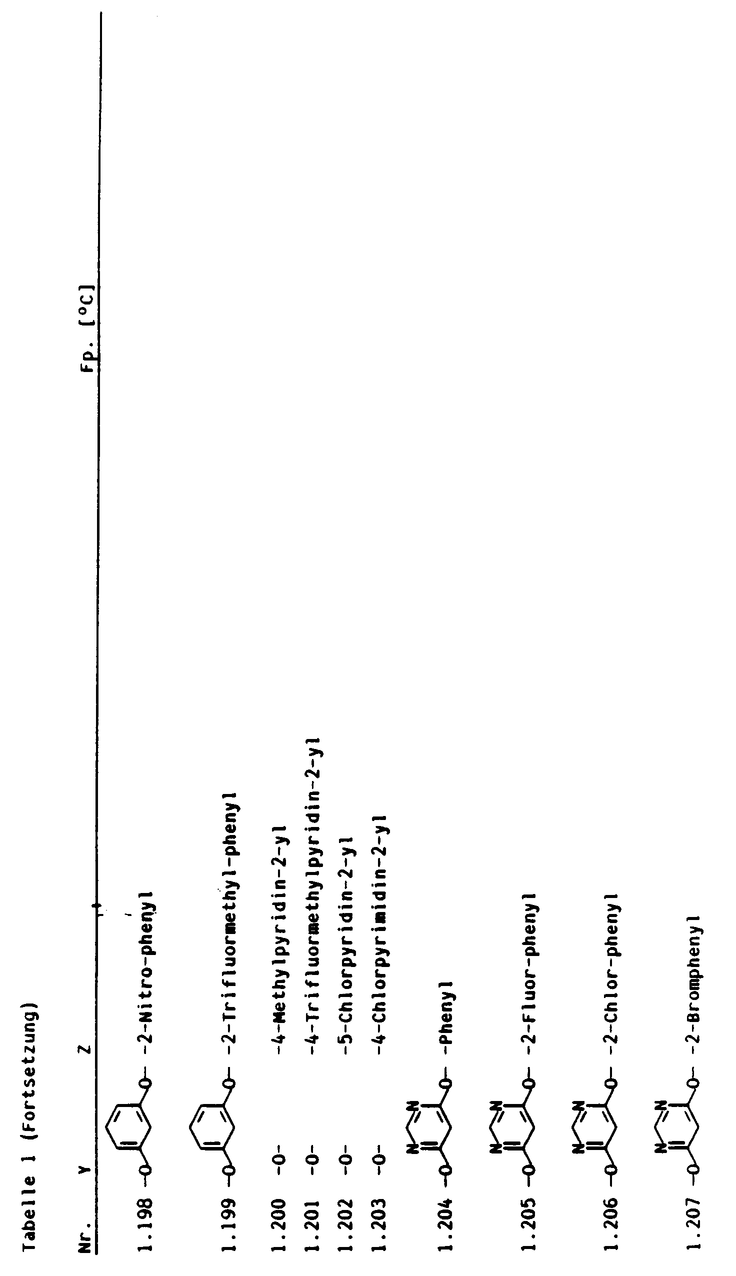

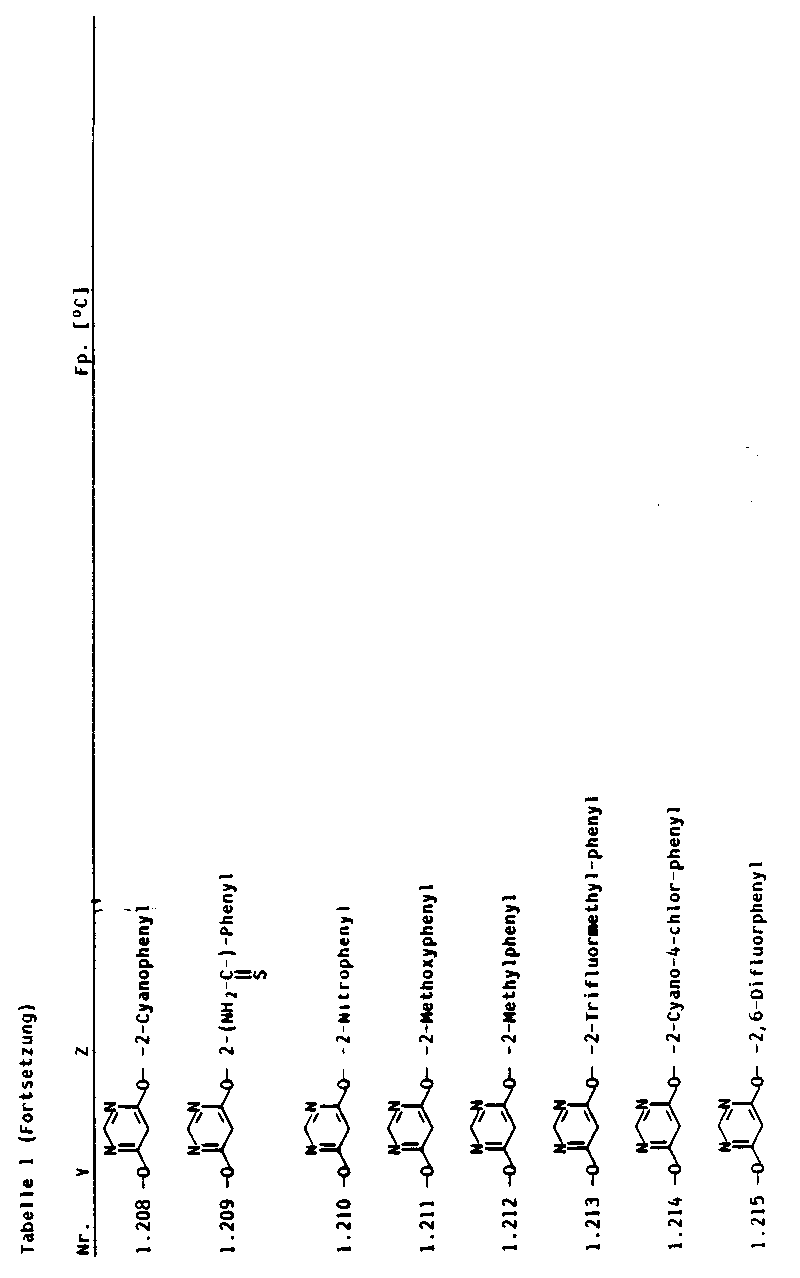

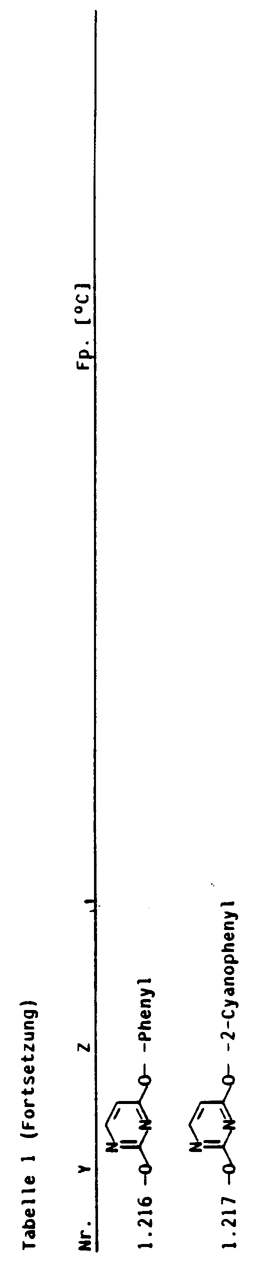

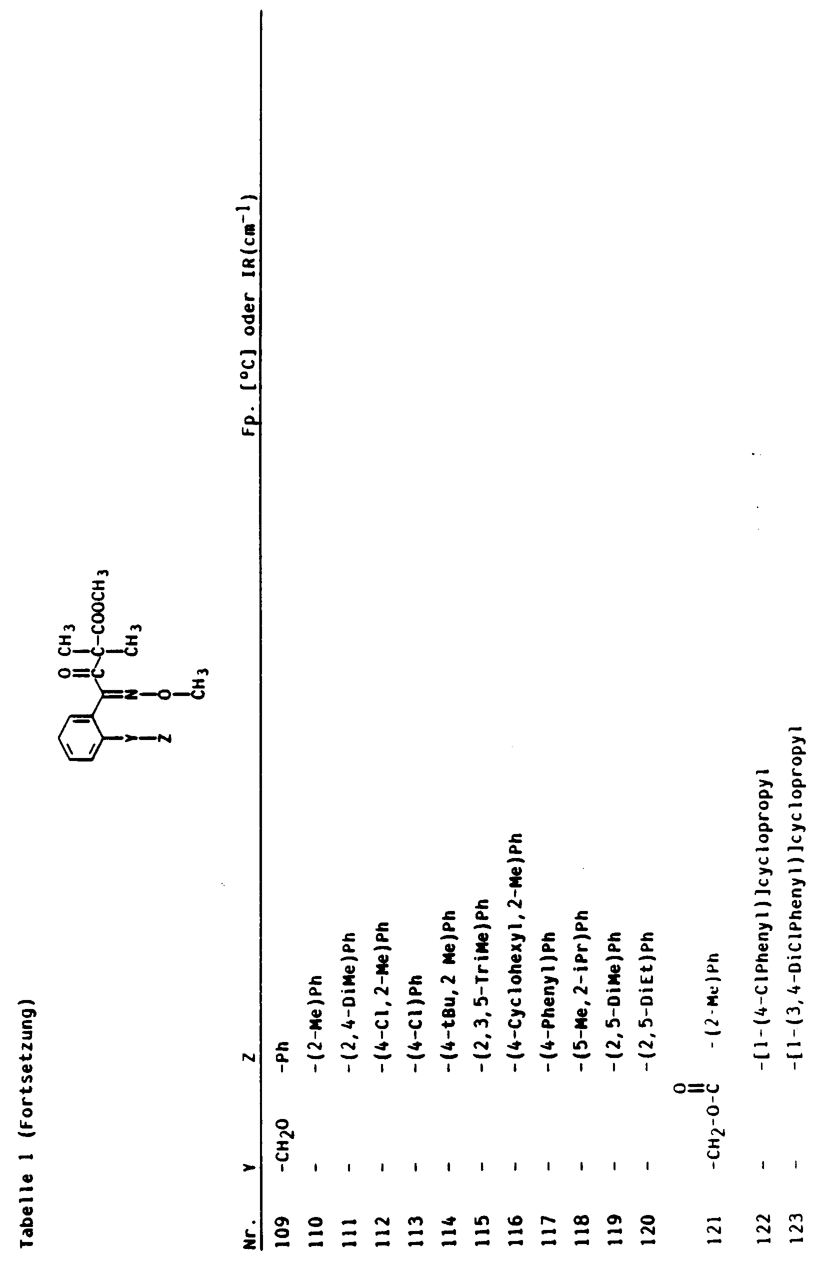

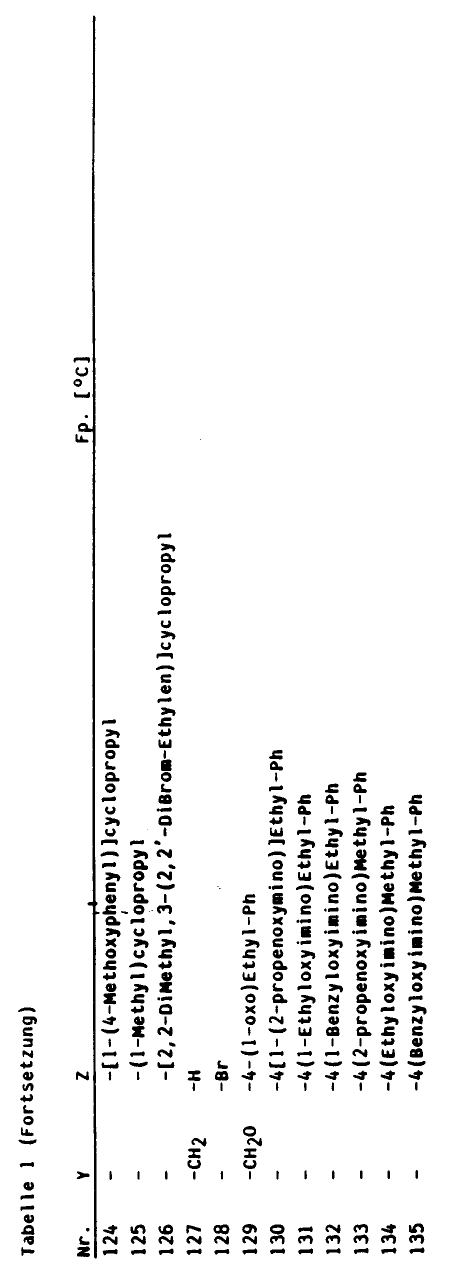

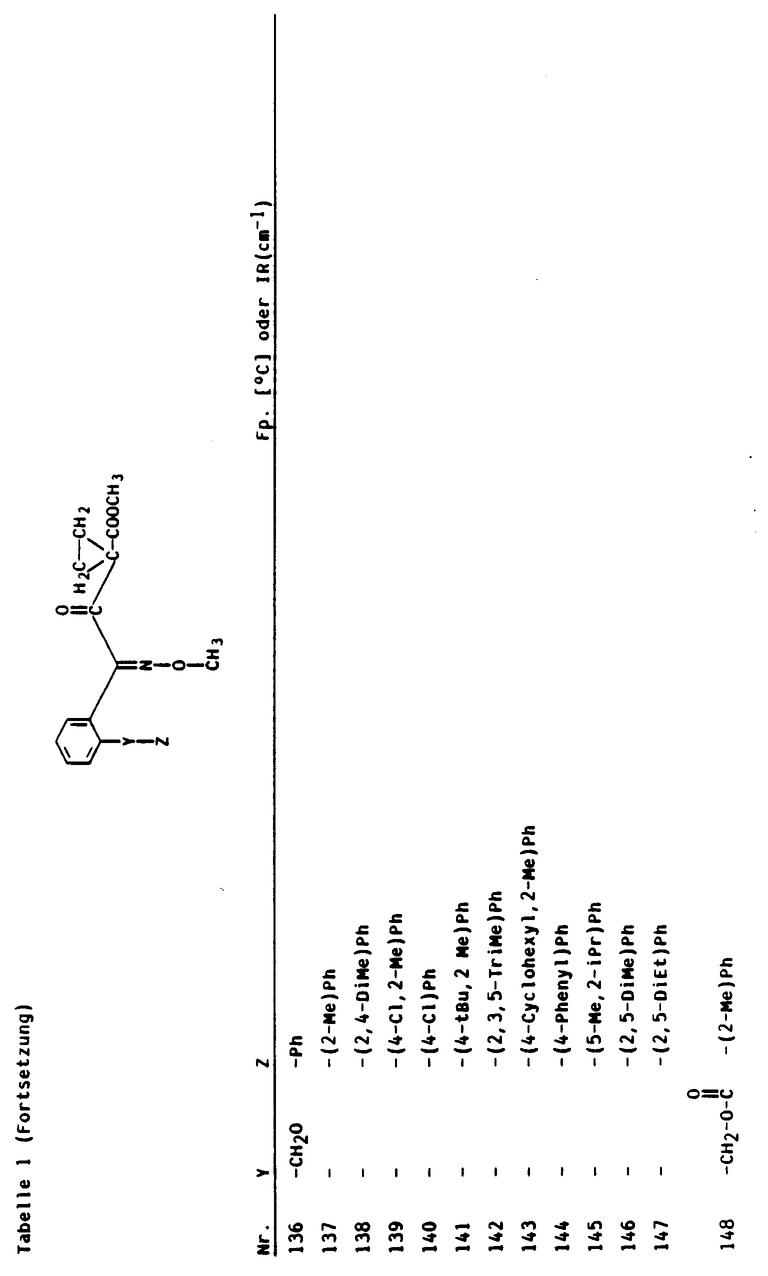

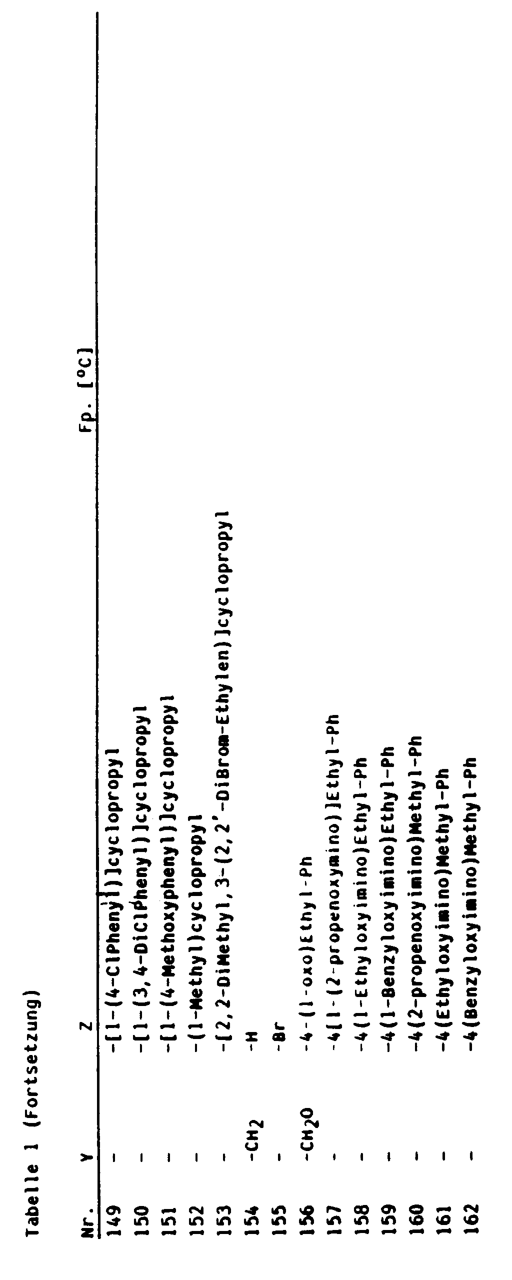

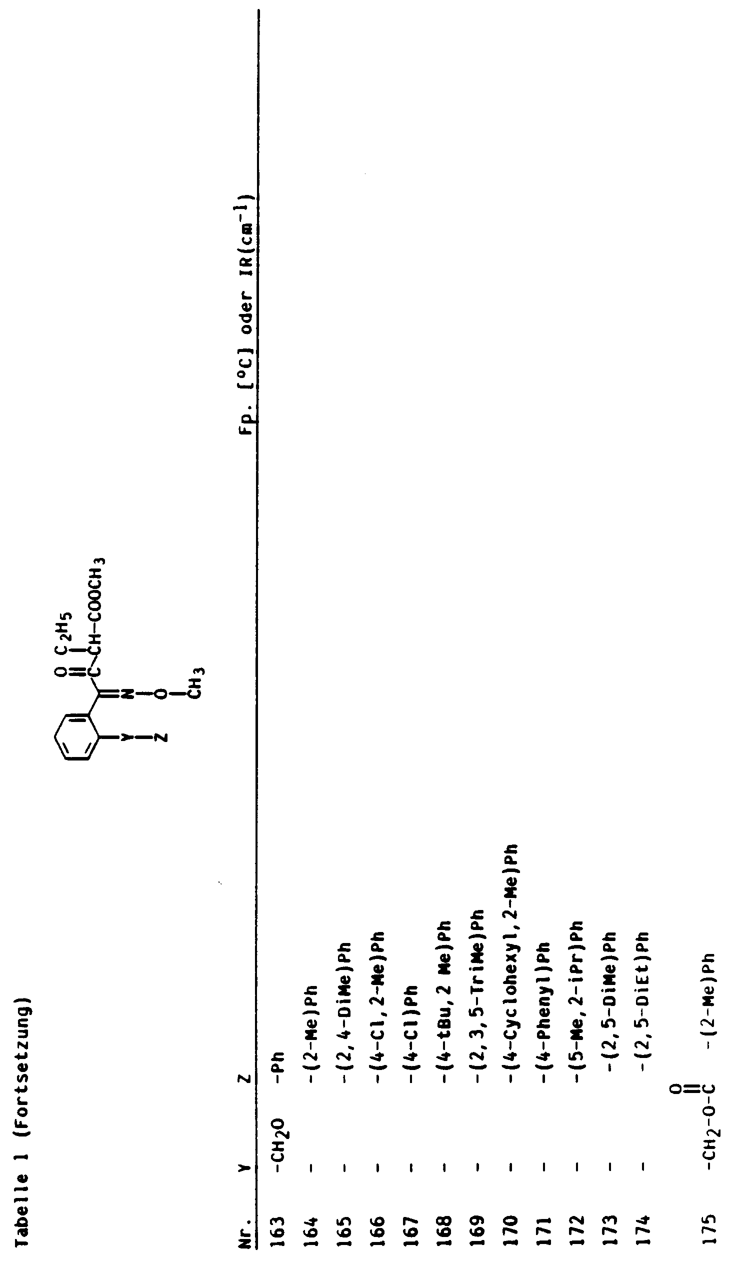

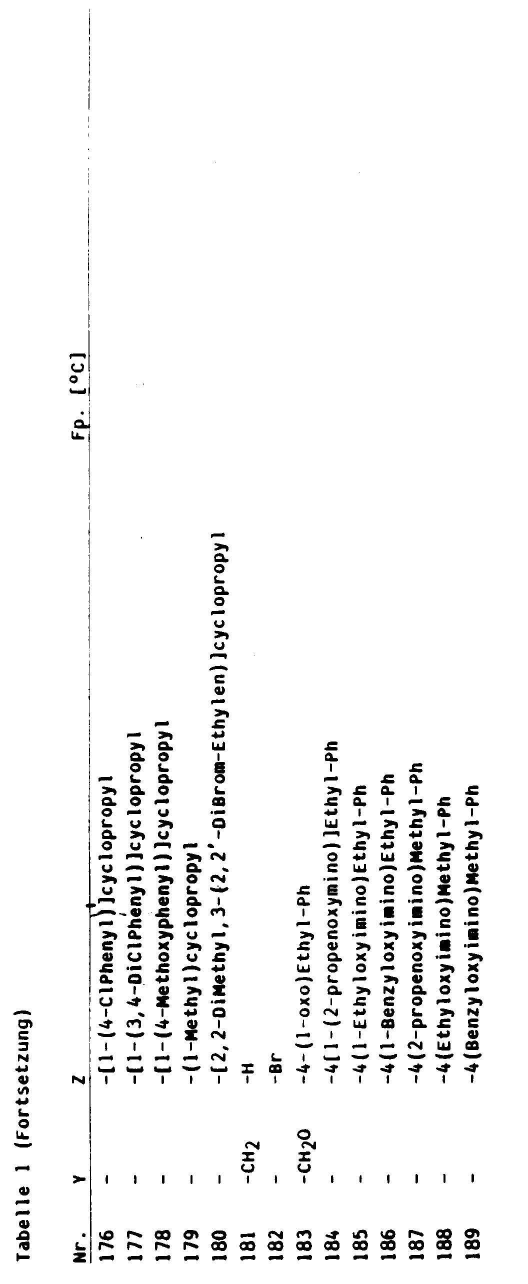

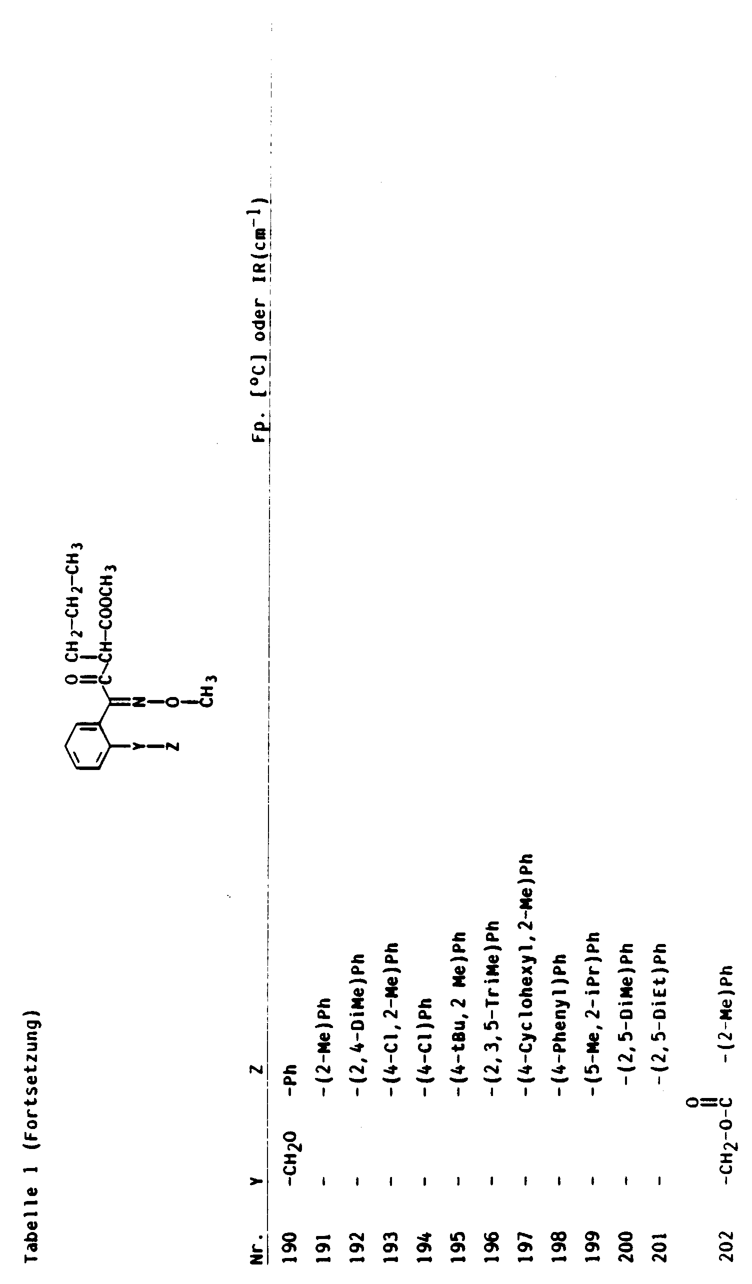

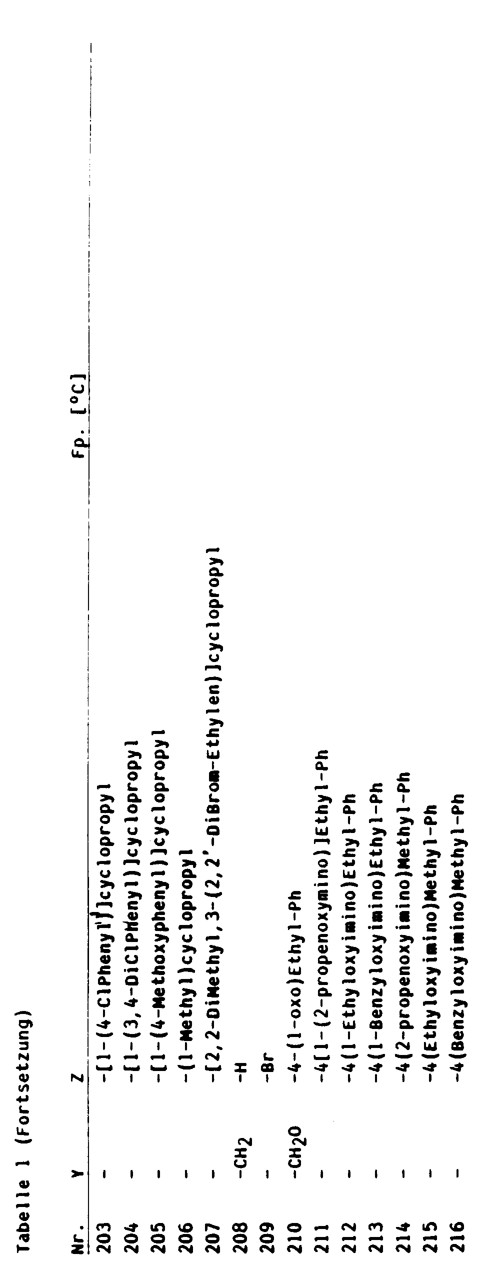

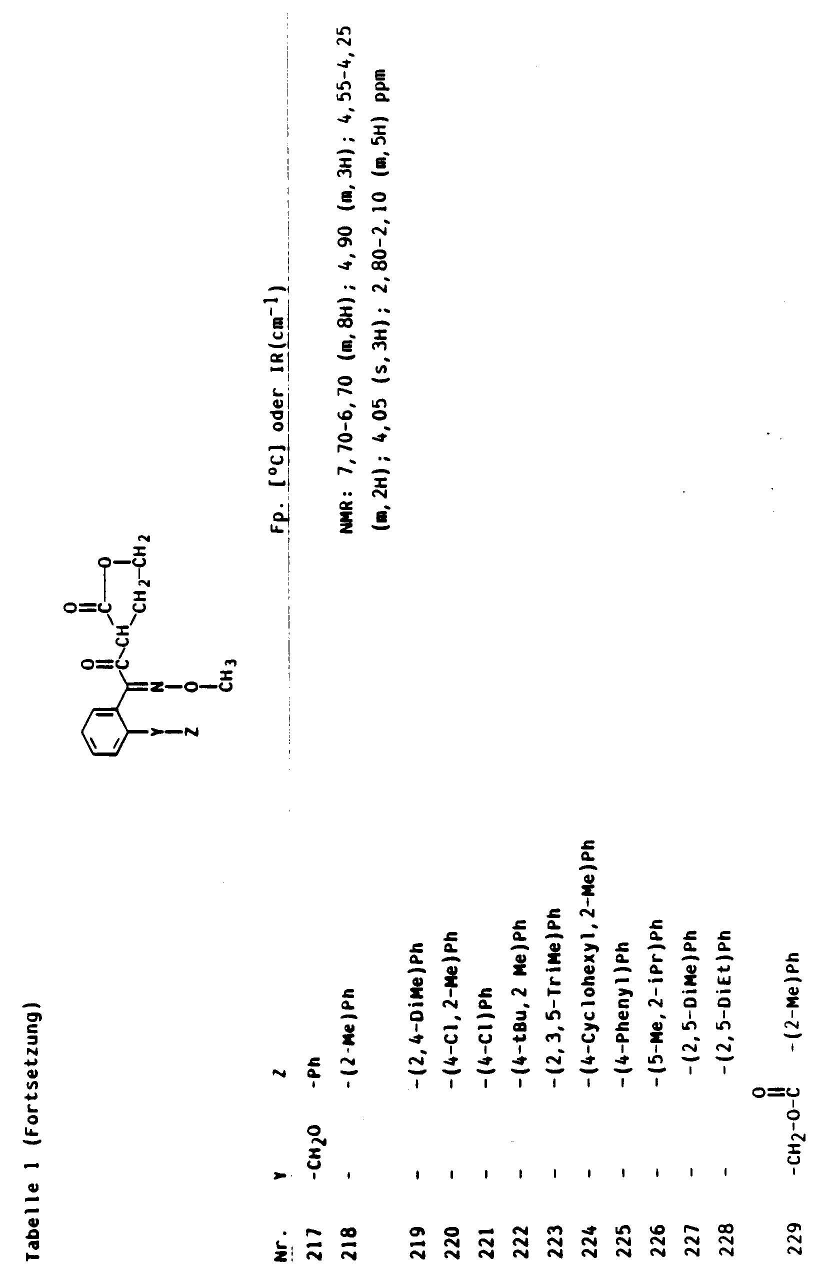

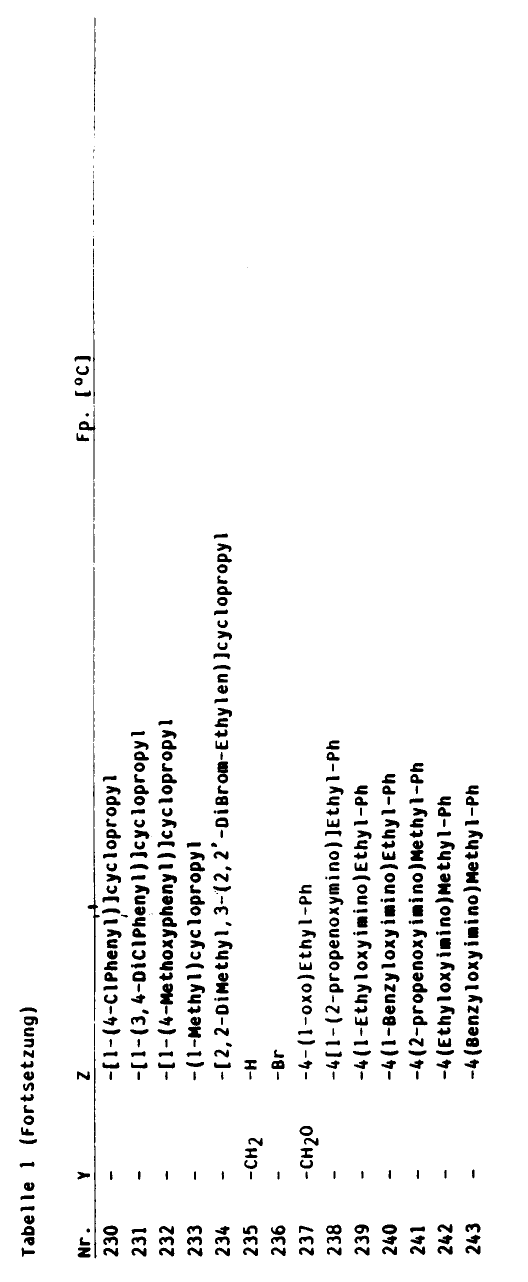

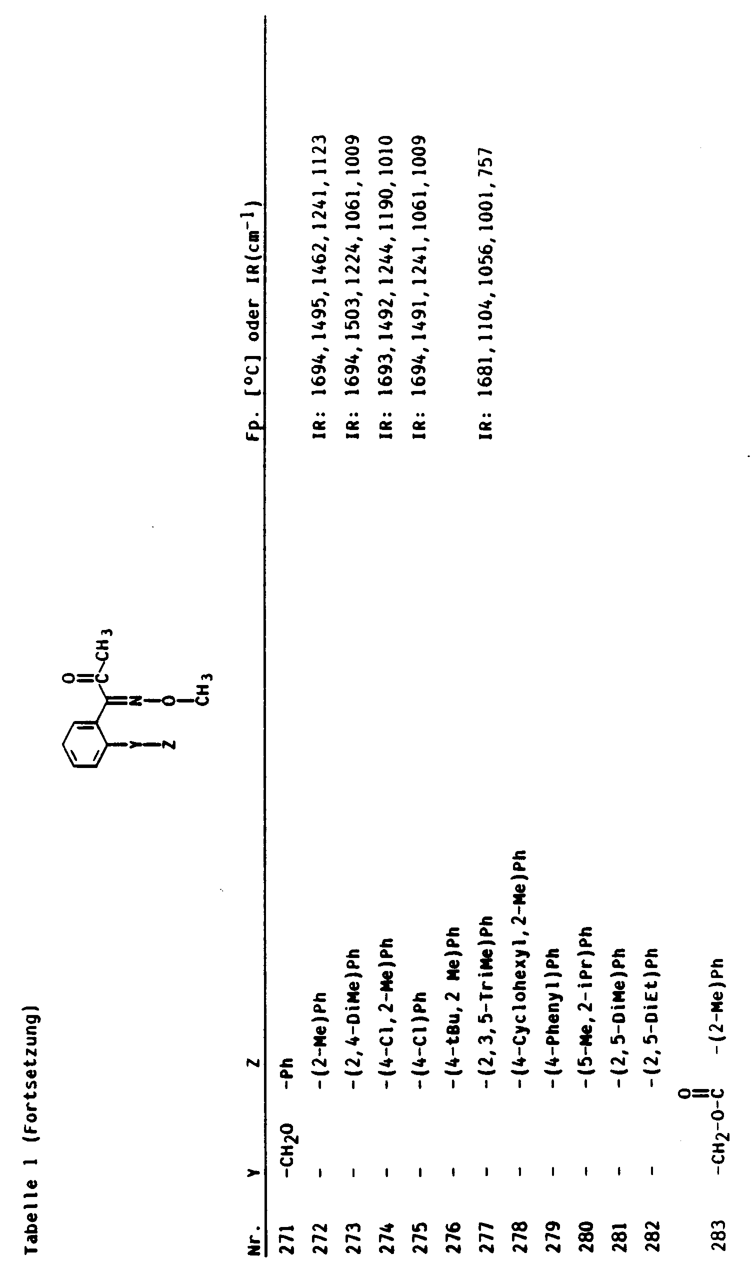

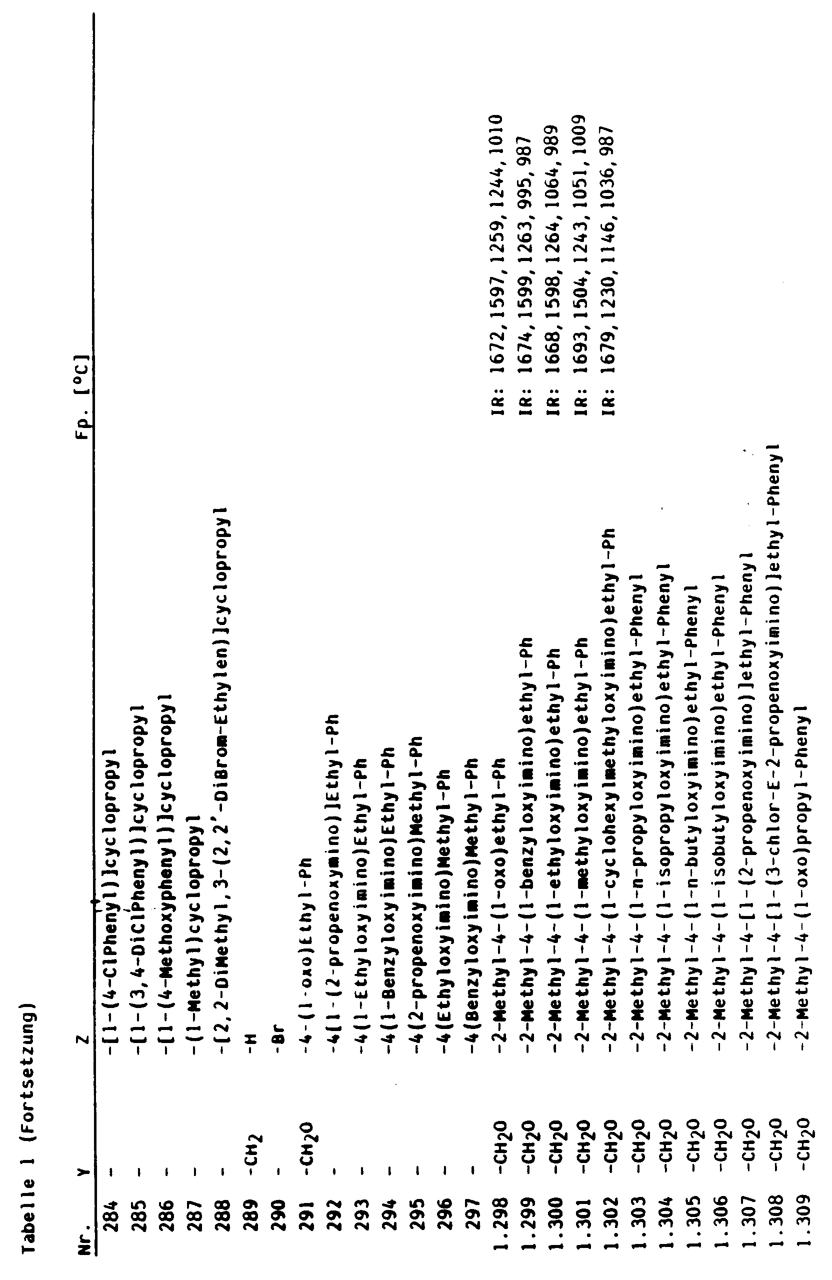

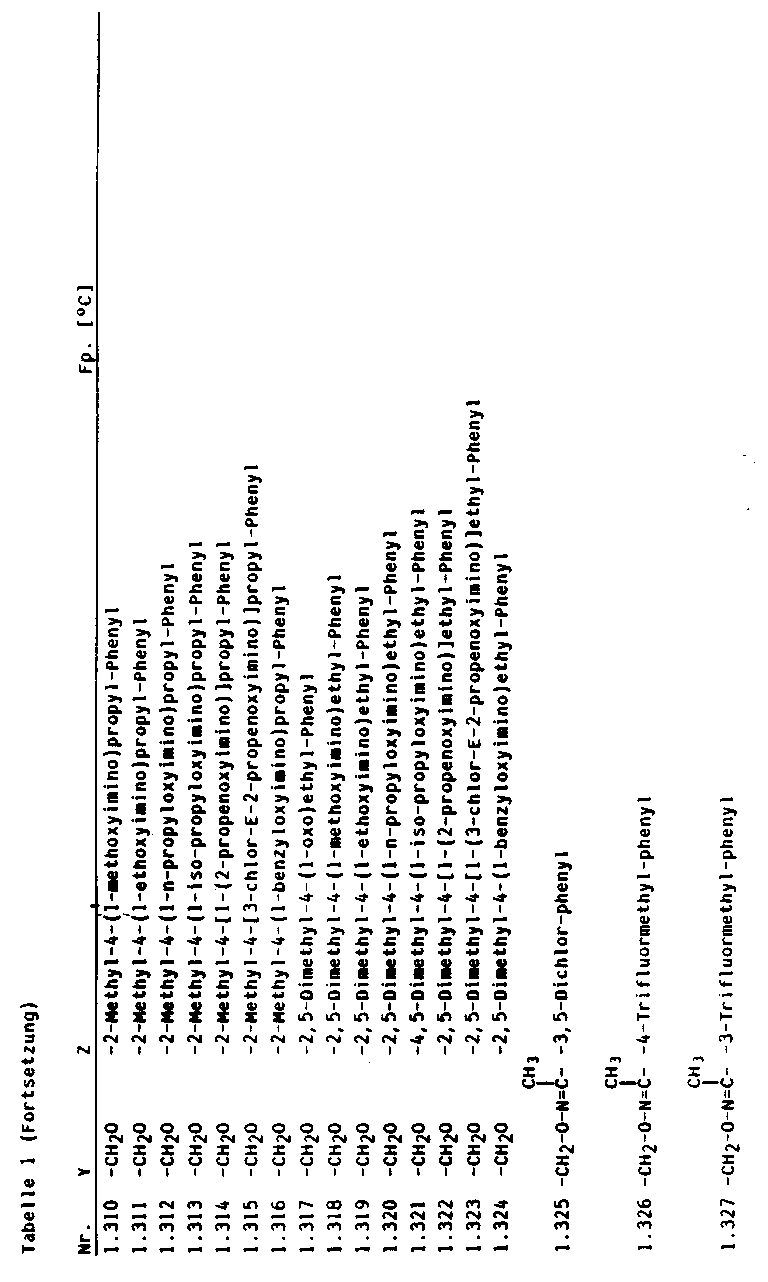

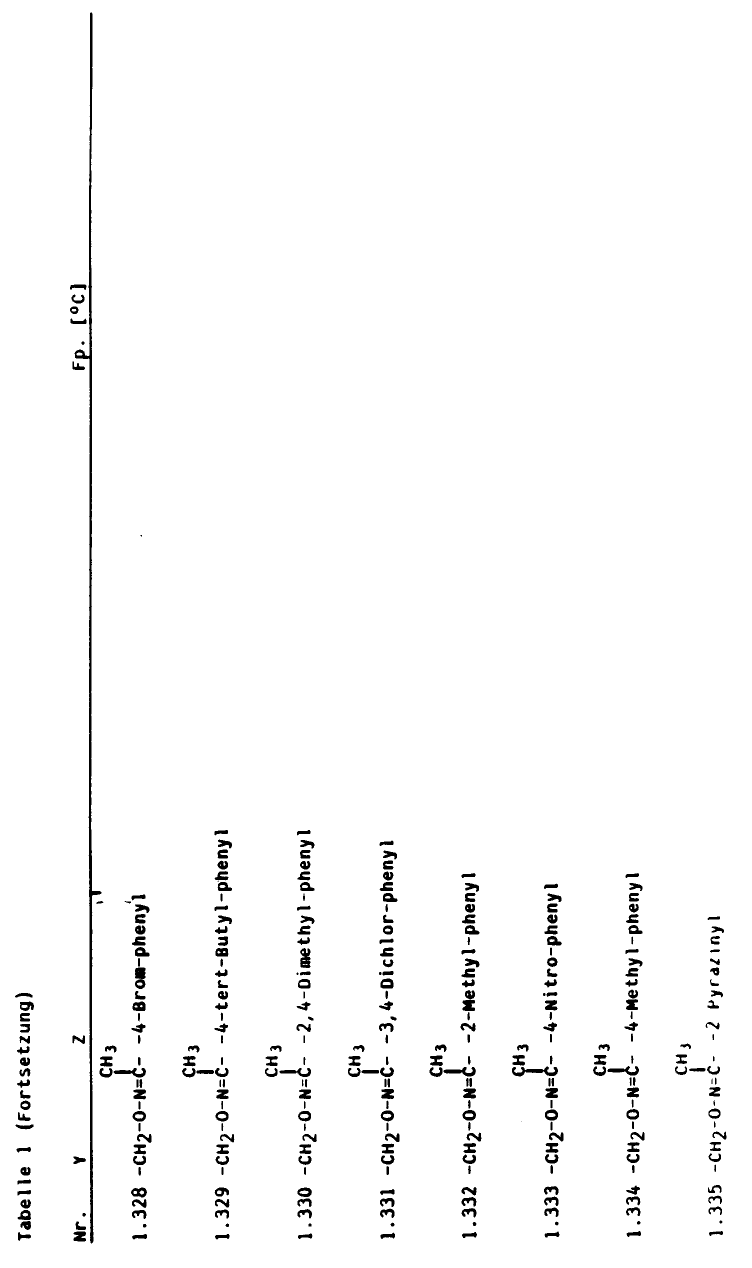

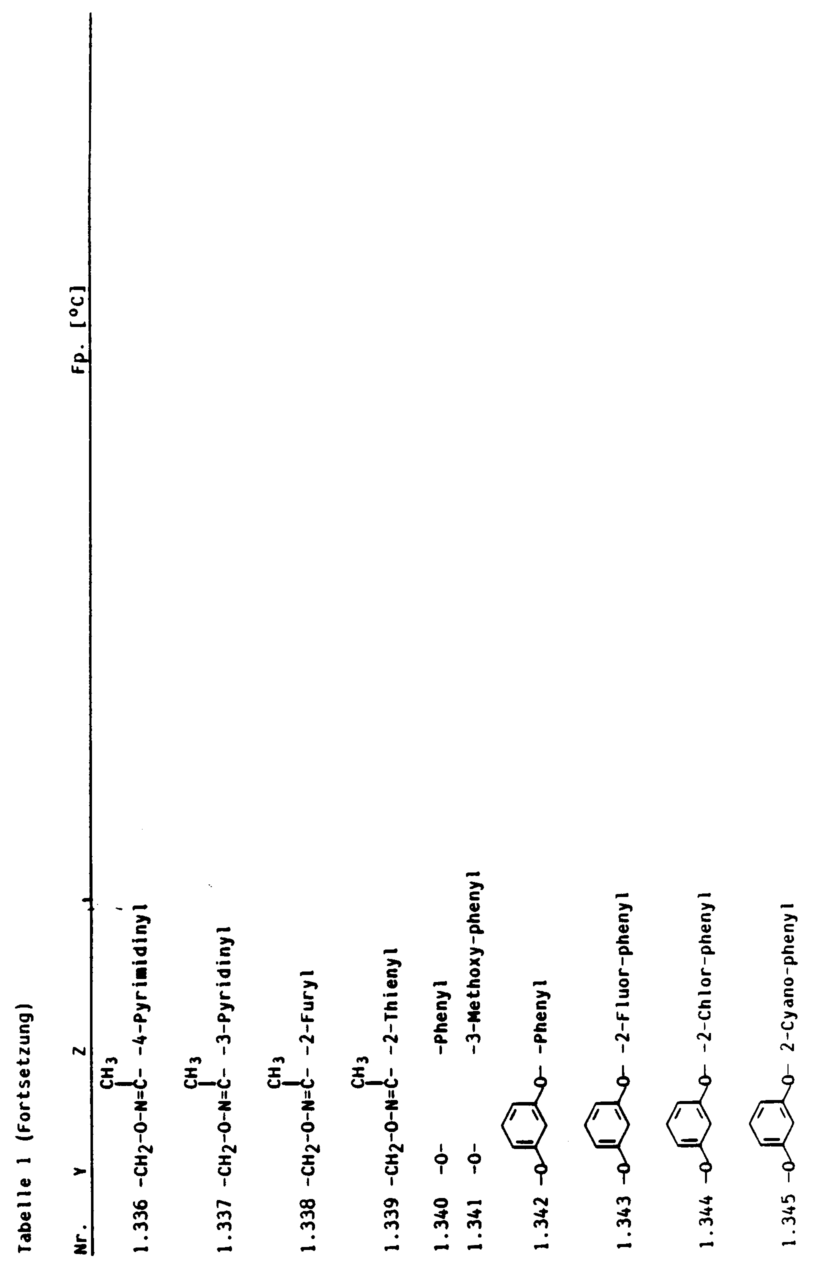

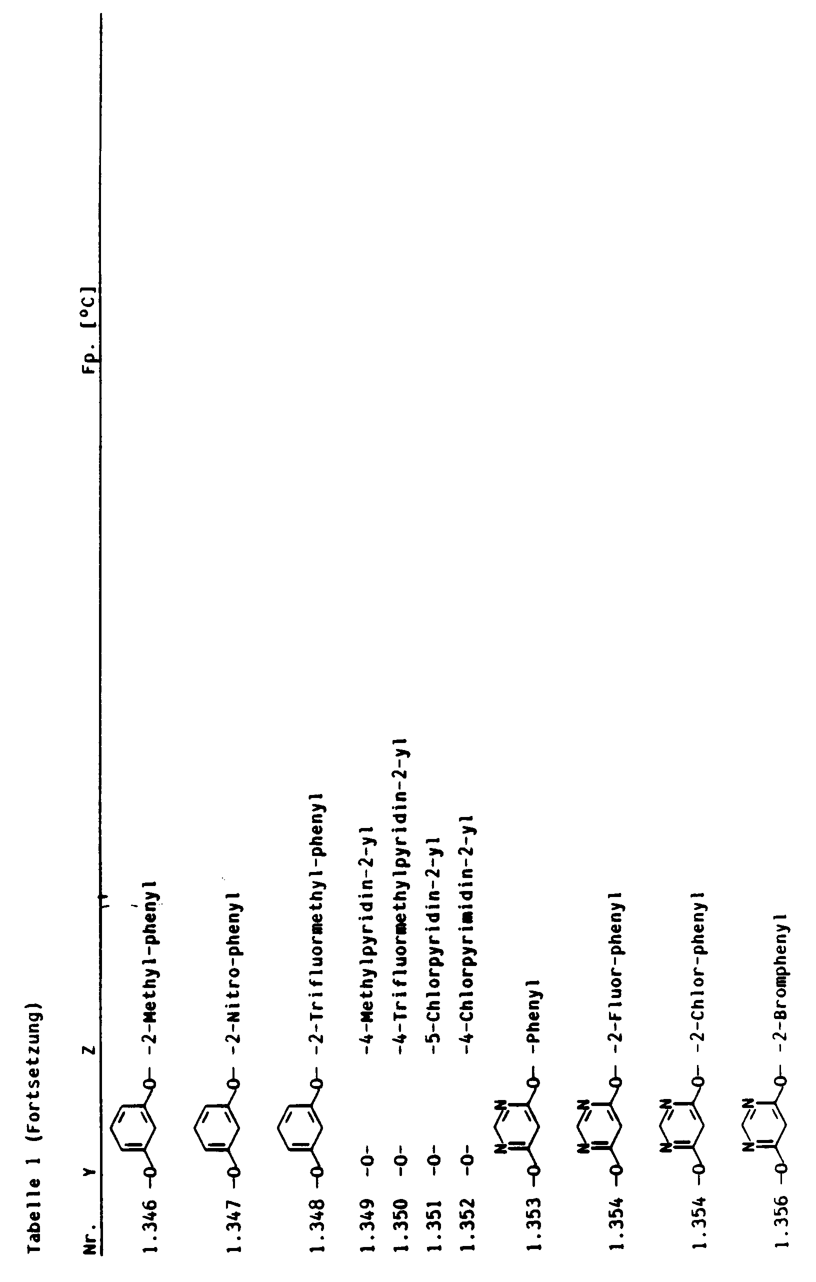

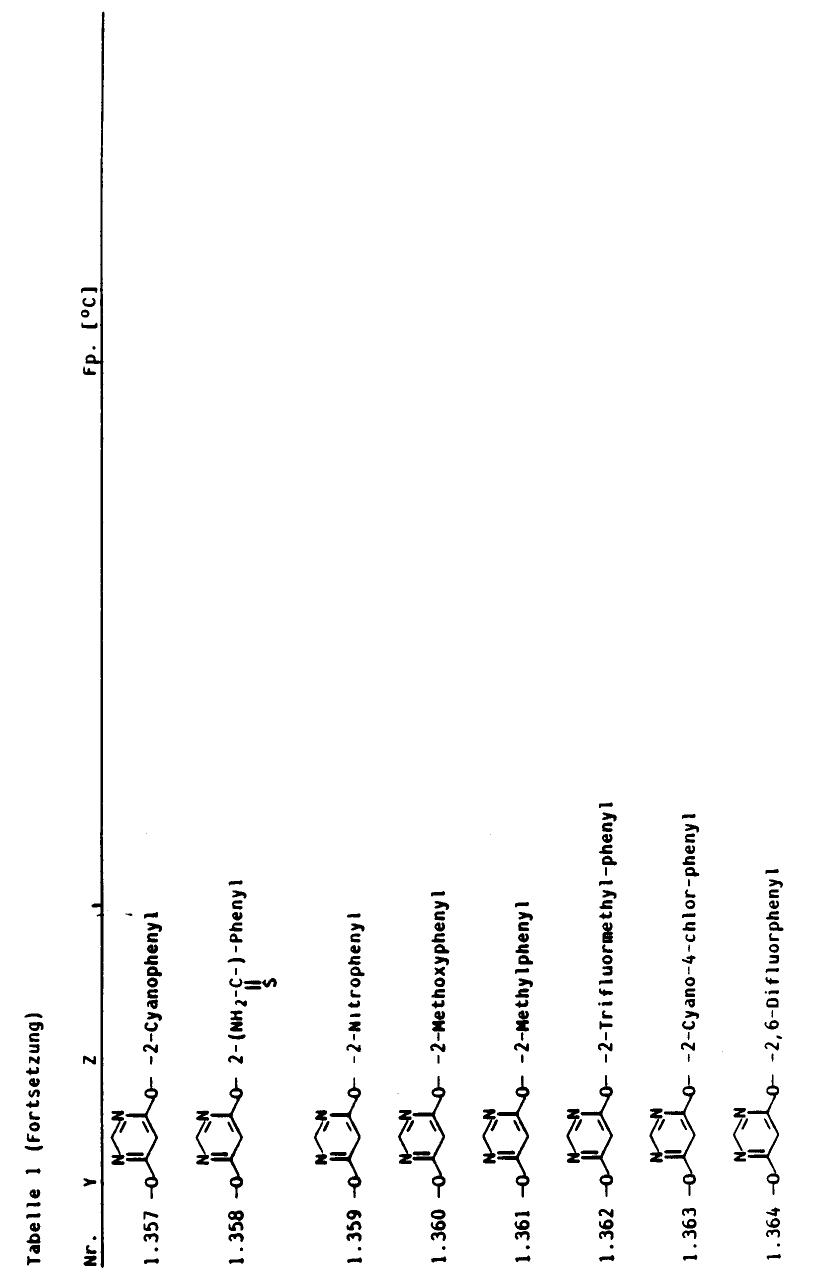

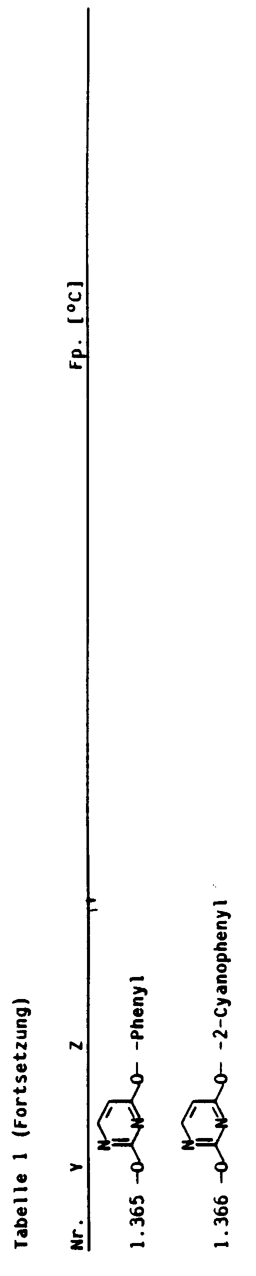

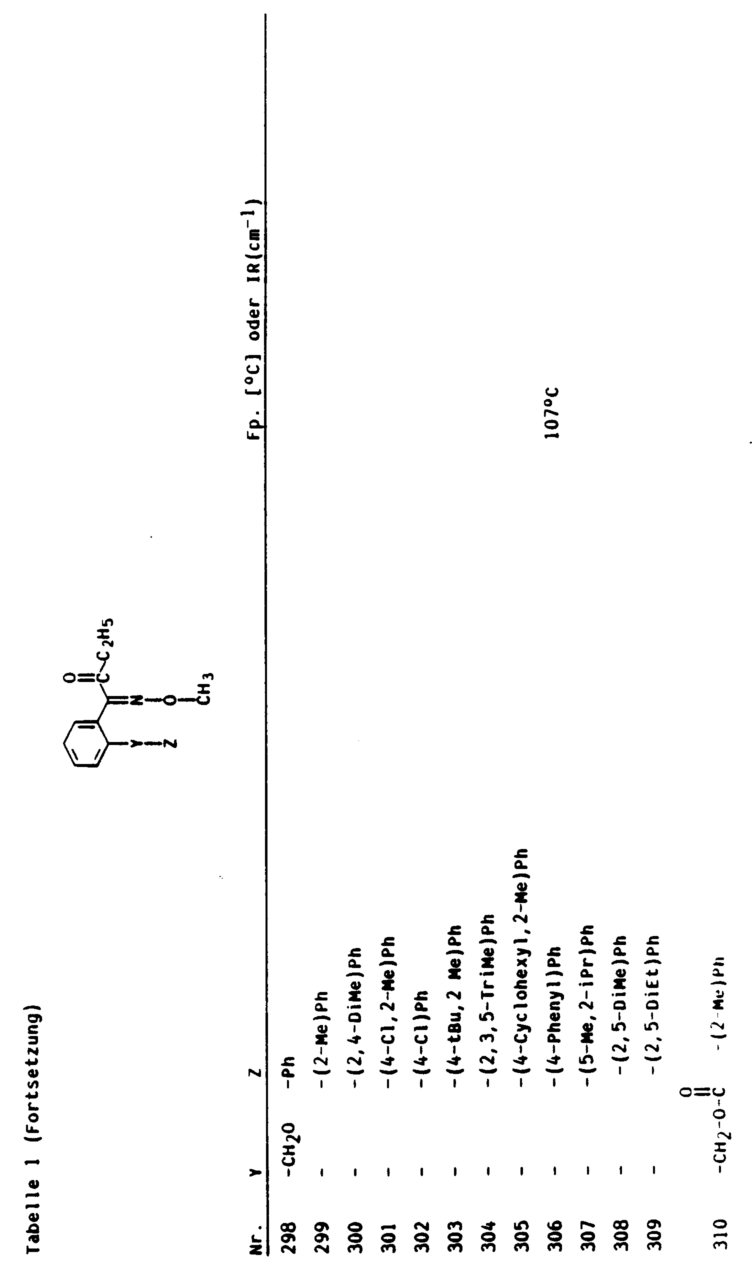

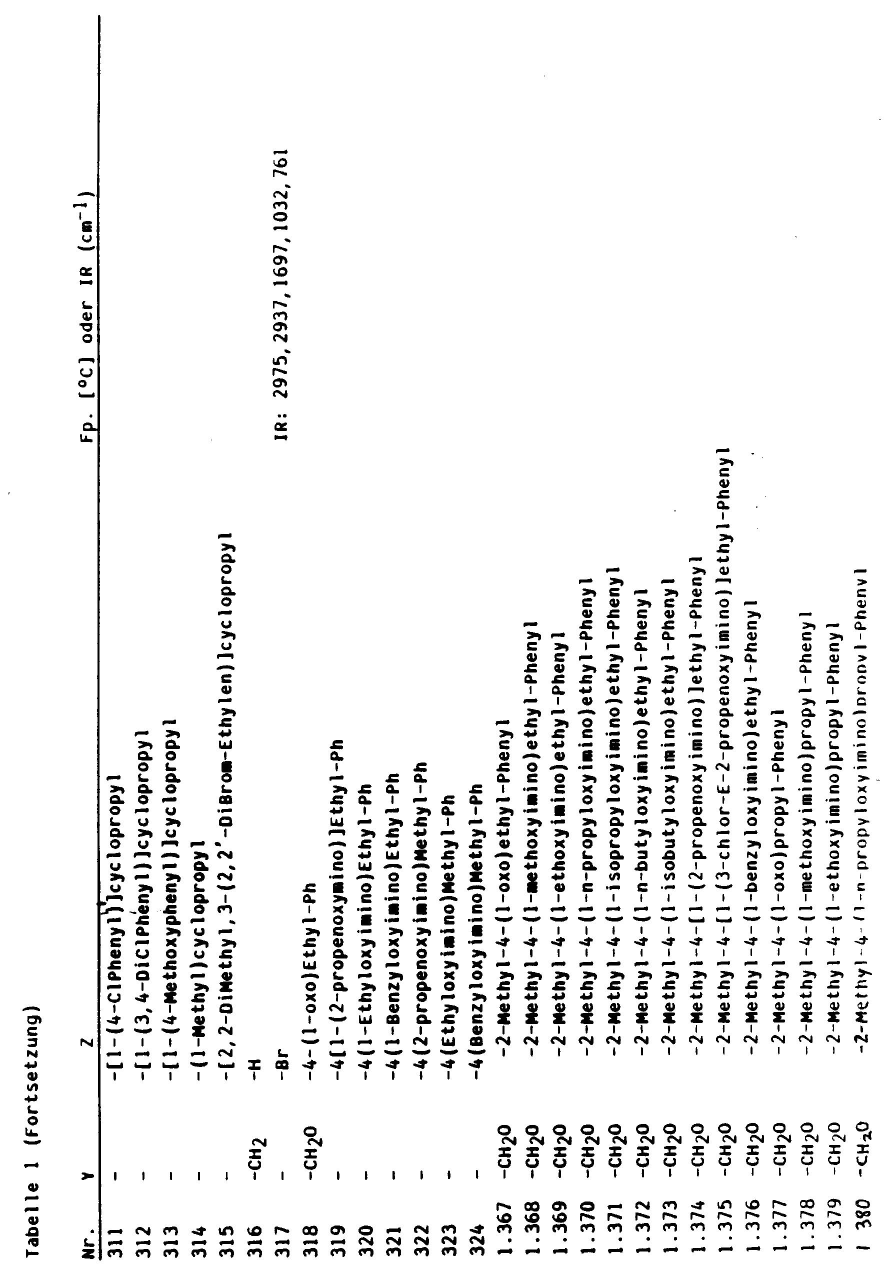

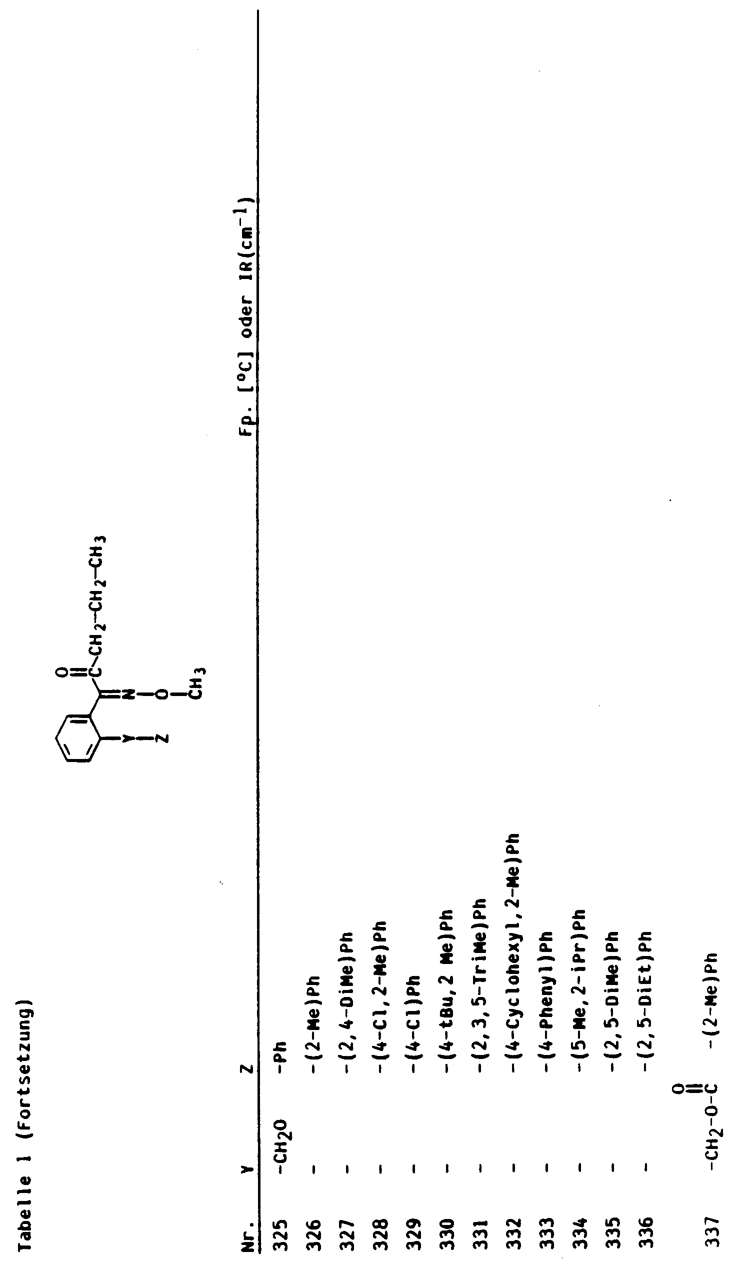

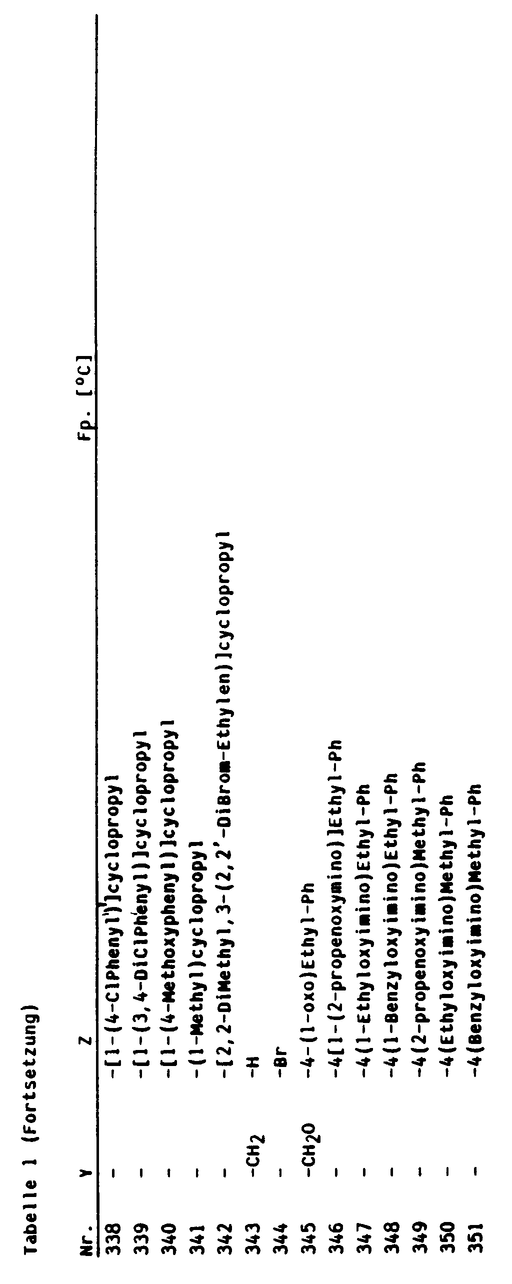

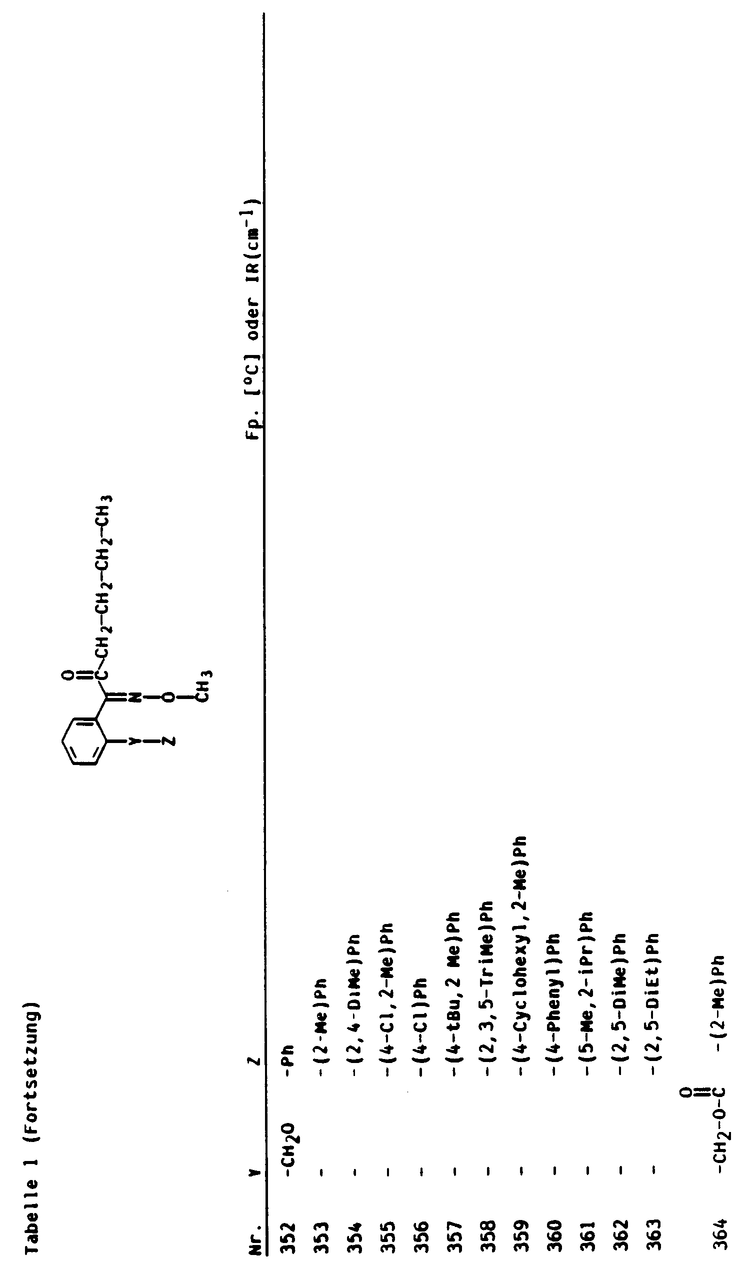

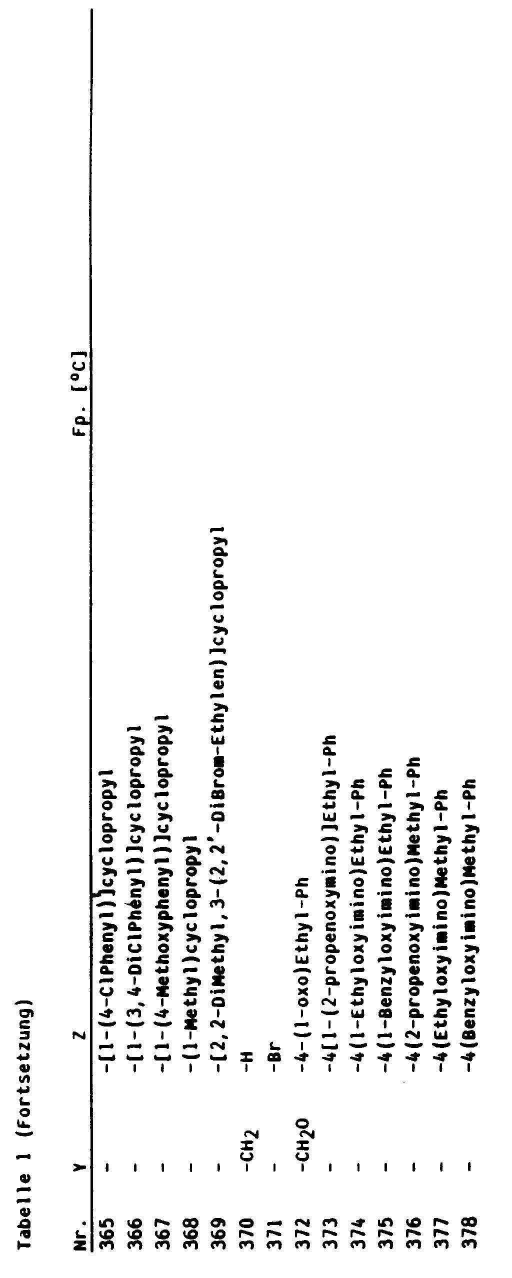

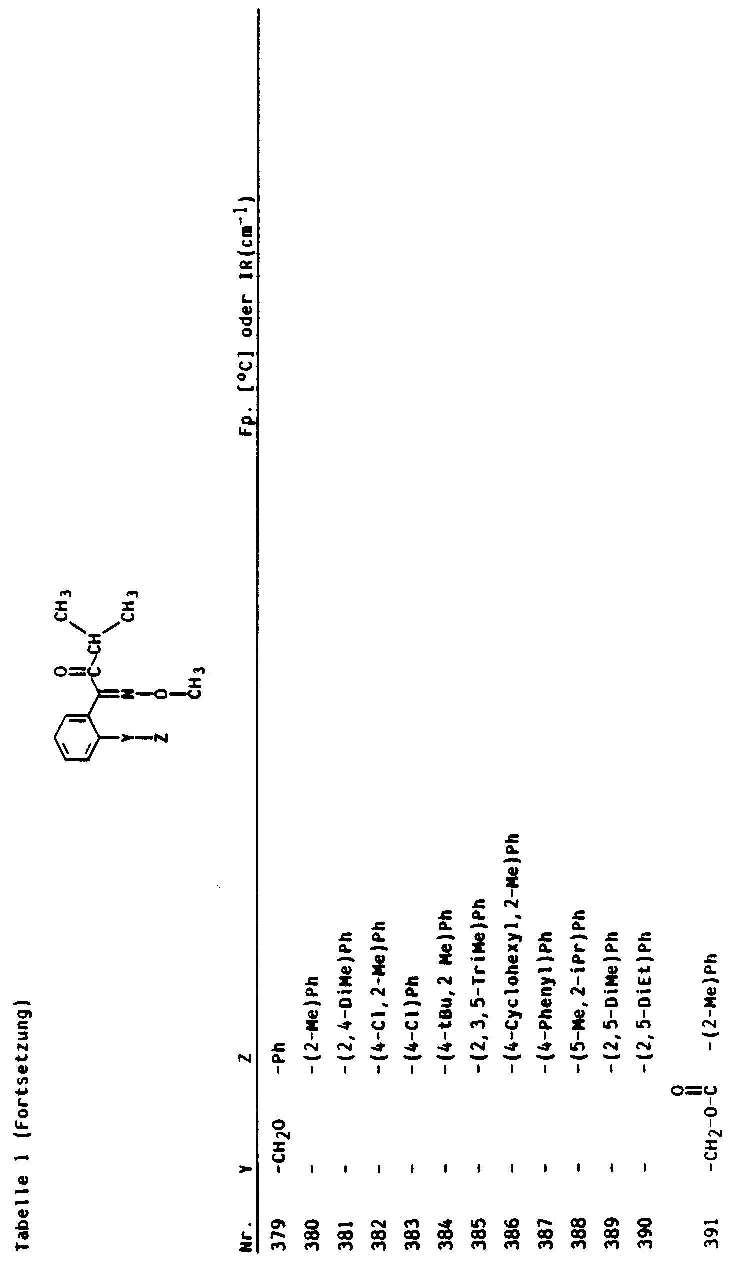

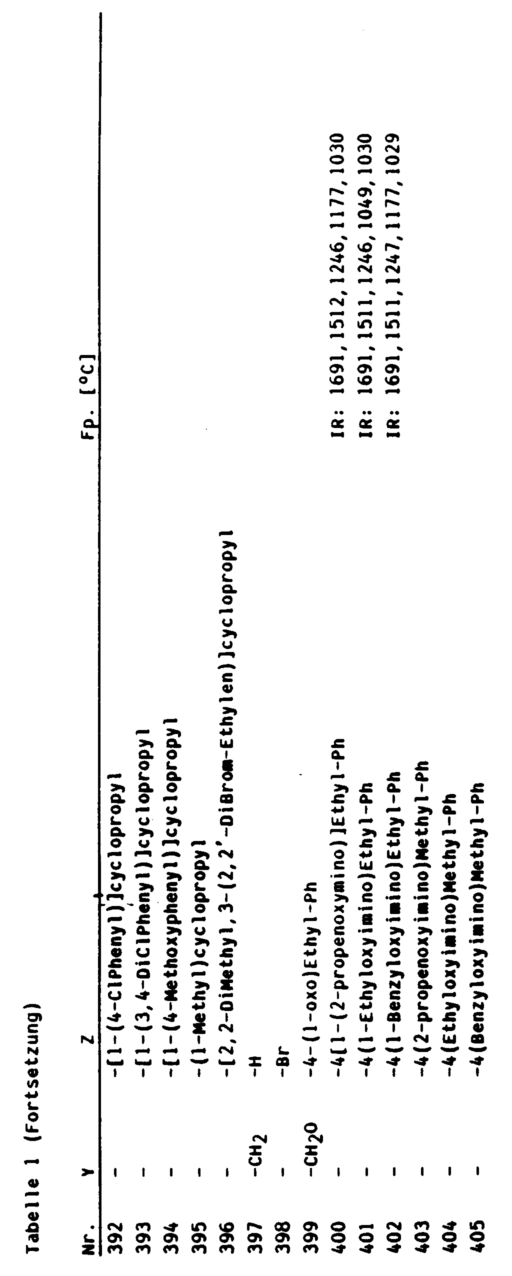

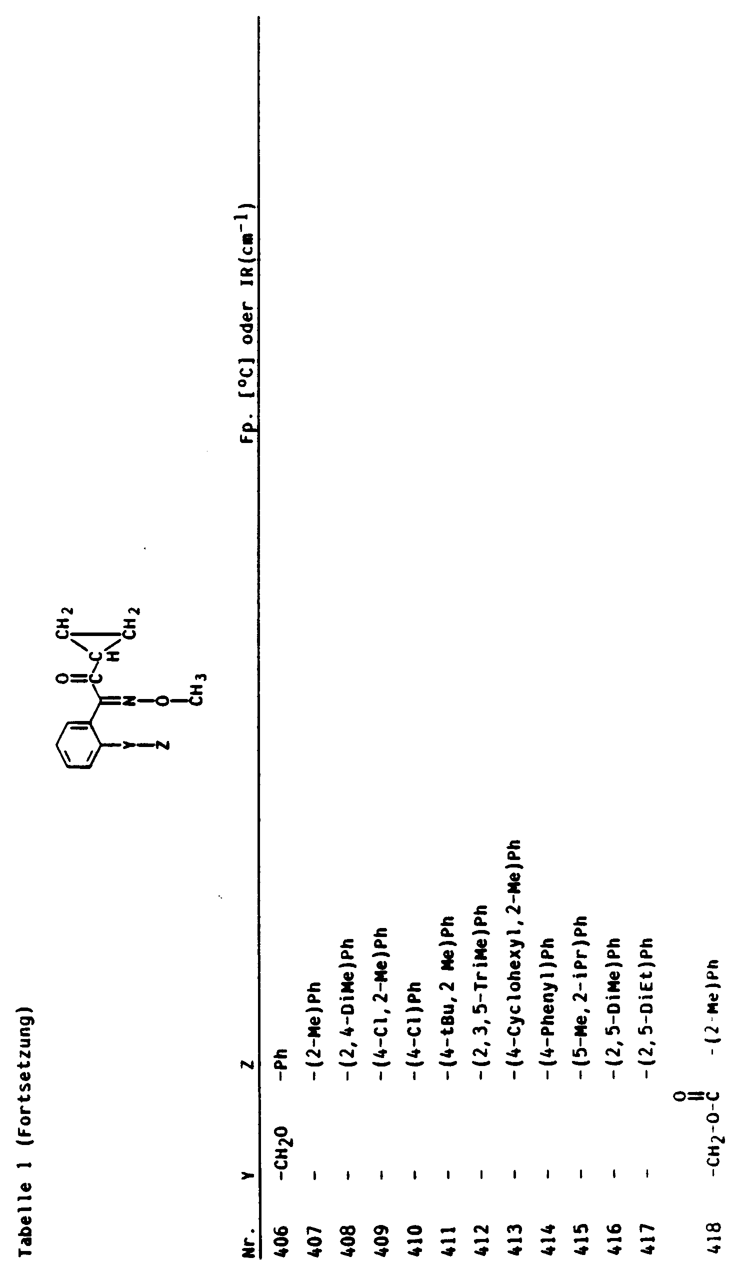

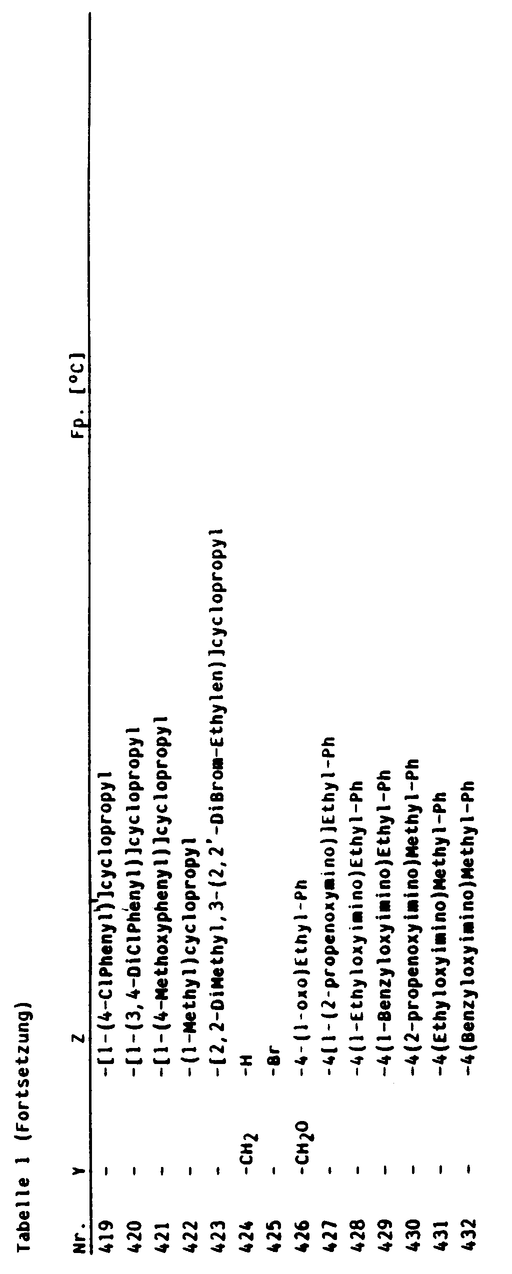

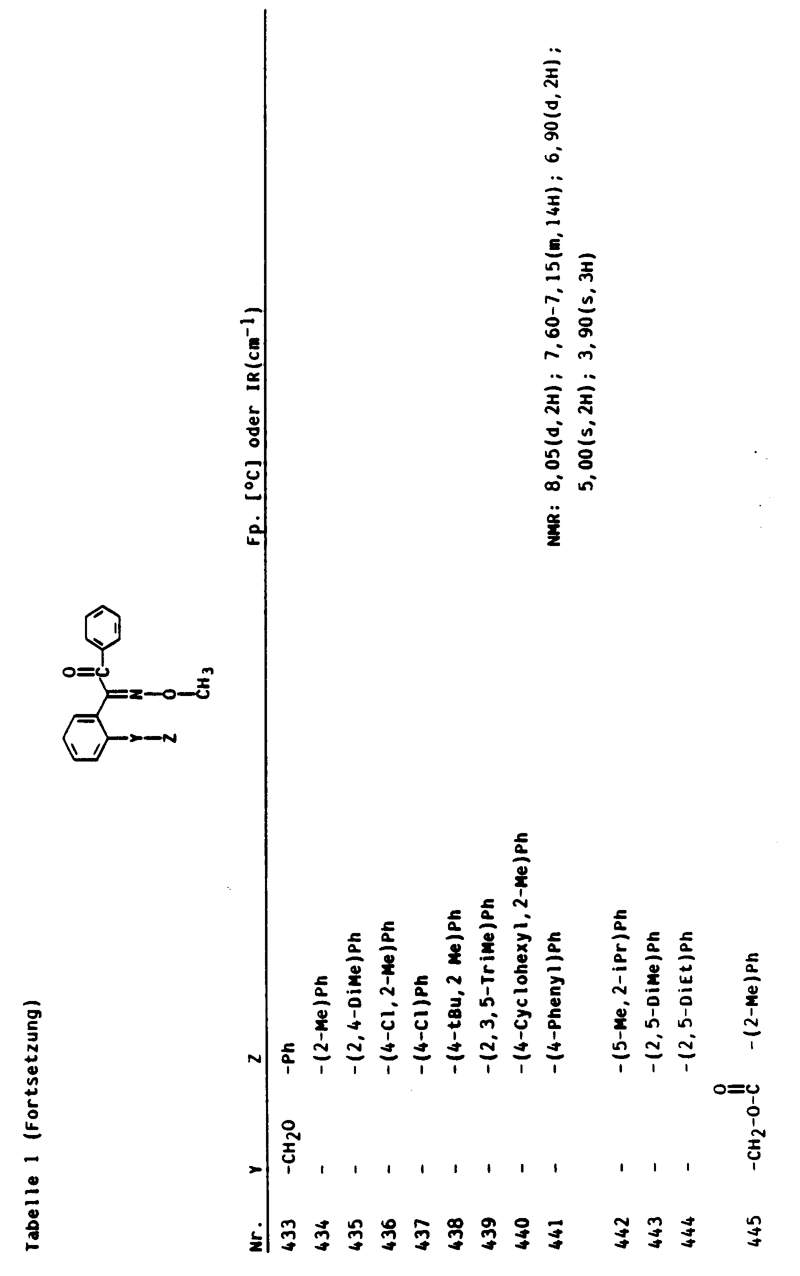

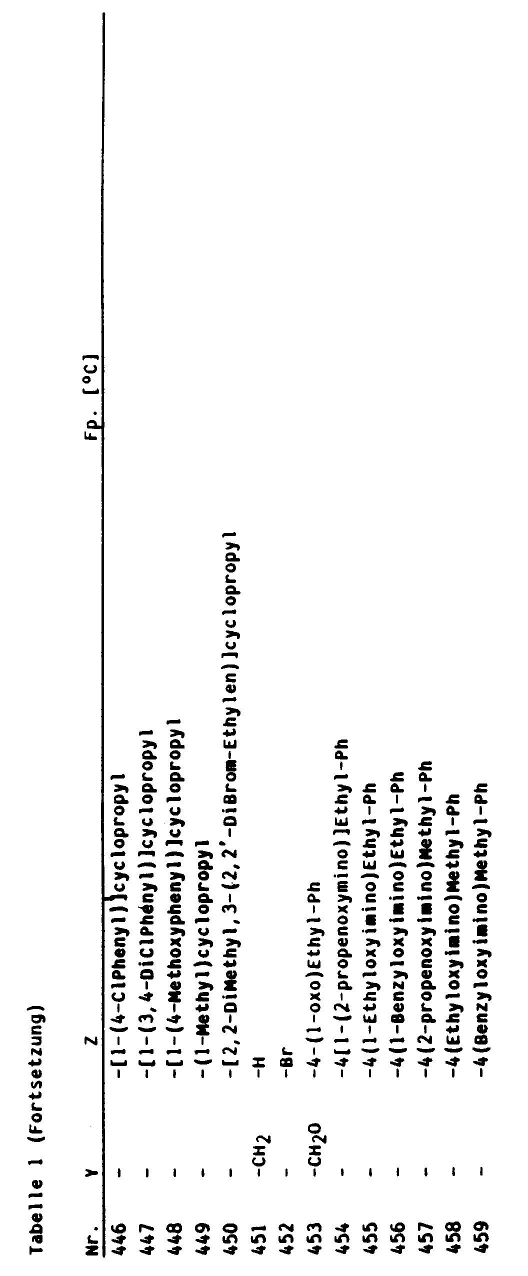

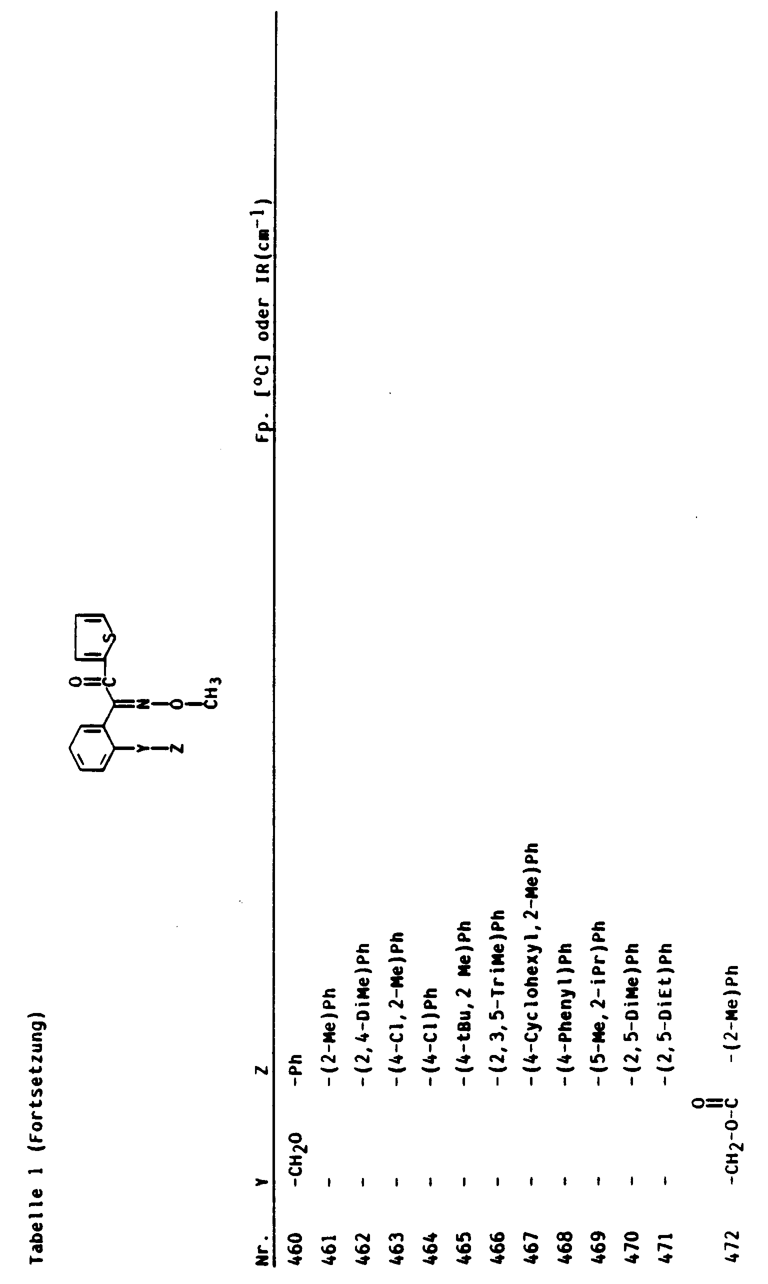

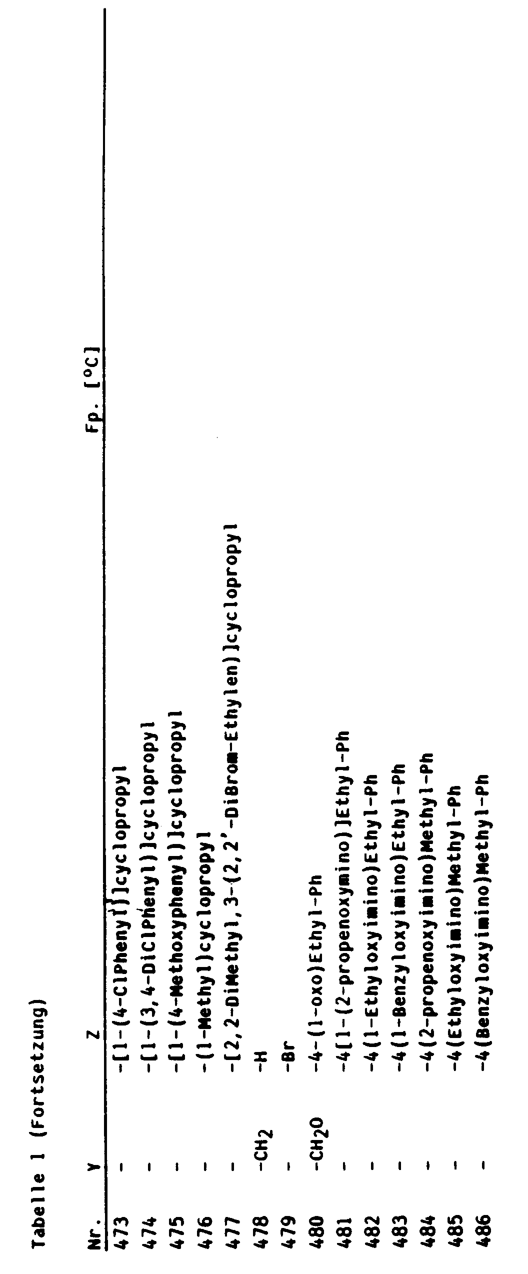

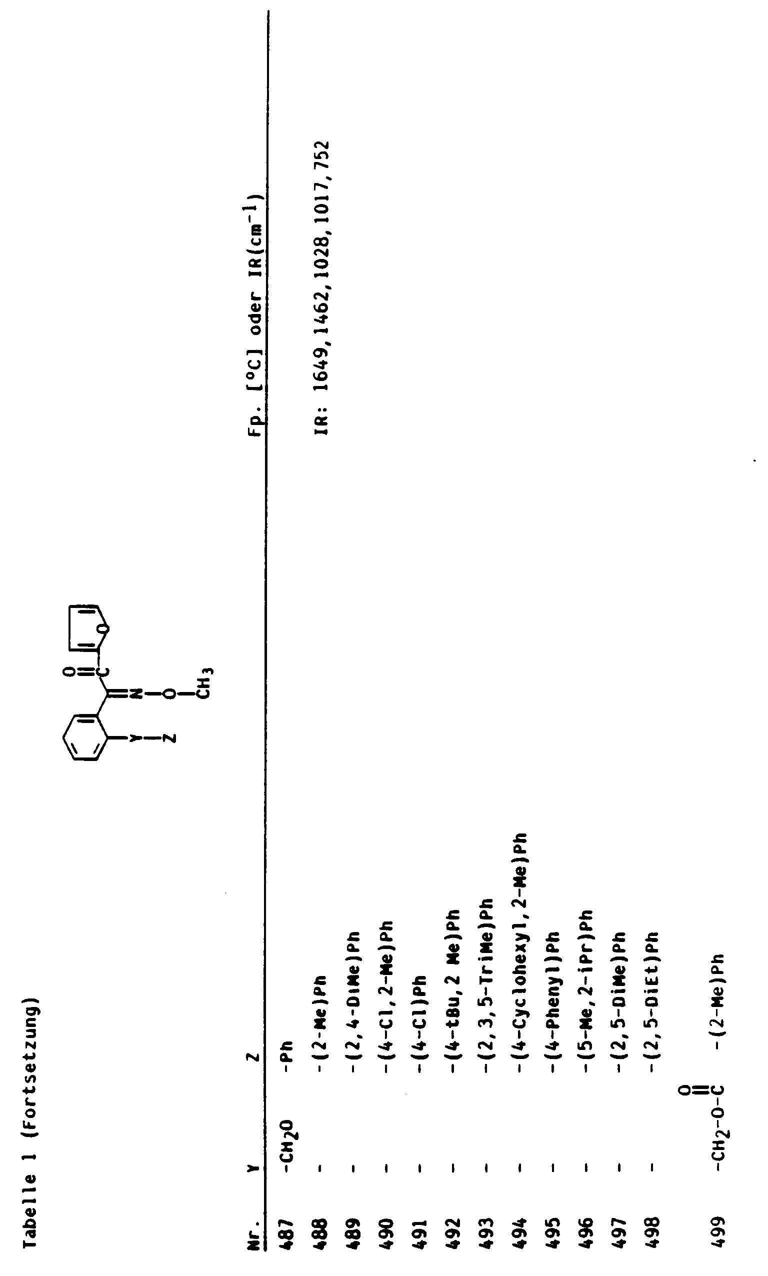

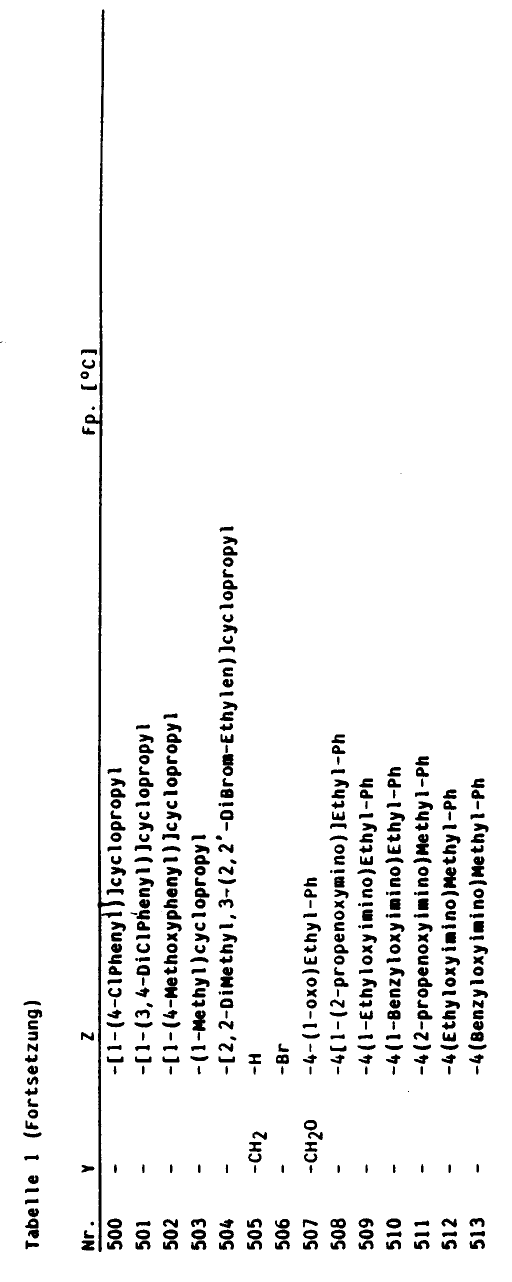

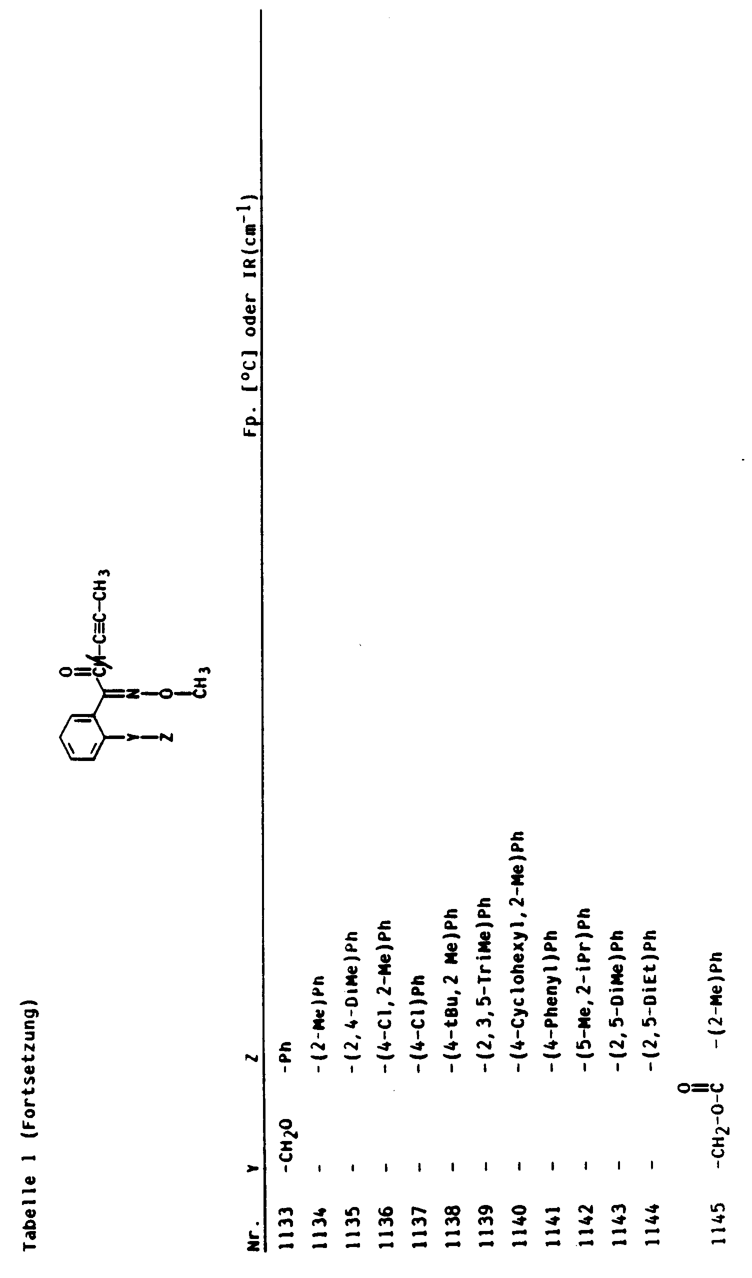

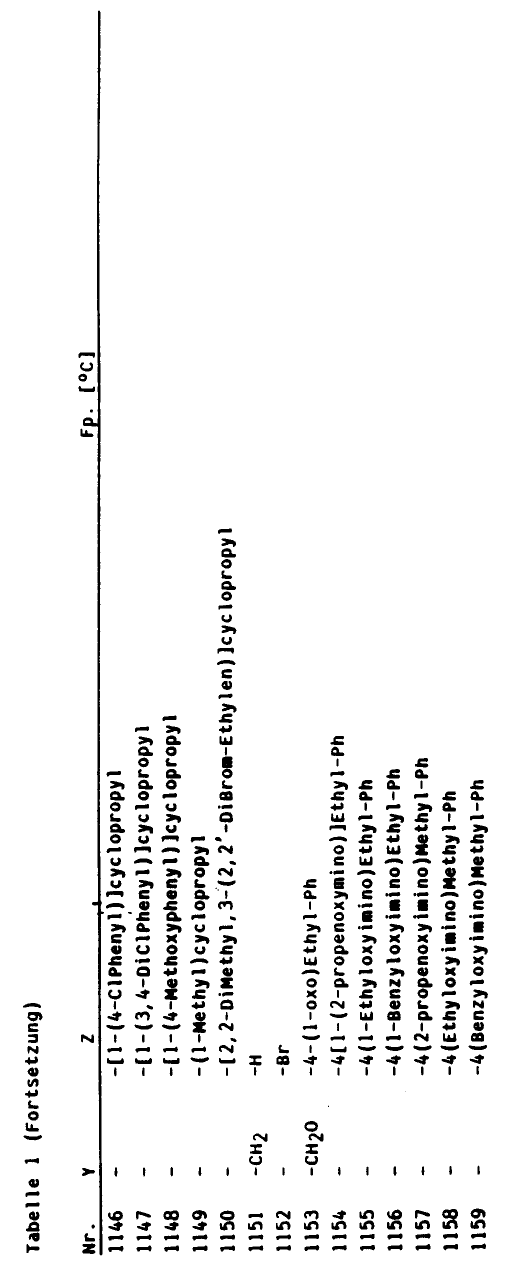

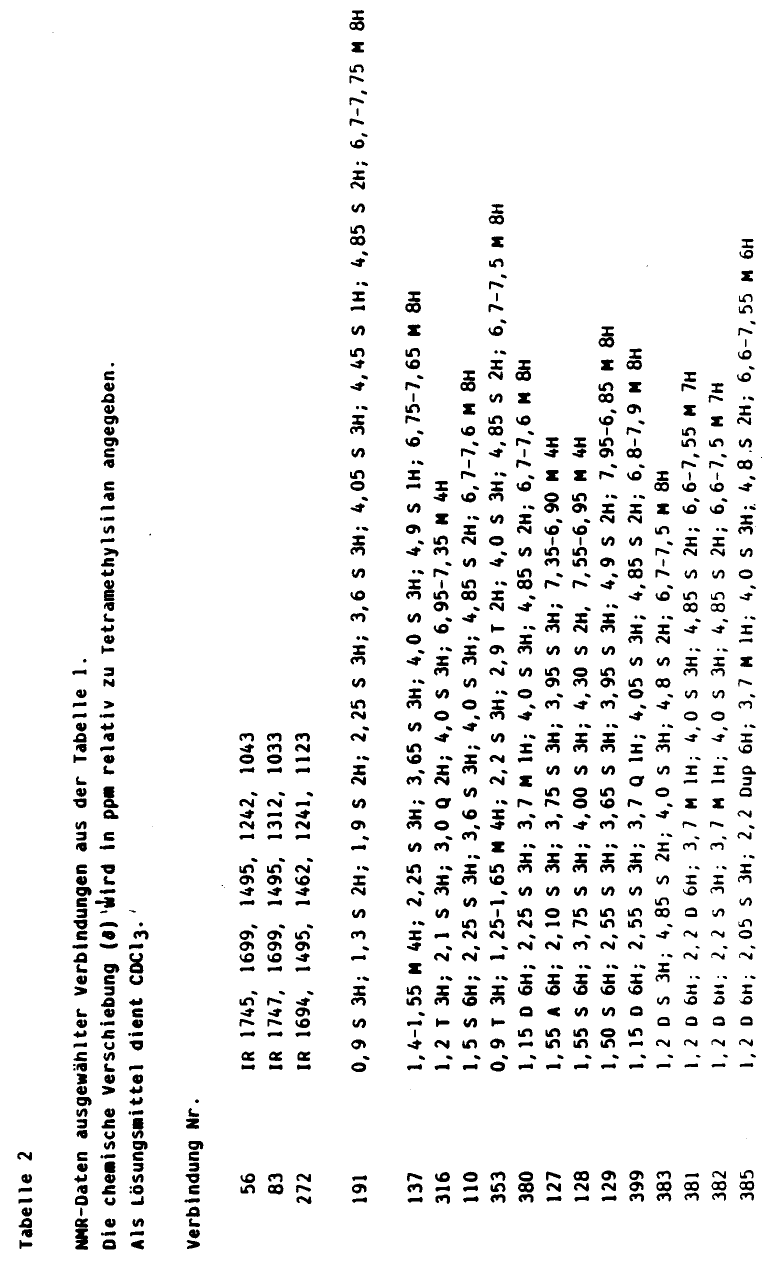

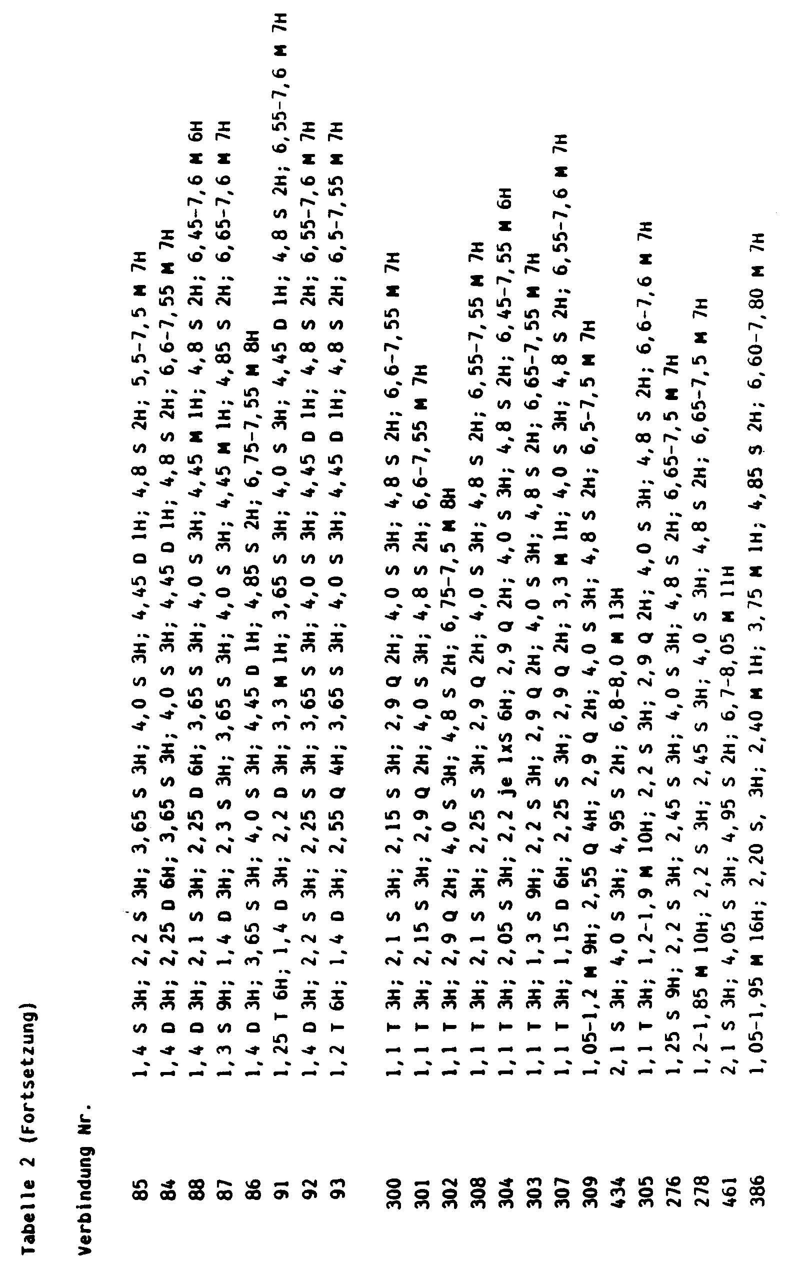

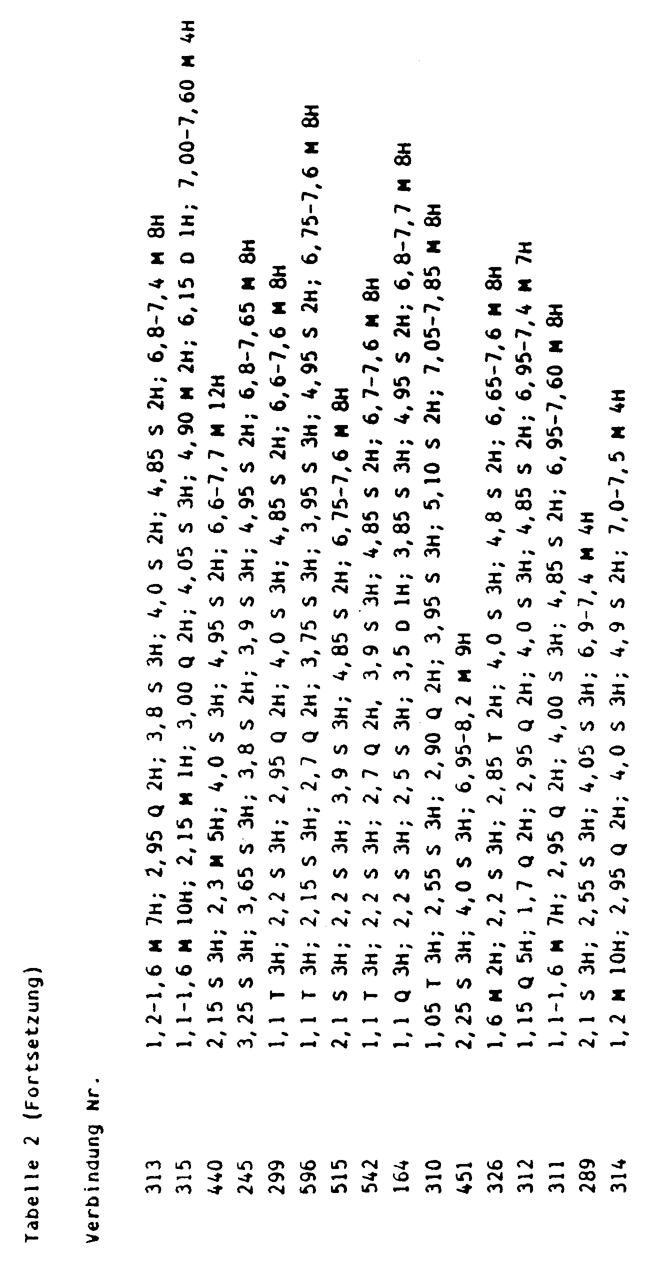

Die neuen Oximether eignen sich als Fungizide.

Die erfindungsgemäßen fungiziden Oximether bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensote, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
- I.: eine Lösung aus 90 Gew.-Teilen der Verbindung Nr. 56 und 10 Gew.-Teilen N-Methyl-α-pyrrolidon, die zur Anwendung in Form kleinster Tropfen geeignet ist;
- II.: eine Mischung aus 20 Gew.-Teilen der Verbindung Nr. 83, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.
- III.: eine wäßrige Dispersion aus 20 Gew.-Teilen der Verbindung Nr. 272, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
- IV.: eine wäßrige Dispersion aus 20 Gew.-Teilen der Verbindung Nr. 191, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
- V.: eine in einer Hammermühle vermahlene Mischung aus 80 Gew.-Teilen der Verbindung Nr. 137, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
- VI.: eine innige Mischung aus 3 Gew.-Teilen der Verbindung Nr. 316 und 97 Gew.-Teilen feinteiligem Kaolin; dieses Stäubemittel enthält 3 Gew.-% Wirkstoff;
- VII.: eine innige Mischung aus 30 Gew.-Teilen der Verbindung Nr. 110, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- VIII.: eine stabile wäßrige Dispersion aus 40 Gew.-Teilen der Verbindung Nr. 353, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;
- IX.: eine stabile ölige Dispersion aus 20 Gew.-Teilen der Verbindung Nr. 380, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die Oximether zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Oximether zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

### Anwendungsbeispiel

Als Vergleichswirkstoff wurde 1-Methoxyimino-1-(2-phenoxymethyl)-phenylessigsäuremethylester (A) - bekannt aus EP 253 213 - benutzt.

### Anwendungsbeispiel

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe 88, 92, 110, 164, 273, 277, 299, 300, 301, 304, 305, 307 und 308 bei der Anwendung als 0,0015 %ige (Gew.%) Spritzbrühe eine bessere fungizide Wirkung zeigen (95 %) als der bekannte Vergleichswirkstoff A (75 %).

## Patentansprüche

1. Oximether der Formel I 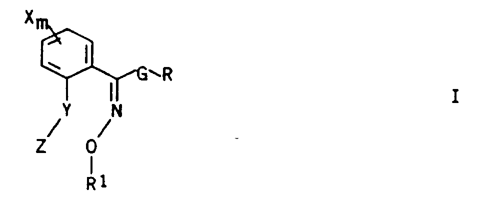 in der der Index und die Substituenten die folgende Bedeutung haben:
G eine Gruppe C=O,
R C₂-C₈-Alkenyl, C₂-C₈-Alkinyl, Phenyl, Hetaryl oder -CR²R³R⁴,
R²,R⁴ Wasserstoff, C₁-C₈-Alkyl, Arylalkyl, Hetarylalkyl, Alkoxyalkyl, Aryloxyalkyl, Aryl oder Hetaryl, oder
R²,R⁴ gemeinsam mit dem C-Atom, dessen Substituenten sie sind, C₃-C₈-Cycloalkyl,
R³ Wasserstoff, Methyl, Cyano, Carboxyl oder C₁-C₈-Alkoxycarbonyl, oder
R²,R³ gemeinsam mit dem C-Atom, an das sie gebunden sind, ein 5- bis 8-gliedriger Lactonring,
R¹ C₁-C₈-Alkyl,
X Nitro, Cyano, Halogen, C₁-C₄-Alkyl, C₁-C₄-Alkoxy oder C₁-C₄-Halogenalkyl,
m 0, 1, 2, 3 oder 4, wobei die Reste X verschieden sein können, wenn m größer als 1 ist,
Y O, S(O)ₙ (n = 0, 1 oder 2), -O-CO-, -CO-O-, Azo, Carbonylamino, Aminocarbonyl, Aminocarbonyloxy,
C₁-C₈-Alkylen, C₂-C₈-Alkenylen oder C₂-C₈-Alkinylen,
C₁-C₈-Alkylenoxy, Oxy-C₁-C₄-Alkylen, C₁-C₈-Thioalkylen, Oxycarbonyl-C₁-C₈-alkylen, Carbonyloxy-C₁-C₈-alkylen, Oxy-C₂-C₈-alkylenoxy, Oxy-C₂-C₈-alkenylen,
C₁-C₈-Alkylamino, Carbonyl-C₁-C₈-alkylamino, C₁-C₈-Alkylaminocarbonyl, C₁-C₈-Alkylaminocarbonyloxy,
Oxyimino oder Iminooxyalkylen,
Z C₁-C₁₈-Alkyl, C₁-C₄-Alkoxy-C₁-C₁₀-alkyl, C₁-C₁₀-Halogenalkyl, C₂-C₁₈-Alkenyl, C₃-C₈-Alkinyl, C₁-C₄-Alkoxycarbonyl,
Aryl, Aryl-C₁-C₁₀-alkyl, Aryl-C₂-C₁₀-alkenyl, Aryloxy-C₁-C₁₀-alkyl oder Hetaryl,
wobei die für Z genannten Reste ggf. substituiert sind durch
Cyano, Formyl, Nitro, Halogen, C₁-C₄-Alkyl, C₁-C₄-Halogenalkyl, C₁-C₄-Alkoxy-C₁-C₄-alkyl, C₁-C₄-Dialkoxy-C₁-C₄-alkyl, C₂-C₄-Alkenyl, C₂-C₄-Halogenalkenyl, C₁-C₄-Alkoxy, C₁-C₄-Alkoxycarbonyl,
ggf. subst. C₁-C₁₀-Alkoxyimino, C₂-C₁₀-Alkenyloxyimino, Aralkyloxyimino,
ggf. subst. C₂-C₁₀-Alkanoyl,
ggf. subst. Phenyl oder Phenoxy, oder
ggf. subst. C-verknüpfter 5-gliedriger Heterocyclus, der ein bis vier gleiche oder verschiedene Heteroatome (Stickstoff, Sauerstoff oder Schwefel) enthält, in dem zwei benachbarte Substituenten einen ggf. subst. aromatischen oder heteroaromatischen Ring bilden können.

2. Oximether der Formel I gemäß Anspruch 1, in der der Index und die Substituenten die folgende Bedeutung haben:
R C₃-C₈-Cycloalkyl, C₂-C₈-Alkinyl, Phenyl, Hetaryl oder -CR²R³R⁴,
R²,R⁴ Wasserstoff, C₁-C₈-Alkyl, C₃-C₈-Cycloalkyl, oder
unsubstituiertes oder durch C₁-C₄-Alkyl, C₁-C₄-Alkoxy oder Halogen substituiertes Phenyl,
R³ Wasserstoff,
R¹ Wasserstoff oder C₁-C₆-Alkyl,
X Cyano, Halogen, C₁-C₄-Alkyl, C₁-C₄-Halogenalkyl oder C₁-C₄-Alkoxy,
Y Sauerstoff, Schwefel, Methylen, Ethylen, Ethenylen, Ethinylen, Methylenoxy, Ethylenoxy, Oxymethylen, Oxyethylen, Thiomethylen, Thioethylen, Methylenthio, Ethylenthio, Carbonyloxy, Carbonyloxymethylen, Aminocarbonyloxy, Oxycarbonylmethylen oder eine Gruppe C=N-O-CH₂, O-N=C, C=N-O oder N=N,
Z C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolnyl, Isochinolinyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzisoxazolyl, Benzopyrrazolyl, Benzoimidazolyl, Indolyl, Isoindolyl, Benzothiophenyl, Isobenzothiophenyl, Benzofuranyl oder Isobenzofuranyl,
wobei die für Z genannten Reste ggf. substituiert sind durch
Cyano, Formyl, Hydroxyimino, Halogen, C₁-C₄-Alkyl, C₂-C₄-Alkenyl, C₂-C₄-Alkinyl, C₁-C₄-Carboxyalkoxy, C₁-C₄-Alkanoyl, C₁-C₄-Alkoxyalkyl, O-Alkoxyimino, O-Alkenyloxyimino, O-Arylalkoxyimino oder C₁-C₄-Dialkoxy-C₁-C₄-alkyl.

3. Oximether der Formel I gemäß Anspruch 1, in der der Index und die Substituenten die folgende Bedeutung haben:
R CR²R³R⁴,
R²,R⁴ Wasserstoff, C₁-C₈-Alkyl, C₃-C₈-Cycloalkyl, oder unsubstituiertes oder durch C₁-C₄-Alkyl, C₁-C₄-Alkoxy oder Halogen substituiertes Phenyl,
R³ Cyano, oder
R²,R⁴ gemeinsam mit dem C-Atom, dessen Substituenten sie sind, C₃-C₈-Cycloalkyl,
R¹ Wasserstoff oder C₁-C₆-Alkyl,
X Cyano, Halogen, C₁-C₄-Alkyl, C₁-C₄-Halogenalkyl oder C₁-C₄-Alkoxy,
Y Sauerstoff, Schwefel, Methylen, Ethylen, Ethenylen, Ethinylen, Methylenoxy, Ethylenoxy, Oxymethylen, Oxyethylen, Thiomethylen, Thioethylen, Methylenthio, Ethylenthio, Carbonyloxy, Carbonyloxymethylen, Aminocarbonyloxy, Oxycarbonylmethylen oder eine Gruppe C=N-O-CH₂, O-N=C, C=N-O oder N=N,
Z C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolnyl, Isochinolinyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzisoxazolyl, Benzopyrrazolyl, Benzoimidazolyl, Indolyl, Isoindolyl, Benzothiophenyl, Isobenzothiophenyl, Benzofuranyl oder Isobenzofuranyl,
wobei die für Z genannten Reste ggf. substituiert sind durch
Cyano, Formyl, Hydroxyimino, Halogen, C₁-C₄-Alkyl, C₂-C₄-Alkenyl, C₂-C₄-Alkinyl, C₁-C₄-Carboxyalkoxy, C₁-C₄-Alkanoyl, C₁-C₄-Alkoxyalkyl, O-Alkoxyimino, O-Alkenyloxyimino, O-Arylalkoxyimino oder C₁-C₄-Dialkoxy-C₁-C₄-alkyl.

4. Oximether der Formel I gemäß Anspruch 1, in der der Index und die Substituenten die folgende Bedeutung haben:
R CR²R³R⁴,
R²,R⁴ Wasserstoff, C₁-C₈-Alkyl, C₃-C₈-Cycloalkyl, oder
unsubstituiertes oder durch C₁-C₄-Alkyl, C₁-C₄-Alkoxy oder Halogen substituiertes Phenyl,
R³ Carboxyl, Methoxycarbonyl, Ethoxycarbonyl, iso-Propyloxycarbonyl, Propyloxycarbonyl, n-Butyloxycarbonyl, sec-Butyloxycarbonyl oder tert.-Butyloxycarbonyl, oder
R²,R³ gemeinsam mit dem C-Atom, an das sie gebunden sind, ein 5- bis 8-gliedriger Lactonring, oder
R²,R⁴ gemeinsam mit dem C-Atom, dessen Substituenten sie sind, C₃-C₈-Cycloalkyl,
R¹ Wasserstoff oder C₁-C₆-Alkyl,
X Cyano, Halogen, C₁-C₄-Alkyl, C₁-C₄-Halogenalkyl oder C₁-C₄-Alkoxy,
Y Sauerstoff, Schwefel, Methylen, Ethylen, Ethenylen, Ethinylen, Methylenoxy, Ethylenoxy, Oxymethylen, Oxyethylen, Thiomethylen, Thioethylen, Methylenthio, Ethylenthio, Carbonyloxy, Carbonyloxymethylen, Aminocarbonyloxy, Oxycarbonylmethylen oder eine Gruppe C=N-O-CH₂, O-N=C, C=N-O oder N=N,
Z C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolnyl, Isochinolinyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzisoxazolyl, Benzopyrrazolyl, Benzoimidazolyl, Indolyl, Isoindolyl, Benzothiophenyl, Isobenzothiophenyl, Benzofuranyl oder Isobenzofuranyl,
wobei die für Z genannten Reste ggf. substituiert sind durch
Cyano, Formyl, Hydroxyimino, Halogen, C₁-C₄-Alkyl, C₂-C₄-Alkenyl, C₂-C₄-Alkinyl, C₁-C₄-Carboxyalkoxy, C₁-C₄-Alkanoyl, C₁-C₄-Alkoxyalkyl, O-Alkoxyimino, O-Alkenyloxyimino, O-Arylalkoxyimino oder C₁-C₄-Dialkoxy-C₁-C₄-alkyl.

5. Oximether der Formel I gemäß Anspruch 1, in der R Ethyl, R¹ Methyl, Y Oxymethylen und Z 2,4-Dimethylphenyl bedeutet.

6. Verfahren zur Herstellung von Oximethern der Formel I gemäß Anspruch 1, dadurch gekennzeichnet, daß man
a) ein Phenylglyoxylsäuremethylester-O-alkyloxim der Formel II 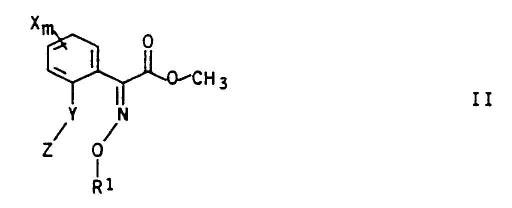 mit einer Verbindung der Formel III 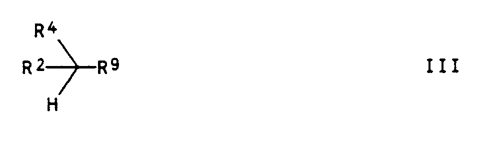 in der R⁹ CO₂-A bedeutet, unter basischen Bedingungen umsetzt und
b) gegebenenfalls die so erhaltenen Verbindungen Ib 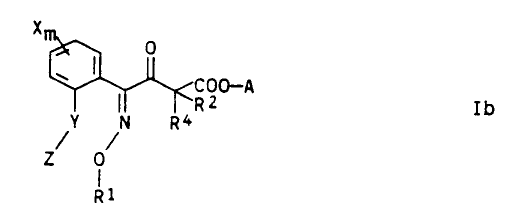 mit DMSO/LiCl zu den Verbindungen der Formel Ia 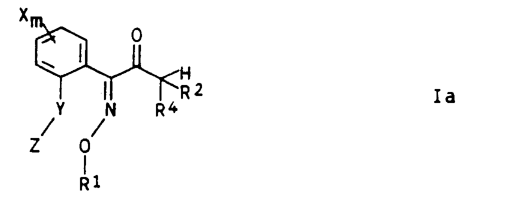 umsetzt, oder
c) die Verbindungen der Formel II mit Natriumhydroxyd und Ethanol, Thionylchlorid und Triethylamin und O-Methyl-N-methylhydroxylamin-hydrochlorid zu den Verbindungen der Formel IV 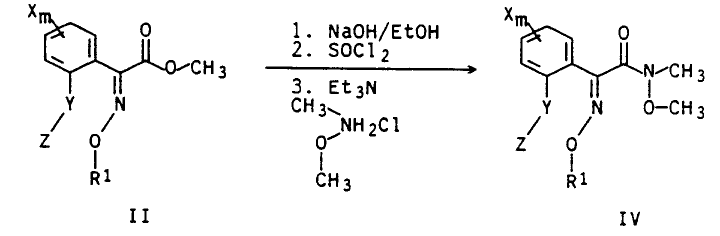 umsetzt, und
d) die so erhaltenen Amide der Formel IV mit Organo-Lithiumreagenzien zu den α-Oxyiminoketonen der Formel Id 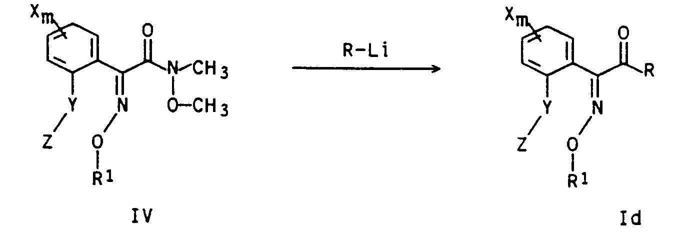 umsetzt.

7. Verfahren zur Bekämpfung von Pilzen, dadurch gekennzeichnet, daß man die Pilze oder die vom Pilzbefall bedrohten Materialien, Pflanzen, Saatgüter oder den Erdboden mit einer fungizid wirksamen Menge einer Verbindung der Formel I gemäß Anspruch 1 behandelt.

8. Fungizid, enthaltend einen inerten Trägerstoff und eine fungizid wirksame Menge einer Verbindung der Formel I gemäß Anspruch 1.

## Claims

1. A oxime ether of the formula I 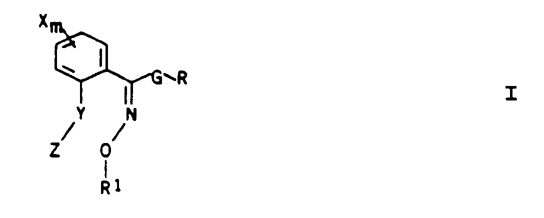 where
G is a group C=O,
R is C₂-C₈-alkenyl, C₂-C₈-alkynyl, phenyl, hetaryl or -CR²R³R⁴,
R² and R⁴ are each hydrogen, C₁-C₈-alkyl, arylalkyl, hetarylalkyl, alkoxyalkyl, aryloxyalkyl, aryl or hetaryl or
R² and R⁴, together with the carbon atom on which they are substituents, form C₃-C₈-cycloalkyl,
R³ is hydrogen, methyl, cyano, carboxyl or C₁-C₈-alkoxycarbonyl or
R² and R³, together with the carbon atom to which they are bonded, form a 5-membered to 8-membered lactone ring,
R¹ is C₁-C₈-alkyl,
X is nitro, cyano, halogen, C₁-C₄-alkyl, C₁-C₄-alkoxy or C₁-C₄-haloalkyl,
m is 0, 1, 2, 3 or 4, and the radicals X may be different when m is greater than 1,
Y is O, S(O)ₙ (n = 0, 1 or 2), -O-CO-, -CO-O-, azo, carbonylamino, aminocarbonyl, aminocarbonyloxy,
C₁-C₈-alkylene, C₂-C₈-alkenylene or C₂-C₈-alkynylene,
C₁-C₈-alkyleneoxy, oxy-C₁-C₄-alkylene, C₁-C₈-thioalkylene, oxycarbonyl-C₁-C₈-alkylene, carbonyloxy-C₁-C₈-alkylene, oxy-C₂-C₈-alkyleneoxy, oxy-C₂-C₈-alkenylene,
C₁-C₈-alkylamino, carbonyl-C₁-C₈-alkylamino, C₁-C₈-alkylaminocarbonyl, C₁-C₈-alkylaminocarbonyloxy,
oxyimino or iminooxyalkylene and
Z is C₁-C₁₈-alkyl, C₁-C₄-alkoxy-C₁-C₁₀-alkyl, C₁-C₁₀-haloalkyl, C₂-C₁₈-alkenyl, C₃-C₈-alkynyl, C₁-C₄-alkoxycarbonyl,
aryl, aryl-C₁-C₁₀-alkyl, aryl-C₂-C₁₀-alkenyl, aryloxy-C₁-C₁₀-alkyl or hetaryl,
where the radicals stated for Z are unsubstituted or substituted by
cyano, formyl, nitro, halogen, C₁-C₄-alkyl, C₁-C₄-haloalkyl, C₁-C₄-alkoxy-C₁-C₄-alkyl, C₁-C₄-dialkoxy-C₁-C₄-alkyl, C₂-C₄-alkenyl, C₂-C₄-haloalkenyl, C₁-C₄-alkoxy, C₁-C₄-alkoxycarbonyl,
unsubstituted or substituted C₁-C₁₀-alkoxyimino, C₂-C₁₀-alkenyloxyimino, aralkyloxyimino,
ubsubstituted or substituted C₂-C₁₀-alkanoyl,
unsubstituted or substituted phenyl or phenoxy or
unsubstituted or substituted C-linked 5-membered heterocyclic structure which contains one to four identical or different heteroatoms (nitrogen, oxygen or sulfur) and in which two neighboring substituents may form an unsubstituted or substituted aromatic or hetero-aromatic ring.

2. An oxime ether of the formula I as claimed in claim 1, wherein
R is C₃-C₈-cycloalkyl, C₂-C₈-alkynyl, phenyl, hetaryl or -CR²R³R⁴,
R² and R⁴ are each hydrogen, C₁-C₈-alkyl, C₃-C₈-cycloalkyl
or
phenyl which is unsubstituted or substituted by C₁-C₄-alkyl, C₁-C₄-alkoxy or halogen,
R³ is hydrogen,
R¹ is hydrogen or C₁-C₆-alkyl,
X is cyano, halogen, C₁-C₄-alkyl, C₁-C₄-haloalkyl or C₁-C₄-alkoxy,
Y is oxygen, sulfur, methylene, ethylene, ethenylene, ethynylene, methyleneoxy, ethyleneoxy, oxymethylene, oxyethylene, thiomethylene, thioethylene, methylenethio, ethylenethio, carbonyloxy, carbonyloxymethylene, aminocarbonyloxy, oxycarbonylmethylene or a group C=N-O-CH₂, O-N=C, C=N-O or N=N and
Z is C₁-C₁₀-alkyl, C₂-C₁₀-alkenyl, C₂-C₁₀-alkynyl, phenyl, pyridyl, pyridazinyl, pyrimidinyl, pyrazinyl, quinolyl, isoquinolyl, naphthyridyl, quinoxalyl, thienyl, furyl, pyrrolyl, oxazolyl, isoxazolyl, pyrazolyl, imidazolyl, thiazolyl, isothiazolyl, benzothiazolyl, benzoisothiazolyl, benzoxazolyl, benzisoxazolyl, benzopyrrazolyl, benzoimidazolyl, indolyl, isoindolyl, benzothiophenyl, isobenzothiophenyl, benzofuranyl or isobenzofuranyl,
where the radicals stated for Z are unsubstituted or substituted by
cyano, formyl, hydroxyimino, halogen, C₁-C₄-alkyl, C₂-C₄-alkenyl, C₂-C₄-alkynyl, C₁-C₄-carboxyalkoxy,
C₁-C₄-alkanoyl, C₁-C₄-alkoxyalkyl, O-alkoxyimino, O-alkenyloxyimino, O-arylalkoxyimino or C₁-C₄-dialkoxy-C₁-C₄-alkyl.

3. An oxime ether of the formula I as claimed in claim 1, wherein
R is CR²R³R⁴,
R² and R⁴ are each hydrogen, C₁-C₈-alkyl, C₃-C₈-cycloalkyl
or
phenyl which is unsubstituted or substituted by C₁-C₄-alkyl, C₁-C₄-alkoxy or halogen,
R³ is cyano or
R² and R⁴, together with the carbon atom on which they are substitutents, form C₃-C₈-cycloalkyl,
R¹ is hydrogen or C₁-C₆-alkyl,
X is cyano, halogen, C₁-C₄-alkyl, C₁-C₄-haloalkyl or C₁-C₄-alkoxy,
Y is oxygen, sulfur, methylene, ethylene, ethenylene, ethynylene, methyleneoxy, ethyleneoxy, oxymethylene, oxyethylene, thiomethylene, thioethylene, methylenethio, ethylenethio, carbonyloxy, carbonyloxymethylene, aminocarbonyloxy, oxycarbonylmethylene or a group C=N-O-CH₂, O-N=C, C=N-O or N=N and
Z is C₁-C₁₀-alkyl, C₂-C₁₀-alkenyl, C₂-C₁₀-alkynyl, phenyl, pyridyl, pyridazinyl, pyrimidinyl, pyrazinyl, quinolyl, isoquinolyl, naphthyridyl, quinoxalyl, thienyl, furyl, pyrrolyl, oxazolyl, isoxazolyl, pyrazolyl, imidazolyl, thiazolyl, isothiazolyl, benzothiazolyl, benzoisothiazolyl, benzoxazolyl, benzisoxazolyl, benzopyrrazolyl, benzoimidazolyl, indolyl, isoindolyl, benzothiophenyl, isobenzothiophenyl, benzofuranyl or isobenzofuranyl,
where the radicals stated for Z are unsubstituted or substituted by
cyano, formyl, hydroxyimino, halogen, C₁-C₄-alkyl, C₂-C₄-alkenyl, C₂-C₄-alkynyl, C₁-C₄-carboxyalkoxy, C₁-C₄-alkanoyl, C₁-C₄-alkoxyalkyl, O-alkoxyimino, O-alkenyloxyimino, O-arylalkoxyimino or C₁-C₄-dialkoxy-C₁-C₄-alkyl.

4. An oxime ether of the formula I as claimed in claim 1, wherein
R is CR²R³R⁴,
R² and R⁴ are each hydrogen, C₁-C₈-alkyl, C₃-C₈-cycloalkyl
or
phenyl which is unsubstituted or substituted by C₁-C₄-alkyl, C₁-C₄-alkoxy or halogen,
R³ is carboxyl, methoxycarbonyl, ethoxycarbonyl, isopropoxycarbonyl, propyloxycarbonyl, n-butoxycarbonyl, sec-butoxycarbonyl or tert-butoxycarbonyl or
R² and R³, together with the carbon atom to which they are bonded, form a 5-membered to 8-membered lactone ring or
R² and R⁴, together with the carbon atom on which they are substituents, form C₃-C₈-cycloalkyl,
R¹ is hydrogen or C₁-C₆-alkyl,
X is cyano, halogen, C₁-C₄-alkyl, C₁-C₄-haloalkyl or C₁-C₄-alkoxy,
Y is oxygen, sulfur, methylene, ethylene, ethenylene, ethynylene, methyleneoxy, ethyleneoxy, oxymethylene, oxyethylene, thiomethylene, thioethylene, methylenethio, ethylenethio, carbonyloxy, carbonyloxymethylene, aminocarbonyloxy, oxycarbonylmethylene or a group C=N-O-CH₂, O-N=C, C=N-O or N=N and
Z is C₁-C₁₀-alkyl, C₂-C₁₀-alkenyl, C₂-C₁₀-alkynyl, phenyl, pyridyl, pyridazinyl, pyrimidinyl, pyrazinyl, quinolyl, isoquinolyl, naphthyridyl, quinoxalyl, thienyl, furyl, pyrrolyl, oxazolyl, isoxazolyl, pyrazolyl, imidazolyl, thiazolyl, isothiazolyl, benzothiazolyl, benzoisothiazolyl, benzoxazolyl, benzisoxazolyl, benzopyrrazolyl, benzoimidazolyl, indolyl, isoindolyl, benzothiophenyl, isobenzothiophenyl, benzofuranyl or isobenzofuranyl,
where the radicals stated for Z are unsubstituted or substituted by
cyano, formyl, hydroxyimino, halogen, C₁-C₄-alkyl, C₂-C₄-alkenyl, C₂-C₄-alkynyl, C₁-C₄-carboxyalkoxy, C₁-C₄-alkanoyl, C₁-C₄-alkoxyalkyl, O-alkoxyimino, O-alkenyloxyimino, O-arylalkoxyimino or C₁-C₄-dialkoxy-C₁-C₄-alkyl.

5. An oxime ether of the formula I as claimed in claim 1, wherein R is ethyl, R¹ is methyl, Y is oxymethylene and Z is 2,4-dimethylphenyl.

6. A process for the preparation of an oxime ether of the formula I as claimed in claim 1, wherein
a) an O-alkyl oxime of a methyl phenylglyoxylate of the formula II 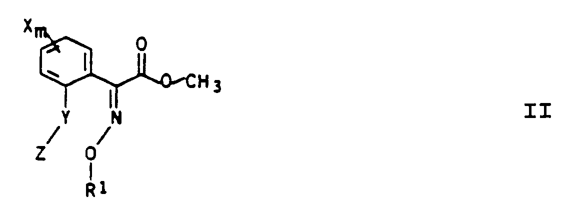 is reacted with a compound of the formula III 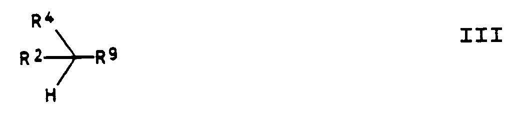 where R⁹ is CO₂-A, under basic conditions, and
b) if required, the resulting compound Ib 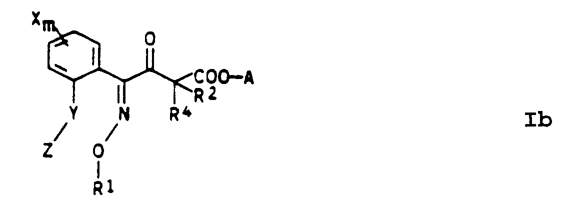 is reacted with dimethyl sulfoxide/LiCl to give a compound of the formula Ia 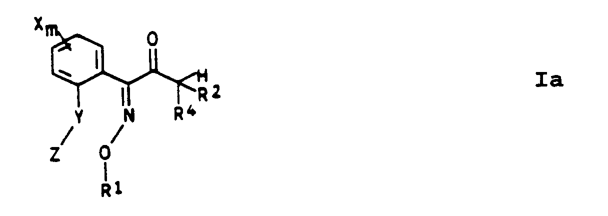 or
c) the compound of the formula II is reacted with sodium hydroxide and ethanol, thionyl chloride and triethylamine and O-methyl-N-methylhydroxyamine hydrochloride to give a compound of the formula IV 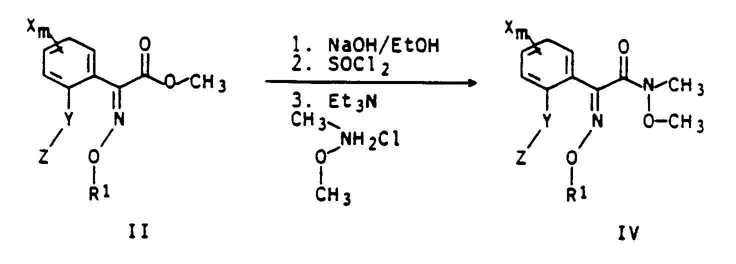 and
d) the resulting amide of the formula IV is reacted with an organolithium reagent to give an α-oximino ketone of the formula Id

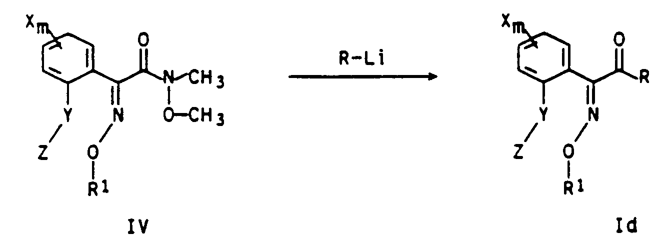

7. A method for controlling fungi, wherein the fungi or the materials, plants, or seeds threatened by fungal attack or the soil are treated with a fungicidal amount of a compound of the formula I as claimed in claim 1.

8. A fungicide containing an inert carrier and a fungicidal amount of a compound of the formula I as claimed in claim 1.

## Revendications

1. Ethers d'oximes de formule I 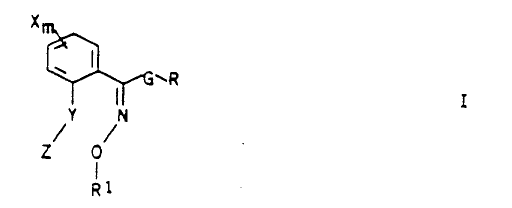 dans laquelle l'indice et les substituants ont la signification suivante :
G un groupe C=O,
R alcényle en C2-C8, alcynyle en C2-C8, phényle, hétaryle ou CR²R³R⁴,
R², R⁴ hydrogène, alkyle en C1-C8, arylalkyle, hétarylalkyle, alcoxyalkyle, aryloxalkyle, aryle ou hétaryle, ou
R², R⁴ ensemble avec l'atome C dont ils sont les substituants, cycloalkyle en C3-C8,
R³ hydrogène, méthyle, cyano, carboxyle ou alcoxy-C1-C8-carbonyle, ou
R², R³ ensemble avec l'atome C auquel ils sont liés, un cycle lactone à 5 à 8 chaînons,
R¹ alkyle en C1-C8,
X nitro, cyano, halogène, alkyle en C1-C4, alcoxy en C1-C4 ou halogéno-alkyle en C1-C4,
m 0, 1, 2, 3 ou 4, les restes X pouvant être différents lorsque m est supérieur à 1,
Y 0, S(0)ₙ (n = 0, 1 ou 2), -0-C0-, -C0-0-, azo, carbonylamino, aminocarbonyle, aminocarbonyloxy,
alkylène en C1-C8, alcénylène en C2-C8 ou alcynylène en C2-C8,
alkylène-C1-C8-oxy, oxy-alkylène-C1-C4, thioalkylène-C1-C8, oxycarbonyl-alkylène-C1-C8, carbonyloxy-alkylène-C1-C8, oxy-alkylène-C2-C8-oxy, oxy-alcénylène-C2-C8,
alkylamino-C1-C8, carbonyl-alkylamino-C1-C8, alkylamino-C1-C8-carbonyle, alkylamino-C1-C8-carbonyloxy,
oxyimino ou iminooxyalkylène,
Z alkyle en C1-C18, alcoxy-C1-C4-alkyle-C1-C10, halogéno-alkyle-C1-C10, alcényle en C2-C18, alcynyle en C3-C8, alcoxy-C1-C4-carbonyle,
aryle, aryl-alkyle-C1-C10, aryl-alcényle-C2-C10, aryloxy-alkyle-C1-C10 ou hétaryle,
les restes indiqués pour Z étant éventuellement substitués par
cyano, formyle, nitro, halogène, alkyle en C1-C4, halogéno-alkyle-C1-C4, alcoxy-C1-C4-alkyle-C1-C4, dialcoxy-C1-C4-alkyle-C1-C4, alcényle-C2-C4, halogéno-alcényle-C2-C4, alcoxy-C1-C4, alcoxy-C1-C4-carbonyle,
alcoxyimino-C1-C10, alcényloxyimino-C2-C10, aralkyloxyimino éventuellement substitués,
alcanoyle-C2-C10 éventuellement substitué,
phényle ou phénoxy éventuellement substitués ou
hétérocycle à 5 chaînons, éventuellement substitué, relié en C, qui contient un à quatre hétéroatomes (azote, oxygène ou soufre) identiques ou différents, dans lequel deux substituants contigus peuvent former un noyau aromatique ou hétéroaromatique éventuellement substitué.

2. Ethers d'oximes de formule I, selon la revendication 1, dans laquelle l'indice et les substituants ont la signification suivante :
R cycloalkyle-C3-C8, alcynyle-C2-C8, phényle, hétaryle ou - CR²R³R⁴,
R², R⁴, hydrogène, alkyle en C1-C8, cycloalkyle en C3-C8, ou
phényle non substitué ou substitué par alkyle en C1-C4, alcoxy en C1-C4 ou halogène,
R³ hydrogène,
R¹ hydrogène ou alkyle en C1-C6,
X cyano, halogène, alkyle en C1-C4, halogéno-alkyle-C1-C4 ou alcoxy-C1-C4,
Y oxygène, soufre, méthylène, éthylène, éthénylène, éthynylène, méthylènoxy, éthylènoxy, oxyméthylène, oxéthylène, thiométhylène, thioéthylène, méthylènethio, éthylènethio, carbonyloxy, carbonyloxyméthylène, aminocarbonyloxy, oxycarbonylméthylène ou un groupe C=N-O-CH₂, O-N=C, C=N-O ou N=N,
Z alkyle en C1-C10, alcényle en C2-C10, alcynyle en C2-C10, phényle, pyridinyle, pyridazinyle, pyrimidinyle, pyrazinyle, quinolinyle, isoquinolinyle, naphtyridyle, quinoxalyle, thiényle, furyle, pyrrolyle, oxazolyle, isoxazolyle, pyrazolyle, imidazolyle, thiazolyle, isothiazolyle, benzothiazolyle, benzoisothiazolyle, benzoxazolyle, benzoisoxazolyle, benzopyrrazolyle, benzoimidazolyle, indolyle, isoindolyle, benzothiophényle, isobenzothiophényle, benzofuranyle ou isobenzofuranyle,
les restes indiqués pour Z étant éventuellement substitués par
cyano, formyle, hydroxyimino, halogène, alkyle en C1-C4, alcényle en C2-C4, alcynyle en C2-C4, carboxy-alcoxy-C1-C4, alcanoyle-C1-C4, alcoxyalkyle-C1-C4, O-alcoxyimino, O-alcényloxyimino, O-arylalcoxyimino ou dialcoxy-C1-C4-alkyle-C1-C4.

3. Ethers d'oximes de formule I, selon la revendication 1, dans laquelle l'indice et les substituants ont la signification suivante :
R CR²R³R⁴,
R², R⁴ hydrogène, alkyle en C1-C8, cycloalkyle en C3-C8,
ou
phényle non substitué ou substitué par alkyle en C1-C4, alcoxy en C1-C4 ou halogène,
R³ cyano, ou
R², R⁴ ensemble avec l'atome C dont ils sont les substituants, cycloalkyle en C3-C8,
R¹ hydrogène ou alkyle en C1-C6,
X cyano, halogène, alkyle en C1-C4, halogéno-alkyle-C1-C4 ou alcoxy en C1-C4,
Y oxygène, soufre, méthylène, éthylène, éthénylène, éthynylène, méthylènoxy, éthylènoxy, oxyméthylène, oxéthylène, thiométhylène, thioéthylène, méthylènethio, éthylènethio, carbonyloxy, carbonyloxyméthylène, aminocarbonyloxy, oxycarbonylméthylène ou un groupe C=N-O-CH₂, O-N=C, C=N-O ou N=N,
Z alkyle en C1-C10, alcényle en C2-C10, alcynyle en C2-C10, phényle, pyridinyle, pyridazinyle, pyrimidinyle, pyrazinyle, quinolinyle, isoquinolinyle, naphtyridyle, quinoxalyle, thiényle, furyle, pyrrolyle, oxazolyle, isoxazolyle, pyrazolyle, imidazolyle, thiazolyle, isothiazolyle, benzothiazolyle, benzoisothiazolyle, benzoxazolyle, benzoisoxazolyle, benzopyrrazolyle, benzoimidazolyle, indolyle, isoindolyle, benzothiophényle, isobenzothiophényle, benzofuranyle ou isobenzofuranyle,
les restes indiqués pour Z étant éventuellement substitués par
cyano, formyle, hydroxyimino, halogène, alkyle en C1-C4, alcényle en C2-C4, alcynyle en C2-C4, carboxyalcoxy-C1-C4, alcanoyle-C1-C4, alcoxy-alkyle-C1-C4, O-alcoxyimino, O-alcényloxyimino, O-arylalcoxyimino ou dialcoxy-C1-C4-alkyle-C1-C4.

4. Ethers d'oximes de formule I, selon la revendication 1, dans laquelle l'indice et les substituants ont la signification suivante :
R CR²R³R⁴,
R², R⁴ hydrogène, alkyle en C1-C8, cycloalkyle en C3-C8, ou
phényle non substitué ou substitué par alkyle en C1-C4, alcoxy en C1-C4 ou halogène,
R³ carboxyle, méthoxycarbonyle, éthoxycarbonyle, iso-propyloxycarbonyle, propyloxycarbonyle, n-butyloxycarbonyle, sec-butyloxycarbonyle ou ter-butyloxycarbonyle, ou
R², R³, ensemble avec l'atome C auquel ils sont liés, un cycle lactone à 5 ou 8 chaînons, ou
R², R⁴, ensemble avec l'atome C dont ils sont les substituants, cycloalkyle en C3-C8,
R¹ hydrogène ou alkyle en C1-C6,
X cyano, halogène, alkyle en C1-C4, halogéno-alkyle-C1-C4 ou alcoxy en C1-C4,
Y oxygène, soufre, méthylène, éthylène, éthénylène, éthynylène, méthylènoxy, éthylènoxy, oxyméthylène, oxyéthylène, thiométhylène, thioéthylène, méthylènethio, éthylènethio, carbonyloxy, carbonyloxyméthylène, aminocarbonyloxy, oxycarbonylméthylène ou un groupe C=N-O-CH₂, O-N=C, C=N-O ou N=N,
Z alkyle en C1-C10, alcényle en C2-C10, alcynyle en C2-C10, phényle, pyridinyle, pyridazinyle, pyrimidinyle, pyrazinyle, quinolinyle, isoquinolinyle, naphtyridyle, quinoxalyle, thiényle, furyle, pyrrolyle, oxazolyle, isoxazolyle, pyrazolyle, imidazolyle, thiazolyle, isothiazolyle, benzothiazolyle, benzoisothiazolyle, benzoxazolyle, benzisoxazolyle, benzopyrrazolyle, benzoimidazolyle, indolyle, isoindolyle, benzothiophényle, isobenzothiophényle, benzofuranyle ou isobenzofuranyle,
les restes indiqués pour Z étant éventuellement substitués par
cyano, formyle, hydroxyimino, halogène, alkyle en C1-C4, alcényle en C2-C4, alcynyle en C2-C4, carboxyalcoxy-C1-C4, alcanoyle-C1-C4, alcoxyalkyle-C1-C4, O-alcoxyimino, O-alcényloxyimino, O-arylalcoxyimino ou dialcoxy-C1-C4-alkyle-C1-C4.

5. Ethers d'oximes de formule I, selon la revendication 1, dans laquelle R représente éthyle, R¹ méthyle, Y oxyméthylène et Z 2,4-diméthylphényle.

6. Procédé de préparation d'éthers d'oximes de formule I, selon la revendication 1 caractérisé par le fait que
a) on met a réagir, dans des conditions basiques, une O-alkyloxime d'ester méthylique d'acide phénylglyoxylique de formule II 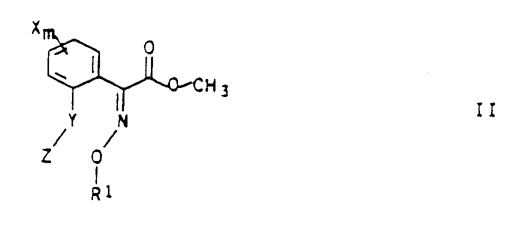 avec un composé de formule III 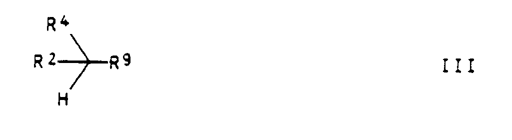 dans laquelle R⁹ représente CO₂-A, et
b) on fait éventuellement réagir les composés Ib ainsi obtenus 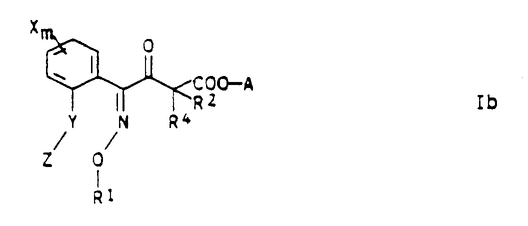 avec DMSO/LiCl pour obtenir les composés de formule Ia 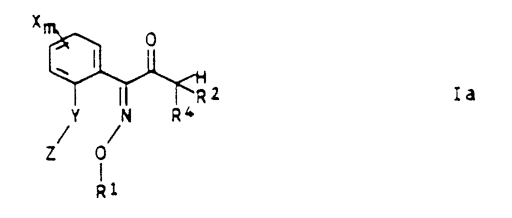 ou
c) on fait réagir les composés de formule II avec de l'hydroxyde de sodium et de l'éthanol, du chlorure de thionyle et de la triéthylamine et du chlorure d'O-méthyl-N-méthylhydroxyl-amine pour obtenir les composés de formule IV 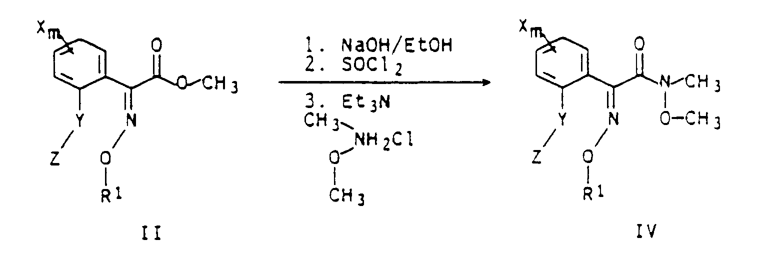 et
d) on fait réagir les amides de formule IV ainsi obtenus avec des réactifs d'organo-lithium pour obtenir les α-oxyiminocétones de formule Id

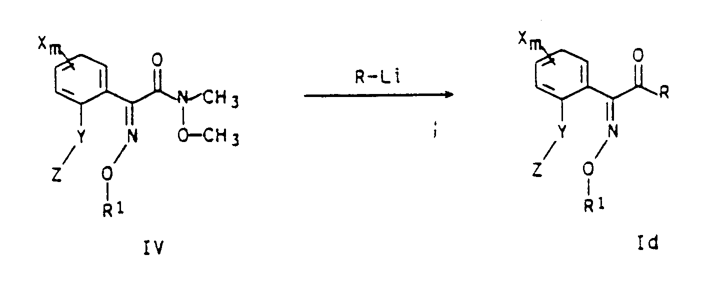

7. Procédé de lutte contre les champignons, caractérisé par le fait que l'on traite les champignons ou les matériaux, plantes, semences ou le sol menacés par l'attaque par les champignons avec une quantité à efficacité fongicide d'un composé de formule I, selon la revendication 1.

8. Fongicide, contenant un support inerte et une quantité à efficacité fongicide d'un composé de formule I, selon la revendication 1.
